# EUROPEAN PATENT APPLICATION

(11) **EP 2 502 991 A1**
(43) Date of publication of application: **26.09.2012**
(21) Application number: 10830048.4
(22) Date of filing: 16.11.2010
(51) Int. Cl.: C12N 15/09, C07K 14/78, C12N 1/19, C12P 21/02

(54) **TRANSFORMANT WHICH PRODUCES COLLAGEN WHEREIN BOTH LYSINE RESIDUE AND PROLINE RESIDUE ARE HYDROXYLATED**

(30) Priority: 16.11.2009 JP 2009260721
(71) Applicant: Sumitomo Chemical Co., Ltd, Chuo-ku, Tokyo 104-8260 (JP); Koken Co., Ltd., Tokyo 112-0004 (JP); Dainippon Sumitomo Pharma Co., Ltd., Osaka 541-8524 (JP)
(72) Inventor: NISHIO, Shoichi, Ikeda-shi Osaka 563-0029 (JP)
(74) Representative: Vossius & Partner
(86) International application number: PCT/JP2010/070356
(87) International publication number: WO 2011/059092

(57) **Abstract**

Disclosed is a transformant in which all of the foreign genes (1)-(3)described below are transfected into a microbial cell in order to obtain collagen that can be a high-performance versatile material which is commercially more valuable as a pharmaceutical product, industrial product, cosmetic product, food and the like. (1) A foreign gene comprising a nucleotide sequence encoding the amino acid sequence of lysyl hydroxylase (2) A foreign gene comprising a nucleotide sequence encoding the amino acid sequence of prolyl hydroxylase (3) A foreign gene comprising a nucleotide sequence encoding the amino acid sequence of collagen.

## Description

### Technical Field

The present invention relates to a transformant that produces collagen in which lysine residues and proline residues are both hydroxylated.

### Background Art

Collagen is a protein contained in a connective tissue existing in various types of body tissues, typically including a subcutaneous tissue, a cartilage, and a bone. Due to high tensile stress of collagen, body tissues containing collagen have a high tolerance for mechanical stress.
Collagen is usable in various applications because it has the excellent physical properties as described, as well as a variety of bioactivities. For example, collagen is used as pharmaceuticals, industrial products, cosmetics and foods, and so it can be regarded as a commercially valuable versatile material.
Collagen occupies an approximately 30% of protein mass present in the body and may be found in substantially all of multicellular animals. To date, it has been found that there may be 20 or more different types of collagens in higher animals, including a human, and also that different types of collagens may be specifically contained in different body tissues (see Non-Patent Documents 1 to 3.)

Representative types of collagens are, for example, Type I collagen, Type II collagen and Type III collagen. Type I collagen is major fibrillar collagen present in bone and skin, which is a heterotrimeric molecule comprising two α1(I) chains and one α2(I) chain. Type II collagen is collagen present in cartilage, which is a homotrimeric molecule comprising three identical α1(II) chains. Type III collagen is collagen present in skin and muscle tissue, which is a homotrimeric molecule comprising three identical α1(III) chains. Type I collagen, Type II collagen and Type III collagen can be purified from the body tissues by, for example, the procedures described in Non-Patent Documents 4 and 5.
Non-Patent Document 6 discloses Type I collagen with hydroxylated proline residues, produced by coexpressing a Type I collagen precursor and a prolyl hydroxylase in yeast cells. Patent Document 1 describes Type I collagen or Type III collagen with hydroxylated proline residues, produced by coexpressing a Type I collagen precursor or a Type III collagen precursor and a prolyl hydroxylase in yeast cells. However, none of these documents disclose hydroxylated lysine residues.

### Prior Art Documents

### Non-Patent Documents

Non-Patent Document 1: Masao Tanihara supervised, "Production and Development of Applications of Collagen," ISBN : 978-4-7813-0071-3, CMC Publishing Co., Lid.;
Non-Patent Document 2: Volume Editors: Brinckmann, J., Notbohm, H., Muller, P.K., "Collagen primer in Structure, Processing and Assembly", Topics in Current Chemistry Vol. 247 (2005);
Non-Patent Document 3: Gelse, K., Poschl, E., Aigner, T., "Collagens-structure, function, and biosynthesis", Advanced Drug Delivery Reviews 55: 1531-1546 (2003);
Non-Patent Document 4: Miller et al., Methods In Enzymology, 82: 33-64 (1982), Academic Press;
Non-Patent Document 5: Byers et al., Biochemistry 13: 5243-5248 (1974); and
Non-Patent Document 6: P. David Toman et al., J. Biol. Chem., Vol. 275, No. 30, July 28, p23303-23309 (2000).

### Patent Documents

Patent Document 1: Kivirikko, Kari I., International Publication No. WO 97/38710 (1997).

### Disclosure of the Invention

### Problems to be Solved by the Invention

There has been a need to find a novel collagen which is usable as a high performance versatile material that is more commercially valuable for pharmaceuticals, industrial products, cosmetics and foods. In particular, for example, there has been a need to find a novel collagen having its stabilized triple-helical structure and an increased ability for fibril formation, and there has been a need to develop a process for producing such novel collagen.

### Means for Solving the Problems

The present invention provides:
1. A transformant (hereinafter, sometimes referred to as a transformant of the present invention) in which all of the following genes (1), (2) and (3):
   (1) a foreign gene comprising a nucleotide sequence encoding an amino acid sequence of lysyl hydroxylase,
   (2) a foreign gene comprising a nucleotide sequence encoding an amino acid sequence of prolyl hydroxylase, and
   (3) a foreign gene comprising a nucleotide sequence encoding an amino acid sequence of collagen;
   are transfected into a microbial cell;
2. The transformant according to the above 1, which coexpresses all of the proteins encoded by the genes (1), (2) and (3) within the cell;
3. The transformant according to the above 1 or 2, wherein the lysyl hydroxylase in (1) is one or more lysyl hydroxylases selected from among lysyl hydroxylase 1, lysyl hydroxylase 2 or lysyl hydroxylase 3;
4. The transformant according to any one of 1 to 3, wherein the prolyl hydroxylase in (2) is an enzyme composed of α subunits of prolyl 4-hydroxylase and β subunits of prolyl 4-hydroxylase;
5. The transformant according to the above 4, wherein the β subunit of prolyl 4-hydroxylase is fused with a secretory signal sequence of a yeast α-factor;
6. The transformant according to the above 4 or 5, wherein the α subunit of prolyl 4-hydroxylase is an α1 subunit, an α2 subunit or an α3 subunit;
7. The transformant according to any one of the above 1 to 6, wherein the collagen in (3) is one or more collagens selected from among Type I to Type XXIX collagens;
8. The transformant according to any one of the above 1 to 7, wherein at least one of the nucleotide sequence encoding the amino acid sequence of the lysyl hydroxylase in (1) and the nucleotide sequence encoding the prolyl hydroxylase in (2) is linked to downstream of a promoter derived from yeast;
9. The transformant according to the above 8, wherein the promoter is an alcohol oxidase 1 gene promoter;
10. The transformant according to any one of the above 1 to 9, wherein the microbial cell is an eukaryote cell;
11. The transformant according to the above 10, wherein the eukaryote cell is a yeast cell;
12. The transformant according to the above 11, wherein the yeast cell is a methylotrophic yeast cell;
13. The transformant according to the above 12, wherein the methylotrophic yeast cell is Komagataella pastoris;
14. Collagen which is obtainable by being produced by the transformant according to any one of the above 1 to 13 (hereinafter, sometimes referred to as collagen of the present invention);
15. The collagen according to the above 14, wherein 15% or more of total lysine residues are hydroxylated;
16. The collagen according to the above 14, wherein the lysine residues in a telopeptide region are hydroxylated;
17. A process for producing collagen (hereinafter, sometimes referred to as production process of the present invention), comprising:
   a first step of transfecting all of the following genes (1), (2) and (3):
      (1) a foreign gene comprising a nucleotide sequence encoding an amino acid sequence of lysyl hydroxylase,
      (2) a foreign genes comprising a nucleotide sequence encoding an amino acid sequence of propyl hydroxylase, and
      (3) a foreign gene comprising a nucleotide sequence encoding an amino acid sequence of collagen;
   into the microbial cell;
   a second step of cultivating the transformant resulting from the first step to produce the collagen; and
   a third step of collecting the collagen produced in the second step; and
18. Collagen produced by the process according to the above 17.

### Effects of the Invention

According to the present invention, it becomes possible to provide collagen which is usable as a high performance versatile material that is more commercially valuable for pharmaceuticals, industrial products, cosmetics and foods. Specifically, for example, collagen which has a stabilized triple-helical structure and an increased ability for fibril formation, and a process for producing the collagen can be provided.

### Brief Description of the Drawings

Fig. 1 is a flow chart schematically illustrating a process of constructing a plasmid for transfection of a prolyl 4-hydroxylase α1 subunit gene, a β subunit of a prolyl 4-hydroxylase gene and a lysyl hydroxylase 1 gene.
Fig. 2 is a flow chart schematically illustrating a process of constructing a plasmid for transfection of a prolyl 4-hydroxylase α1 subunit gene, a β subunit of a prolyl 4-hydroxylase gene and a lysyl hydroxylase 2 gene.
Fig. 3 is a flow chart schematically illustrating a process of constructing a plasmid for transfection of a human collagen Type III gene.
Fig. 4 is a schematic view illustrating a structure of the plasmid for transfection of a prolyl 4-hydroxylase α1 subunit gene, a β subunit of a prolyl 4-hydroxylase gene and a lysyl hydroxylase 1 gene.
Fig. 5 is a schematic view illustrating a structure of the plasmid for transfection of a prolyl 4-hydroxylase α1 subunit gene, a β subunit of a prolyl 4-hydroxylase gene and a lysyl hydroxylase 2 gene.
Fig. 6 is an electrophoretic profile illustrating the collagens purified individually from a TT061226-1-3 strain and a TT061226-3-6 strain, wherein Lane M shows an electrophoretic profile of a molecular weight marker, Lane 1 shows an electrophoretic profile of the collagen purified from the TT061226-1-3 strain, and Lane 2 shows an electrophoretic profile of the collagen purified from the TT061226-3-6 strain.
Fig. 7 is a graph illustrating the ability for fibril formation of collagens.

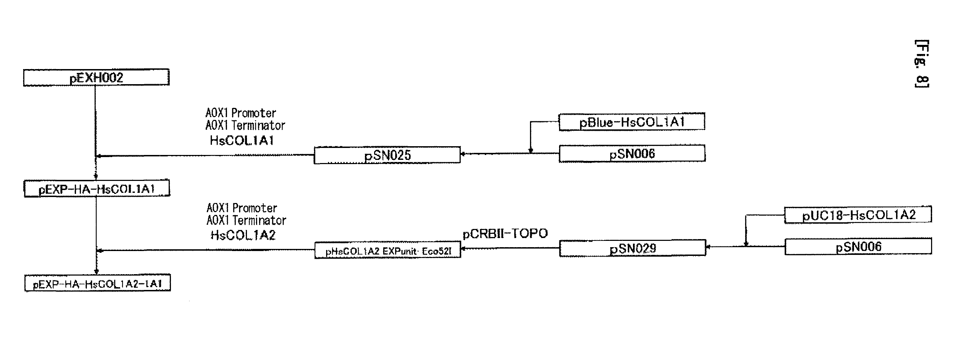

Fig. 8 is a flow chart schematically illustrating a process of constructing a plasmid for transfection of a human collagen Type I α1 gene and a human collagen Type I α2 gene.
Fig. 9 is an electrophoretic profile illustrating the collagens purified individually from a TT080417-1-8 strain, a TT080724-1-11 strain and a 060713-1-3 strain, wherein Lane M shows an electrophoretic profile of a molecular weight marker, Lane 1 shows an electrophoretic profile of the collagen purified from the 060713-1-3 strain, Lane 2 shows an electrophoretic profile of the collagen purified from the TT080417-1-8 strain, and Lane 3 shows an electrophoretic profile of the collagen purified from the TT080724-1-11 strain.
Fig. 10 is a graph illustrating the ability for fibril formation of collagens, wherein rhCl corresponds to the collagen purified from the 060713-1-3 strain, rhCl+LH1 corresponds to the collagen purified from the TT080417-1-8 strain and rhCl+LH2 corresponds to the collagen purified from the TT080724-1-11 strain.

### Mode for Carrying Out the Invention

It is considered that the invention described herein is not limited to specific methodologies, protocols and reagents described, but may be modified. It is also considered that the terms used herein are used merely for description of specific embodiments, without intention to limit the scope of the present invention.
Unless otherwise specified, all technical and chemical terms used herein have the same meanings as commonly understood by those with an ordinary skill in the art to which the present invention belongs. Although any methods and materials similar or equivalent to those described herein may be used in the exploitation or verification of the invention, preferred methods, apparatuses and materials are described below.

The transformant of the present invention is characterized in that it is obtained by transfecting all of the following genes (1), (2) and (3):
(1) a foreign gene comprising a nucleotide sequence encoding an amino acid sequence of lysyl hydroxylase;
(2) a foreign gene comprising a nucleotide sequence encoding an amino acid sequence of prolyl hydroxylase; and
(3) a foreign gene comprising a nucleotide sequence encoding an amino acid sequence of collagen;
into the microbial cell. Accordingly, the transformant of the present invention is a microbial cell which contains a polynucleotide comprising a foreign nucleotide sequence encoding an amino acid sequence of lysyl hydroxylase, a polynucleotide comprising a foreign nucleotide sequence encoding an amino acid sequence of prolyl hydroxylase, and a polynucleotide comprising a foreign nucleotide sequence encoding an amino acid sequence of collagen.

The transformant of the present invention can produce a stable collagen in which lysine residues and proline residues are both hydroxylated. Specifically, each of the transformant comprising a lysyl hydroxylase 1 gene, prolyl hydroxylase genes and a collagen gene, and the transformant comprising a lysyl hydroxylase 3 gene, prolyl hydroxylase genes and a collagen gene can produce collagen, in which, for example, the composition ratio of hydroxylated lysine residues to total lysine residues is equivalent to or higher than that of natural collagen, and the composition ratio of hydroxylated proline residues to total proline residues is substantially equal to that of the natural collagen, respectively. More specifically, the transformant of the present invention comprising a lysyl hydroxylase 1 gene, prolyl hydroxylase genes and collagen Type I genes may produce collagen, in which 30% or more of total lysine residues are hydroxylated and 45% or more of total proline residues are hydroxylated, preferably in which 50% or more of total lysine residues are hydroxylated and 45% or more of total proline residues are hydroxylated. The transformant of the present invention comprising a lysyl hydroxylase 1 gene, prolyl hydroxylase genes and a collagen Type III gene can also produce collagen, in which 15% or more of total lysine residues are hydroxylated and 50% or more of total proline residues are hydroxylated, preferably in which 35% or more of total lysine residues and 50% or more of total proline residues are both hydroxylated. The transformant comprising a lysyl hydroxylase 2 gene, prolyl hydroxylase genes and a collagen gene can also produce collagen, in which lysine residues in the telopeptide region and, for example, 45% or more of total proline residues are both hydroxylated. The transformant of the present invention may be used to produce collagen in which for example, 45% or more of proline are hydroxylated, but for practical purposes, the composition ratio of the hydroxylated proline residues is not particularly intended to be limited as long as the collagen forms stable triple-helical structure.
Since collagen in which lysine residues and proline residues are both hydroxylated is collagen which has a stable triple-helical structure and an enhanced ability for fibril formation, it can be regarded as being a high performance versatile material that is more commercially valuable for pharmaceuticals, industrial products, cosmetics and foods.

The transformant of the present invention may be produced, for example, by constructing a plasmid to be used in gene transfer techniques and using the resulting Gene Transfer Plasmid to transform a host cell. The transformant of the present invention may be prepared in accordance with a typical genetic engineering procedure.
In the genetic engineering procedure, a microbial cell-(i.e., host-) vector system can include, for example, but not especially limited to, the host-vector system using bacteria such as Escherichia, Bacillus or Pseudomonas as the host, and a bacteriophage, a plasmid or a cosmid as the vector; the host-vector system using the yeast such as Komagataella, Saccharomyces, Hansenula, Candida or Ogataea as the host, and an episomal plasmid, a ARS-CEN plasmid or a plasmid integrated into the chromosome as the vector; or the like. Preferably, the Gene Transfer Plasmid may be produced by using Escherichia as the host and the plasmid as the vector. Further, the transformant of the present invention can be produced by using the yeast such as Komagataella, Saccharomyces, Hansenula, Candida or Ogataea as the host, and the plasmid integrated into the chromosome as the vector.

The transformant of the present invention may be produced by transfecting all of the following genes (1), (2) and (3):
(1) a foreign gene comprising a nucleotide sequence encoding an amino acid sequence of lysyl hydroxylase;
(2) a foreign gene comprising a nucleotide sequence encoding an amino acid sequence of prolyl hydroxylase; and
(3) a foreign gene comprising a nucleotide sequence encoding an amino acid sequence of collagen;
into the microbial cell. There is no particular limitation on the order in which the foreign genes are transfected, and therefore, any one of the foreign genes (1), (2) and (3) may be transfected primarily. Two or more of the foreign genes can be also transfected simultaneously.
In the present invention, the "lysyl hydroxylase" can include, for example, a lysyl hydroxylase 1, a lysyl hydroxylase 2 or a lysyl hydroxylase 3.
The origin of "lysyl hydroxylase" is, but not especially limited to, for example, preferably higher animals, and more preferably a human.

Each of the lysyl hydroxylase 1, lysyl hydroxylase 2 and lysyl hydroxylase 3 is an enzyme to form a homodimer, respectively. The lysyl hydroxylase 1 hydroxylates lysine at 6-position located in Y site of the Gly-X-Y repeating sequence existing in the helical structure of the collagen. The lysyl hydroxylase 2 is considered to catalyze hydroxylation of lysine at δ-position located in Y site of the Gly-X-Y, Ser-X-Y or Ala-X-Y sequence in the telopeptide region. And, the lysyl hydroxylase 3 has the ability to hydroxylate lysine located in Y site of the Gly-X-Y repeating sequence existing in the helical structure of the collagen, as well as a galactosyltransferase activity and a glycosyltransferase activity.
The transformant of the present invention may be obtained by transfecting the gene comprising a nucleotide sequence encoding the amino acid sequence of lysyl hydroxylase, along with the gene comprising a nucleotide sequence encoding the amino acid sequence of collagen and the genes comprising a nucleotide sequence encoding the amino acid sequence of prolyl hydroxylase, thereby having an ability to effectively hydroxylate not only proline residues, but also lysine residues, of the collagen.

Examples of the foreign gene comprising a nucleotide sequence encoding the lysyl hydroxylase to be used to produce the transformant of the present invention can include a gene comprising a nucleotide sequence encoding a lysyl hydroxylase 1, a lysyl hydroxylase 2 or a lysyl hydroxylase 3. Particularly, for example, a lysyl hydroxylase 1 gene comprising a nucleotide sequence encoding the amino acid sequence indicated by SEQ ID NO: 52, a lysyl hydroxylase 2 gene comprising a nucleotide sequence encoding the amino acid sequence indicated by SEQ ID NO: 54, or a lysyl hydroxylase 3 gene comprising a nucleotide sequence encoding the amino acid sequence indicated by SEQ ID NO: 56 may be used. Further, specific examples of the lysyl hydroxylase gene may include a lysyl hydroxylase 1 gene comprising a nucleotide sequence indicated by SEQ ID NO: 53, a lysyl hydroxylase 2 gene comprising a nucleotide sequence indicated by SEQ ID NO: 55 and a lysyl hydroxylase 3 gene comprising a nucleotide sequence indicated by SEQ ID NO: 57. A gene artificially obtained by partially introducing deletion, substitution or addition of a base into the gene comprising a nucleotide sequence encoding the lysyl hydroxylase 1, the gene comprising a nucleotide sequence encoding the lysyl hydroxylase 2 or the gene comprising a nucleotide sequence encoding the lysyl hydroxylase 3 may be used, and also, a gene encoding an amino acid sequence of a lysyl hydroxylase where an amino acid has been deleted, substituted or added in the amino acid sequence of the lysyl hydroxylase may be used. In other words, as long as the genes encoding the lysyl hydroxylase has an ability to hydroxylate the lysine residue of the collagen, the genes may be preferably used to produce the transformant of the present invention.

Determination of expression of the lysyl hydroxylase (e.g., lysyl 4-hydroxylase) may be conducted as follows.
To detect the lysyl hydroxylase and the like, for example, immunodetection techniques (i.e., immunoassays) is preferably performed. A wide variety of useful immunodetection techniques (i.e., immunoassays) has been found in, for example, Towbin, et al, Proc. Natl. Acad. Sci. USA, 76,4350-4354 (1979).
The immunoassays may be categorized into the most direct and simplest methods of determination. The immunoassays can include, for example, but not especially limited to, enzyme-linked immunosorbent assay (ELISA), radioimmunoassay (RIA) and immunobeads-trapping assay, or western blot analysis, or the like. Preferably, the immunoassays include the western blot analysis and the like.
The resulting sample may be brought into contact with an antibody under the condition which allows to form the immune complex, thereby forming an immune complex of any combination of antigen and antibody. That is, the antibody and the mixture are incubated for a sufficiently long time to bind them together. After that, it is only needed to detect the antibody specifically bound to the sample. Detection of the immune complex may be typically performed by detecting a radioactivity, fluorescence, biological or enzymatic tag or label used normally in the art.

In the present invention, examples of the "prolyl hydroxylase" can include a prolyl 4-hydroxylase consisting of an α subunit and a β subunit.
Herein, examples of the "α subunit" includes an α1 subunit, an α2 subunit, an α3 subunit, and the like, and preferably an α1 subunit.
The origin of "prolyl hydroxylase" is, but not especially limited to, for example, preferably higher animals, and more preferably a human.
The prolyl 4-hydroxylase is an enzyme consisting of an α subunit and a β subunit, and the prolyl hydroxylase derived from higher animals is a heterotetramer consisting of two α subunits and two β subunits. The α subunit is a subunit which contains a catalytic site involved in the hydroxylation of the proline residue. On the other hand, the β subunit is a subunit which is necessary to form the complex with the α subunit and stabilize it. Both the α subunit and the β subunit are required for function of the prolyl 4-hydroxylase.
The prolyl 4-hydroxylase is the enzyme to hydroxylate the proline residue at the 4-position located in Y site of the Gly-X-Y repeating sequence to form 4-hydroxyproline, and therefore, it is one of enzymes important for synthetic process of the collagen. The hydroxyproline improves the stability of the triple helical structure of the collagen. Appropriate hydroxylation of proline residue located in Y site plays a key role in stabilizing the triple helical structure of the collagen.
The transformant of the present invention may be obtained by transfecting the genes comprising the nucleotide sequence encoding the amino acid sequence of prolyl hydroxylase together with the gene comprising the nucleotide sequence encoding the amino acid sequence of collagen and the gene comprising the nucleotide sequence encoding the amino acid sequence of lysyl hydroxylase, thereby having an ability to effectively hydroxylate not only lysine residues but also of proline residues, of the collagen.

Examples of the foreign gene comprising a nucleotide sequence encoding prolyl hydroxylase to be used to produce the transformant of the present invention can include a gene comprising a nucleotide sequence encoding α subunit of prolyl 4-hydroxylase and a gene comprising a nucleotide sequence encoding β subunit of prolyl 4-hydroxylase. Specifically, for example, as the gene comprising a nucleotide sequence encoding an α subunit of prolyl 4-hydroxylase, a prolyl 4-hydroxylase α1 subunit gene comprising a nucleotide sequence encoding the amino acid sequence indicated by SEQ ID NO: 58, a prolyl 4-hydroxylase α2 subunit gene comprising a nucleotide sequence encoding the amino acid sequence indicated by SEQ ID NO: 60 or a prolyl 4-hydroxylase α3 subunit gene comprising a nucleotide sequence encoding the amino acid sequence indicated by SEQ ID NO: 62 may be used. As the gene comprising a nucleotide sequence encoding a β subunit of prolyl 4-hydroxylase, a prolyl 4-hydroxylase β subunit gene comprising a nucleotide sequence encoding the amino acid sequence indicated by SEQ ID NO: 64 may be used. In addition, as to specific examples of the gene comprising a nucleotide sequence encoding the prolyl hydroxylase gene, examples of the gene comprising a nucleotide sequence encoding an α subunit of prolyl 4-hydroxylase can include a prolyl 4-hydroxylase α1 subunit gene comprising a nucleotide sequence indicated by SEQ ID NO: 59, a prolyl 4-hydroxylase α2 subunit gene comprising a nucleotide sequence indicated by SEQ ID NO: 61 and a prolyl 4-hydroxylase α3 subunit gene comprising a nucleotide sequence indicated by SEQ ID NO: 63; and examples of the gene comprising a nucleotide sequence encoding a subunit of prolyl 4-hydroxylase can include a prolyl 4-hydroxylase β subunit gene comprising a nucleotide sequence indicated by SEQ ID NO: 65. A gene artificially obtained by partially introducing deletion, substitution or addition of a base into the gene comprising a nucleotide sequence encoding the α subunit of prolyl 4-hydroxylase or the gene comprising a nucleotide sequence encoding the β subunit of prolyl 4-hydroxylase may be used, and also, a gene encoding an amino acid sequence of a prolyl hydroxylase where an amino acid has been deleted, substituted or added in the amino acid sequence of the prolyl hydroxylase may be used.
In the other words, a gene encoding a prolyl hydroxylase may be preferably used for the production of the transformant of the present invention as long as the prolyl hydroxylase would have the ability to hydroxylate the proline residue of collagen. The transformant of the present invention may express the prolyl hydroxylase within the microbial cell, and the proline residues in the collagen may be hydroxylated by the resulting prolyl hydroxylase.

The expression of the prolyl hydroxylase( e.g., prolyl 4-hydroxylase) may be determined as follows.
Detection of the prolyl 4-hydroxylase and the like may be preferably performed by, for example, the immunodetection techniques (i.e., immunoassays). A variety of the useful immunodetection techniques (immunoassays) has been found in, for example, Towbin, et al, Proc. Natl. Acad. Sci. USA, 76, 4350-4354 (1979).
The immunoassays may be categorized into the most direct and simplest methods of determination. The immunoassays can include, for example, but not especially limited to, enzyme-linked immunosorbent assay (ELISA), radioimmunoassay (RIA) and immunobeads-trapping assay, or western blot analysis, or the like. Preferably, the immunoassays include the western blot analysis and the like.
The resulting sample may be brought into contact with an antibody under the condition which enables to form the immune complex, forming an immune complex of any combination of antigen and antibody. That is, the antibody and the mixture are incubated for a sufficiently long time to bind them together. After that, it is sufficientto detect the antibody specifically bound to the sample. Detection of the immune complex may be typically performed by detecting a radioactivity, fluorescence, biological or enzymatic tag or label used normally in the art.

In the present invention, examples of the "collagen" can include one or more collagens selected from among Type I to Type XXIX collagens. Preferably, it includes Fibril-forming collagens such as Type I collagen, Type II collagen, Type III collagen, Type V collagen, Type XI collagen, Type XXIV collagen and Type XXVII collagen, and more preferably Type I collagen, Type III collagen and the like. The origin of "collagen" is, but not especially limited to, for example, preferably higher animals, and more preferably a human.
The Type I collagen is a heterotrimer composed of two α1(I) chains and one α2(I) chain, and the Type III collagen is a homotrimer composed of three α1(III) chains.
The transformant of the present invention may be obtained by transfecting the gene comprising the nucleotide sequence encoding the amino acid sequence of collagen together with the gene comprising the nucleotide sequence encoding the amino acid sequence of the lysyl hydroxylase and the genes comprising the nucleotide sequence encoding the amino acid sequence of the prolyl hydroxylase, thereby having an abilityto produce the collagen in which lysine residues and proline residues are both hydroxylated.

Examples of the gene comprising the nucleotide sequence encoding the amino acid sequence of collagen to be used for the production of the transformant of the present invention can include a gene comprising the nucleotide sequence encoding the Type I collagen or a gene comprising the nucleotide sequence encoding the Type III collagen. Specifically, for example, as the gene comprising the nucleotide sequence encoding the Type I collagen derived from human, a gene comprising the nucleotide sequence encoding the amino acid sequence indicated by SEQ ID NO: 66 and a gene comprising the nucleotide sequence encoding the amino acid sequence indicated by SEQ ID NO: 68 may be used; and as the gene comprising the nucleotide sequence encoding the Type III collagen derived from human, a gene comprising the nucleotide sequence encoding the amino acid sequence indicated by SEQ ID NO: 70 may be used. Further, as to specific examples of the collagen genes, examples of a gene comprising the nucleotide sequence encoding the Type I collagen can include a COL1A1 gene comprising the nucleotide sequence indicated by SEQ ID NO: 67 and a COL1A2 gene comprising the nucleotide sequence indicated by SEQ ID NO: 69; and examples of the gene comprising the nucleotide sequence encoding the Type III collagen can include a gene comprising the nucleotide sequence indicated by SEQ ID NO: 71. A gene artificailly obtained by partially introducing deletion, substitution or addition of a nucleotide into the gene comprising the nucleotide sequence encoding the Type I collagen or the gene comprising the nucleotide sequence encoding the Type III collagen may be used, and a gene encoding a partial nucleotide sequence of the collagen may be also used. Furthermore, a gene comprising a nucleotide sequence encoding an amino acid sequence of collagen where an amino acid has been deleted, substituted or added in the amino acid sequence of the collagen or a gene encoding the partial amino acid sequence of the collagen may be also used. In other words, a gene encoding a nucleotide sequence encoding Type I collagen and a gene encoding a nucleotide sequence encoding Type III collagen may be appropriately used for the present invention as long as each of the Type I collagen and the Type III collagen comprises the Gly-X-Y repeating sequence within the amino acid sequence thereof and hold a property to form the collagen-specific triple helical structure.
The expression of the collagen within the microbial cell, as well as the enzymatic conversion of both the lysyl hydroxylase and the prolyl hydroxylase which are coexpressed within the microbial cell, may lead to production of the collagen in which lysine residues and proline residues are hydroxylated.

As a method for transfecting the foreign genes (1) to (3) into the microbial cell, the known techniques such as, for example, but not especially limited to, lithium process, spheroplast procedure and electroporation techniques may be available. The foreign genes transfected into the microbial cell may be maintained in a form of plasmid within the microbial cell, or may be integrated into the chromosome within the microbial cell.
In case of maintaining the foreign genes within the microbial cell, Saccharomyces cerevisiae, Komagataella pastoris, Hansenula polymorpha, Pichia methanolica, Candida Boidini or Ogataea minuta may be used as the microbial cell (i.e., host). When Saccharomyces cerevisiae is used as the host, preferably, a vector to be integrated into the chromosome (type Ylp), a vector comprising the replication origin of the yeast endogenous plasmid 2 µm DNA (type YEp) and a vector comprising autonomously replicating sequence derived from the chromosome (type YCp) may be used as a vector which carries the foreign genes. Among these vectors, the one in a form of a shuttle vector replicable even in Escherichia coli is more preferably used because it may facilitate the construction of the Gene Transfer Plasmid. When Saccharomyces cerevisiae is used, the type YEp plasmid may be preferably used. When Komagataella pastoris, Hansenula polymorpha, Pichia methanolica, Candida Boidini or Ogataea minuta is used as a host, the vector to be integrated into the chromosome may be used as the vector which carries the foreign genes. Among these vectors, typically, the one in a form of a shuttle vector generally replicable even in Escherichia coli may be more preferably used because it may facilitate the construction of the Gene Transfer Plasmid. Specifically, pA0815, pPIC9K and the like are preferably used as the vector for Komagataella pastoris.

When the foreign genes are transfected into the host cell using the vector to be integrated into the chromosome, homologous recombination in the homologous region which is integrated into the vector leads to the integration of the foreign genes into the chromosome and their stable retention within the host cell. The nucleotide sequence in the homologous region can include, but not limited to, for example, the nucleotide sequences such as an amino acid or a nucleic acid synthetic pathway gene, ribosomal DNA and Transposon of Yeast element. In case of using the host which lacks in the amino acid synthetic pathway gene or the nucleic acid synthetic pathway gene, the nucleotide sequence in the homologous region may lead to the complementation of host mutation to be used as a selective marker for the transformant.
When the foreign gene integrated into the vector is transfected into the microbial cell, the selective marker may be used to serve as a guide for generation of gene transfer. As the selective marker gene, typically used may be a gene which leads to phenotypical change when it is integrated into the vector together with the foreign genes and introduced into the cell. As the selective marker gene, an amino acid or nucleic acid synthetic pathway gene, an antibiotic-resistant gene or the like may be used. The amino acid or the nucleic acid synthetic pathway gene can include, but not limited to, for example, LEU2, HIS4, ARG4, TRP1, URA3, ADE2 and the like. Further, the antibiotic-resistant gene can include, but not limited to, for example, genes which induce tolerance to antibiotics such as Zeocyn, Blasticidin S, Geneticin, G418, Chloramphenicol and bleomycin.

A preferable embodiment of the transformant of the present invention may include, for example, the microbial cell which may be an eukaryote cell. Herein, the eukaryote cell can include, for example, yeast cell and the like. Further, the yeast cell may include, for example, a methylotrophic yeast cell and the like. Furthermore, an example of the methylotrophic yeast cell may include Komagataella pastoris and the like.
For the foreign genes (1) to (3), the nucleotide sequence encoding the amino acid sequence of protein may be preferably integrated downstream of the promoter in a form which enable the expression.
As the promoter, without especially any limiting as long as it functions within the host cell, an inducible promoter of which the transcriptional activity may be induced by specific nutrients or substrates may be preferably used. For the foreign genes (1) to (3), the terminator is preferably located downstream of the nucleotide sequence encoding the amino acid sequence of protein.

There is no particular limitation on the promoter and, for example, a gene promoter such as a galactose metabolizing enzyme gene (GAL1, GAL10), an inhibitory acid phosphatase (PHO5), a glyceraldehyde-3-phosphate dehydrogenase gene (TD), a phosphoglycerate kinase gene (PGK), an alcohol oxidase gene (ADH1, AOX1, AOX2, MOX, AOD1), a formic dehydrogenase gene (FDH1, FMD1), a dihydroxyacetone synthase gene (DAS), a peroxisome membrane protein synthetic gene (PER3) and a formaldehyde dehydrogenase gene (FLD1) may be available. The terminator may be effectively used as long as it has a transcription termination activity, and, for example, a terminator of the gene such as the alcohol oxidase gene (AOX1) and the formaldehyde dehydrogenase gene (FLD1) may be available.

The present invention comprises the collagen (collagen of the present invention) obtainable by being produced by the transformant of the present invention. The collagen of the present invention may be one or more collagens selected from among Type I to Type XXIX collagens. The collagen of the present invention obtained from the transformant of the present invention comprising the lysyl hydroxylase 1 gene, the prolyl Hydroxylase genes and collagen gene, and the collagen of the present invention obtained from the transformant of the present invention comprising the lysyl hydroxylase 3 gene, the prolyl hydroxylase genes and collagen gene are collagens in which, for example, the composition ratio of hydroxylated lysine residues to total lysine residues is higher than that of natural collagen, and the composition ratio of hydroxylated proline residues to total proline residues is substantially equal to that of the natural collagen. More specifically, the collagen obtained from the transformant of the present invention comprising the lysyl hydroxylase 1 gene, the prolyl hydroxylase genes and the collagen Type I genes is the collagen in which 30% or more of total lysine residues are hydroxylated and 45% or more of total proline residues are hydroxylated, preferably wherein 50% or more of total lysine residues are hydroxylated and 45% or more of total proline residues are hydroxylated. Further, the collagen obtained from the transformant of the present invention comprising a lysyl hydroxylase 1 gene, a prolyl hydroxylase genes and a collagen Type III gene is the collagen in which 15% or more of total lysine residues are hydroxylated and 50% or more of total proline residues are hydroxylated, preferably in which both of 35% or more of total lysine residues and 50% or more of total proline residues are hydroxylated. Furthermore, the collagen of the present invention obtained from the transformant comprising lysyl hydroxylase 2 gene, a prolyl hydroxylase genes and a collagen gene may lead to production of the collagen in which lysine residues in the telopeptide region and, for example, 45% or more of total proline residues are both hydroxylated. The transformant of the present invention may be used to produce the collagen wherein for example, 45% or more of proline are hydroxylated, but for practical purposes, the composition ratio of the hydroxylated proline residues is not particularly intended to be limited as long as the collagen forms stable triple-helical structure.
Each composition ratio of the hydroxylated proline residues and the hydroxylated lysine residues in the collagen may be determined in the known method of amino acid composition analysis.
The ability for fibril formation of the collagen may be determined, for example, by the following procedures. Specifically, for example, readjustment of a solution of the purified collagens to salt concentration of 1 × D-PBS(-), pH 7.3 to 7.4 followed by keeping at the temperature of 37°C leads to reorientation of the collagen molecules and then clouding of the solution. Such clouding may be regarded as an indication of the ability for fibril formation of the collagen. Accordingly, by means of such property, the ability for fibril formation of the collagen may be determined by measuring the absorbance of the solution of collagens over time while keeping the solution in the collagen concentration of 0.05% to 0.2% at the temperature of 37°C in a salt concentration of 1 × D-PBS(-), pH 7.3 to 7.4.

The production process of the present invention includes:
a first step of transfecting all of the following genes (1), (2) and (3):
   (1) a foreign gene comprising a nucleotide sequence encoding an amino acid sequence of lysyl hydroxylase,
   (2) a foreign gene comprising a nucleotide sequence encoding an amino acid sequence of prolyl hydroxylase, and
   (3) a foreign gene comprising a nucleotide sequence encoding an amino acid sequence of collagen;
   into the microbial cell;
a second step of cultivating transformant resulting from the first step to produce the collagen; and
a third step of collecting the collagen produced from the second step.

Since the first step has been previously described in the description for the transformant of the present invention, herein, detailed description of it is omitted.
The second step can be performed in accordance with the standard microbiological techniques.
As a medium used for cultivation of the transformant resulting from the first step, in general, the medium known in the art may be preferably used, and its cultivation condition may be provided in accordance with the standard techniques.
There is no particular limitation on the "medium", and any of a synthetic medium and a natural medium may be used, preferably in a form of liquid media.
For example, the "synthetic medium" is preferably used with the inclusion of a carbon source, such as a variety of sugar, glycerol, methanol; a nitrogen source, such as urea, ammonium salt or nitrate salt; micronutrients, such as a range of vitamins or nucleotides; and the other mineral salts, such as Mg, Ca, Fe, Na, K, Mn, Co or Cu or the like. Further, the carbon source may be feeded into the medium moderately little by little with a concentrated form in order to prevent inhibition of collagen production due to the high carbon-source contentration.
Alternatively, as the "natural medium," YPD Liquid Medium (1% Yeast Extract (Difco), 2% Bacto Peptone (Difco), 2% glucose) and YPM Liquid Medium may be also preferably used.

There is no particular limitation on the pH of the medium, and the pH is preferably neutral, weakly basic or weakly acidic. When the transformant of the present invention is a methylotrophic yeast cell, a methanol containing medium is preferably used. In this case, its methanol concentration may preferably range from approximately 0.01 to 5%.
There is no particular limitation on the cultivating temperature, and the the cultivating temperature is preferably between 15°C and 43°C. There is no particular limitation on the elapsed cultivating time, and the elapsed cultivating time is preferably between 1 and 1,000 hours. The cultivating is preferably conducted under the stationary standing, with shaking , agitation or aeration,and preferably conducted in batch cultivation, semibatch cultivation or continuous cultivation.
Further, prior to the cultivation (i.e., the main incubation), pre-cultivation is preferably performed. As mediums for pre-cultivation, for example, YNB liquid medium, YPD liquid medium or the like may be preferably used. There is no particular limitation on the cultivation condition for the pre-cultivation, and the elapsed cultivation time is preferably between 10 and 100 hours and for example, when the transformant of the present invention is yeast, the cultivation temperature is preferably between 15°C and 45°C.

The step of colleting the collagen may be performed in accordance with a standard biochemical and a protein engineering techniques. To collect the collagen produced in the second step, it is sufficient to purify the collagen from culture supernatant or cultivated microbial cell containing the collagen produced by the transformant.
The purification technique is not especially limited, but the known techniques may be appropriately used. For instance, fractionation technique, ion-exchange technique, gel filtration chromatography technique, hydrophobic interaction chromatography technique or affinity column chromatography technique or the like may be used.
More convenient purification techniques may include the purification technique with the use of the property possessed by the collagen, i.e., tolerance of triple helical structure for protease. Such purification technique may preferably include the following steps (a) to (e) of:
(a) a step of breaking the transformant in a pH buffer solution;
(b) a step of adding a protease into the lysis solution and degrading contaminants;
(c) a step of colleting a supernatant by centrifuging the degraded broken solution;
(d) a step of salting-out from the supernatant under a various pH conditions; and
(e) a step of dissolving the precipitation collected by the salting-out for desalting.

Thus, the collagen produced by the production process of the present invention can be regarded as being a high performance versatile material that is more commercially valuable for pharmaceuticals, industrial products, cosmetics and foods. Specifically, for example, the collagen produced by the production process of the present invention can be collagen having its stabilized triple-helical structure and an increased ability for fibril formation.

### EXAMPLES

The present invention will be described in more detail below by way of Examples, but the present invention is not limited thereto.
In cloning of the genes and the like, the Gene Transfer Plasmid used for production of the transformant of the present invention may be obtained by cloning the foreign gene to bring into the plasmid in accordance with the techniques described in "Molecular Cloning: A Laboratory Manual 2nd edition", Cold Spring Harbor Laboratory Press (1989), ISBN 0-87969-309-6; "Current Protocols in Molecular Biology", John Wiley & Sons (1987), Inc. ISBN O-471-50338-X; and the like. As the plasmid used for production of the Gene Transfer Plasmid, without especially limiting, for example, pUC119 (Takara Bio Inc.), pTV118N (Takara Bio Inc.), pUC19 (Toyobo Co., Ltd.), pBluescriptII (Stratagene), pCR2.1-TOPO (Invitrogen), pCR-BluntII-TOPO (Invitrogen) and the like may be available. The cloning procedures will be described below in detail.

### Example 1 (Cloning of Genes)

### (1-1) Cloning of Lysyl Hydroxylase 1 Gene (PLOD1)

A cDNA clone (ID: 3917351) was purchased from Invitrogen. The following oligonucleotides 1 and 2 were synthesized. PLOD1 was amplified by PCR using the cDNA clone as a template and the oligonucleotides 1 and 2 as primers.
(a) Oligonucleotide 1: CGTCATATGATGCGGCCCCTGCTGC (SEQ ID NO: 1)
(b) Oligonucleotide 2: ATCCTCGAGTTAGGGATCGACGAAGGAGAC (SEQ ID NO: 2)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO4 (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 ul |
| (g) | Sterile distilled water | 33 µl |

PCR was conducted using PERKIN ELMER GeneAmp PCR System 9700. This reaction was conducted under the following conditions: heating the reaction solution at 94°C for 2 minutes followed by 25 cycles of denaturation at 94°C for 15 seconds, annealing at 50°C for 30 seconds and extension at 68°C for 2 minutes, and then maintaining the reaction solution at 68°C for 5 minutes.
The about 2000bp double-stranded DNA fragment prepared by the PCR was ligated to the "PCR Product insertion site" of pCR2.1-TOPO plasmid, and the resulting ligation solution was used for transformation of E. coli (Competent high DH5α, Toyobo Co., Ltd.). For the ligation, Invitrogen TOPO TA cloning kit was used.
On LB agar medium (1% Bacto Tripton, 0.5% Bacto Yeast Extract, 1% sodium chloride) containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pPLOD1-TOPO hereinafter.

### (1-2) Cloning of Lysyl Hydroxylase 2 Gene (PLOD2)

cDNA clone (ID: 4994235) was purchased from Invitrogen. The following oligonucleotides 3 and 4 were synthesized. PLOD2 was amplified by PCR using the cDNA clone as a template and the oligonucleotides 3 and 4 as primers.
(a) Oligonucleotide 3: CGAGATCTGATGGGGGGATGCACGGT (SEQ ID NO: 3)
(b) Oligonucleotide 4: GCAGATCTCGTTAGGGATCTATAAATGACACTG (SEQ ID NO: 4)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgS0₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 2 minutes followed by 25 cycles of denaturation at 94°C for 15 seconds, annealing at 50°C for 30 seconds and extension at 68°C for 2 minutes, and then maintaining the reaction solution at 68°C for 5 minutes.
The about 2000bp double-stranded DNA fragment prepared by the PCR was ligated to the "PCR Product insertion site" of pCR2.1-TOPO plasmid, and the resulting ligation solution was used for transformation of E. coli (Competent high DH5α, Toyobo Co., Ltd.). For the ligation, Invitrogen TOPO TA cloning kit was used.
On LB agar medium (1% Bacto Tripton, 0.5% Bacto Yeast Extract, 1% sodium chloride) containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pPLOD2-TOPO hereinafter.

### (1-3) Cloning of Histidinol Dehydrogenase Gene (HIS4)

The sequence information of HIS4 (Accession No.X56180) was obtained through NCBI online database. The following oligonucleotides 5 and 6 were synthesized based on the sequence information. HIS4 was amplified by PCR using the oligonucleotides 5 and 6 as primers and genomic DNA of Komagataella pastoris ATCC76273 obtained from ATCC as a template. The genomic DNA was isolated using QIAGEN Genomic-tip 100/G and Genomic DNA Buffer Set.
(a) Oligonucleotide 5: GATCTCCTGATGACTGACTCACTGATAATA (SEQ ID NO: 5)
(b) Oligonucleotide 6: TAATTAAATAAGTCCCAGTTTCTCCATACG (SEQ ID NO: 6)
As a polymerase for the PCR, Invitrogen AccuPrime Pfx Polymerase was used. The details of composition of the reaction solutions are given as follows:

| | | |
|---|---|---|
| (a) | Genomic DNA (15 ng/µl) | 1 µl |
| (b) | Primers (10 pmol/µl) | 1.5 µl each |
| (c) | 10 × AccuPrime Pfx reaction mix | 5 µl |
| (d) | AccuPrime Pfx DNA polymerase (2.5 U/µl) | 0.5 µl |
| (e) | Sterile distilled water | 40.5 µl |

PCR was conducted using PERKIN ELMER GeneAmp PCR System 9700. This reaction was conducted under the following conditions: heating the reaction solution at 94°C for 2 minutes followed by 35 cycles of denaturation at 94°C for 15 seconds, annealing at 60°C for 30 seconds and extension at 68°C for 2.5 minutes, and then maintaining the reaction solution at 68°C for 5 minutes.
The about 2600bp double-stranded DNA fragment prepared by the PCR was ligated to the "PCR Product insertion site" of pCR-BluntII-TOPO plasmid, and the resulting ligation solution was used for the transformation of E. coli (One Shot TOP10F' Chemically Compitent E.coli, Invitorgen). For the ligation, Invitrogen ZERO-BluntII TOPO PCR cloning kit was used.
On LB agar medium containing 50 µg/ml of kanamycin, the transformed E. coli was inoculated to cultivate it. A colony formed, on the agar medium was inoculated into sterile LB medium (2 ml) containing 50 µg/ml of kanamycin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pHIS4-TOPO hereinafter.

### (1-4) Cloning of Argininosuccinate Lyase Gene (ARG4)

The sequence information of ARG4 (Accession No. AF321097) was obtained through NCBI online database. The following oligonucleotides 7 and 8 were synthesized based on the sequence information. ARG4 was amplified by PCR using the oligonucleotides 7 and 8 as primers and genomic DNA of Komagataella pastoris ATCC76273 obtained from ATCC as a template. The genomic DNA was isolated using QIAGEN Genomic-tip 100/G and Genomic DNA Buffer Set.
(a) Oligonucleotide 7: ACGAAAATATGGTACCTGCCCTCAC (SEQ ID NO: 7)
(b) Oligonucleotide 8: GTTCTATCTACCCGAGGAAACCGATACATA (SEQ ID NO: 8)
As a polymerase for the PCR, Invitrogen AccuPrime Pfx Polymerase was used. The details of composition of the reaction solutions are given as follows:

| | | |
|---|---|---|
| (a) | Genomic DNA (15 ng/µl) | 1 µl |
| (b) | Primers (10 pmol/µl) | 1.5 µl each |
| (c) | 10 × AccuPrime Pfx reaction mix | 5 µl |
| (d) | AccuPrime Pfx DNA polymerase (2.5 U/µl) | 0.5 µl |
| (e) | Sterile distilled water | 40.5 µl |

PCR was conducted using PERKIN ELMER GeneAmp PCR System 9700. This reaction was conducted under the following conditions: heating the reaction solution at 94°C for 2 minutes followed by 35 cycles of denaturation at 94°C for 15 seconds, annealing at 65°C for 30 seconds and extension at 68°C for 2.5 minutes, and then maintaining the reaction solution at 68°C for 5 minutes.
The about 2200 bp double-stranded DNA fragment prepared by the PCR was ligated to the "PCR Product insertion site" of pCR-BluntII-TOPO plasmid, and the resulting ligation solution was used for the transformation of E. coli (One Shot TOP10F' Chemically Compitent E.coli, Invitrogen). For the ligation, Invitrogen ZERO-BluntII TOPO PCR cloning kit was used.
On LB agar medium containing 50 µg/ml of kanamycin, the transformed E. coli was inoculated to cultivate it. A colony formed on the agar medium was inoculated into sterile LB medium (2 ml) containing 50 µg/ml of kanamycin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pARG4-TOPO hereinafter.

### (1-5) Cloning of Alcohol Dehydrogenase 1 (AOX1) Promoter

The sequence information of AOX1 (Accession No. E00913 and U96967) was obtained through NCBI online database. The following oligonucleotides 9 and 10 were synthesized based on the sequence information. AOX1 promoter was amplified by PCR using the oligonucleotides 9 and 10 as primers and genomic DNA of Komagataella pastoris ATCC76273 obtained from ATCC as a template. The genomic DNA was isolated using QIAGEN Genomic-tip 100/G and Genomic DNA Buffer Set.
(a) Oligonucleotide 9: AGATCTAACATCCAAAGACGAAAGGTT (SEQ ID NO: 9)
(b) Oligonucleotide 10: ATCCACCACCTAGAACTAGGATATCAAAC (SEQ ID NO: 10)
As a polymerase for the PCR, Invitrogen AccuPrime Pfx Polymerase was used. The details of composition of the reaction solutions are given as follows:

| | | |
|---|---|---|
| (a) | Genomic DNA (15 ng/µl) | 1 µl |
| (b) | Primers (10 pmol/µl) | 1.5 µl each |
| (c) | 10 × AccuPrime Pfx reaction mix | 5 µl |
| (d) | AccuPrime Pfx DNA polymerase (2.5 U/µl) | 0.5 µl |
| (e) | Sterile distilled water | 40.5 µl |

PCR was conducted using PERKIN ELMER GeneAmp PCR System 9700. This reaction was conducted under the following conditions: heating the reaction solution at 94°C for 2 minutes followed by 35 cycles of denaturation at 94°C for 15 seconds, annealing at 62°C for 30 seconds and extension at 68°C for 1 minute, and then maintaining the reaction solution at 68°C for 5 minutes.
The about 940bp double-stranded DNA fragment prepared by the PCR was ligated to the "PCR Product insertion site" of pCR-BluntII-TOPO plasmid, and the resulting ligation solution was used for the transformation of E. coli (One Shot TOP10F' Chemically Compitent E.coli, Invitrogen). For the ligation, Invitrogen ZERO-BluntII TOPO PCR cloning kit was used.
On LB agar medium containing 50 µg/ml of kanamycin, the transformed E. coli was inoculated to cultivate it. A colony formed on the agar medium was inoculated into sterile LB medium (2 ml) containing 50 µg/ml of kanamycin, and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pAOXlPro+15aa-TOPO hereinafter.

### (1-6) Cloning of Alcohol Dehydrogenase 1 (AOX1) Terminator

The sequence information of AOX1 (Accession No. U96967) was obtained through NCBI online database. The following oligonucleotides 11 and 12 were synthesized based on the sequence information. AOX1 terminator was amplified by PCR using the oligonucleotides 11 and 12 as primers and genomic DNA of Komagataella pastoris ATCC76273 obtained from ATCC as a template. The genomic DNA was isolated using QIAGEN Genomic-tip 100/G and Genomic DNA Buffer Set.
(a) Oligonucleotide 11: CCTTAGACATGACTGTTCCTCAGTTC (SEQ ID NO: 11)
(b) Oligonucleotide 12: GCACAAACGAACGTCTCACTTAAT (SEQ ID NO: 12)
As a polymerase for the PCR, Invitrogen AccuPrime Pfx Polymerase was used. The details of composition of the reaction solutions are given as follows:

| | | |
|---|---|---|
| (a) | Genomic DNA (15 ng/µl) | 1 µl |
| (b) | Primers (10 pmol/µl) | 1.5 µl each |
| (c) | 10 × AccuPrime Pfx reaction mix | 5 µl |
| (d) | AccuPrime Pfx DNA polymerase (2.5 U/µl) | 0.5 µl |
| (e) | Sterile distilled water | 40.5 µl |

PCR was conducted using PERKIN ELMER GeneAmp PCR System 9700. This reaction was conducted under the following conditions: heating the reaction solution at 94°C for 2 minutes followed by 35 cycles of denaturation at 94°C for 15 seconds, annealing at 60°C for 30 seconds and extension at 68°C for 30 seconds, and then maintaining the reaction solution at 68°c for 5 minutes.
The about 300bp double-stranded DNA fragment prepared by the PCR was ligated to the "PCR Product insertion site" of pCR-BluntII-TOPO plasmid, and the resulting ligation solution was used for the transformation of E. coli (One Shot TOP10F' Chemically Compitent E.coli, Invitrogen). For the ligation, Invitrogen ZERO-BluntII TOPO PCR cloning kit was used.
On LB agar medium containing 50 µg/ml of kanamycin, the transformed E. coli was inoculated to cultivate it. A colony formed on the agar medium was inoculated into sterile LB medium (2 ml) containing 50 µg/ml of kanamycin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pAOXlterm-TOPO hereinafter.

### (1-7) Cloning of Non-coading Region located 3'-downstream of Alcohol Dehydrogenase 1 (AOX1)

The sequence information of 3'-downstream region of AOX1 was obtained from the sequence information of a plasmid (pA0815) manufactured by Invitrogen. The following oligonucleotides 13 and 14 were synthesized based on the sequence information. The 3'-downstream region of AOX1 was amplified by PCR using the oligonucleotides 13 and 14 as primers and genomic DNA of Komagataella pastoris ATCC76273 obtained from ATCC as a template. The genomic DNA was isolated using QIAGEN Genomic-tip 100/G and Genomic DNA Buffer Set.
(a) Oligonucleotide 13: TCGAGTATCTATGATTGGAAGTATGGGAAT (SEQ ID NO: 13)
(b) Oligonucleotide 14: GATCTTGAGATAAATTTCACGTTTAAAATC (SEQ ID NO: 14)
As a polymerase for the PCR, Invitrogen AccuPrime Pfx Polymerase was used. The details of composition of the reaction solutions are given as follows:

| | | |
|---|---|---|
| (a) | Genomic DNA (15 ng/µl) | 1 µl |
| (b) | Primers (10 pmol/µl) | 1.5 µl each |
| (c) | 10 × AccuPrime Pfx reaction mix | 5 µl |
| (d) | AccuPrime Pfx DNA polymerase (2.5 U/ul) | 0.5 µl |
| (e) | Sterile distilled water | 40.5 µl |

PCR was conducted using PERKIN ELMER GeneAmp PCR System 9700. This reaction was conducted under the following conditions: heating the reaction solution at 94°C for 2 minutes followed by 35 cycles of denaturation at 94°C for 15 seconds, annealing at 58°C for 30 seconds and extension at 68°C for 1.5 minutes, and then maintaining the reaction solution at 68°C for 5 minutes.
The about 750bp double-stranded DNA fragment prepared by the PCR was ligated to the "PCR Product insertion site" of pCR-BluntII-TOPO plasmid, and the resulting ligation solution was used for the transformation of E. coli (One Shot TOP10F' Chemically Compitent E.coli, Invitrogen). For the ligation, Invitrogen ZERO-BluntII TOPO PCR cloning kit was used.
On LB agar medium containing 50 µg/ml of kanamycin, the transformed E. coli was inoculated to cultivate it. A colony formed on the agar medium was inoculated into sterile LB medium (2 ml) containing 50 µg/ml of kanamycin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pAOX1―3'―TOPO hereinafter.

### (1-8) Cloning of α-Factor Signal Sequence

The sequence information of a sex determining gene α― factor of Saccharomyces cerevisiae (Accession No. J0134 and X01581) was obtained through NCBI online database. The following oligonucleotides 15 and 16 were synthesized based on the sequence information. DNA encoding α-factor was amplified by PCR using the oligonucleotides 15 and 16 as primers and genomic DNA of Saccharomyces cerevisiae NBRC1136 obtained from NBR as a template. The genomic DNA was isolated using QIAGEN Genomic-tip 100/G and Genomic DNA Buffer Set.
(a) Oligonucleotide 15: TCAAACAAGAAGATTACAAACTATCAATTTCA (SEQ ID NO: 15)
(b) Oligonucleotide 16: TTTGTTACATCTACACTGTTGTTATCAGTCG (SEQ ID NO: 16)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Genomic DNA solution (15 ng/µl) | 1 µl |
| (b) | dNTP (2 mM-mix each) | 5 µl |
| (c) | MgSO₄ (25 mM) | 2 µl |
| (d) | Primers (10 pmol/µl | 1.5 µl each |
| (e) | 10 × PCR buffer for KOD-plus- | 5 µl |
| (f) | KOD-plus- DNA polymerase (1 U/µl | 1 µl (1U) |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 35 cycles of denaturation at 94°C for 15 seconds, annealing at 60°C for 30 seconds and extension at 68°C for 1 minute, and then maintaining the reaction solution at 68°C for 5 minutes.
The about 600bp double-stranded DNA fragment prepared by the PCR was ligated to the "PCR Product insertion site" of pCR-BluntII-TOPO plasmid, and the resulting ligation solution was used for the transformation of E. coli (One Shot TOP10F' Chemically Compitent E.coli, Invitrogen). For the ligation, Invitrogen ZERO-BluntII TOPO PCR cloning kit was used.
On LB agar medium containing 50 µg/ml of kanamycin, the transformed E. coli was inoculated to cultivate it. A colony formed on the agar medium was inoculated into sterile LB medium (2 ml) containing 50 µg/ml of kanamycin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pα-factor-TOPO hereinafter.

### (1-9) Cloning of Human Type III Collagen Gene

The sequence information of human Type III Collagen (Accession No. NM000090) was obtained through NCBI online database. The following oligonucleotides 17 and 18 were synthesized based on the sequence information. A DNA encoding human Type III Collagen was amplified by PCR using the oligonucleotides 17 and 18 with addition of a phosphate group to each 5' end as primers and Human brain mRNA obtained from Toyobo Co., Ltd. as a template .
(a) Oligonucleotide 17: GGCTGAGTTTTATGACGGGC (SEQ ID NO: 17)
(b) Oligonucleotide 18: GACAAGATTAGAACAAGAGG (SEQ ID NO: 18)
As a polymerase for the PCR, KOD―Plus― PCR polymerase, manufactured by Toyobo Co., Ltd., was used. The details of composition of the reaction solutions are given as follows:

| | | |
|---|---|---|
| (a) | Genomic DNA solution (15 ng/µl) | 1 µl |
| (b) | dNTP(2 mM―mix each) | 5 µl |
| (c) | MgSO₄ (25 mM) | 2 µl |
| (d) | Primers (10 pmol/µl | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted by 35 cycles of denaturation at 98°C for 20 seconds, annealing at 58°C for 10 seconds and extension at 74°C for 5 minutes.
pUC18 plasmid was cut with the restriction enzyme SmaI and then dephosphorylated using alkaline phosphatase.
The double-stranded DNA fragment and the plasmid were ligated, and the resulting ligation solution was used for the transformation of E. coli (Component high DH5α, manufactured by Toyobo Co., Ltd.) was transformed. For the ligation, Ligation high manufactured by Toyobo Co., Ltd. was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pUC19-COL3Al hereinafter.

### (1-10) Cloning of Human Collagen Type I α1 Gene (Construction of pBlue-HsCOL1A1)

The following oligonucleotides 52 and 53 were synthesized. After a phosphate group was added to each 5' end of the oligonucleotides 52 and 53 using T4 polynucleotide kinase (Toyobo Co., Ltd.), PCR was conducted using these oligonucleotides as primers and Human brain cDNA (Toyobo Co., Ltd.) as a template to amplify DNA encoding human collagen Type I α1.
(a) Oligonucleotide 52: CAGCCACAAAGAGTCTACATGTCTAGG (SEQ ID NO: 72)
(b) Oligonucleotide 53: AGGTTGGGATGGAGGGAGTT (SEQ ID NO: 73)
The composition of the reaction solutions is given as follows:

| | | |
|---|---|---|
| (a) | cDNA solution | 5 µl |
| (b) | dNTP (2 mM―mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- (Toyobo Co.,Ltd.) | 5 µl |
| (f) | KOD-plus- DNA polymerase (1 U/µl, ToyoboCo., Ltd.) | 1 µl |
| (g) | Sterile distilled water | 29 µl |

PCR was conducted by heating the reaction solution at 94°C for 2 minutes and then 35 cycles of denaturation at 98°C for 20 seconds, annealing at 58°C for 10 seconds and extensione at 74°C for 5 minutes.
Approximately 4.5 kb of the DNA obtained from the PCR was purified using MagExtractor PCR&Gel Clean up (Toyobo Co., Ltd.)
A pBluescriptII KS(+) plasmid (Stratagene) was cut with the restriction enzyme EcoRV, dephosphorylated by the use of alkaline phosphatase, and then purified using MagExtractor PCR&Gel Clean up (Toyobo Co., Ltd.)
The about 4.5kb of the DNA and the plasmid were ligated, and the resulting ligation solution was used for the transformation of E. coli (Competent high DH5α, Toyobo Co., Ltd.). For the ligation, Ligation high, manufactured by Toyobo Co., Ltd., was used.
On. LB agar medium containing 50 µg/ml of ampicillin, the transformed E. coli was inoculated to cultivate it. A colony formed on the agar medium was inoculated into LB medium containing 50 µg/ml of ampicillin and incubated in the test tube with shaking (37°C, 17 hours). Then, using MagExtractor plasmid (Toyobo Co., Ltd.), the plasmid into which the DNA encoding human collagen Type I αl was inserted (hereinafter, it may be referred to as pBlue-HsCOL1A1) was isolated from the cultivated bacterial cells to give pBlue-HsCOL1A1.

### (1-11) Cloning of Human Collagen Type I α2 Gene (Construction of pUC18―HsCOL1A2)

Total RNA derived from Human Neonatal Dermal Fibroblasts was obtained from Cell Applications, Inc. cDNA was synthesized using ReverTra Plus (Toyobo Co., Ltd.). The following oligonucleotides 54 and 55 were synthesized. PCR, was conducted using oligonucleotides 54 and 55 as primers and the cDNA as a template to amplify DNA encoding human collagen Type I α2.
(a) Oligonucleotide 54: GCCAAGCTTGCATGCTCAGCTTTGTGGATACGCGGAC (SEQ ID NO: 74)
(b) Oligonucleotide 55: CGGTACCCGGGGATCCTTATTTGAAACAGACTGGGCCAATGTCC (SEQ ID NO: 75)
The composition of the reaction solutions is given as follows:

| | | |
|---|---|---|
| (a) | cDNA solution | 5 µl |
| (b) | dNTP (2 mM―mix each) | 5 µl |
| (c) | MgSO₄ (25 mM) | 2 µl |
| (d) | Primers (10 pmol/µl) | 1.5 µl each |
| (e) | 10 x PCR buffer for KOD-plus- (Toyobo Co., Ltd.) | 5 µl |
| (f) | KOD-plus- DNA polymerase (1 U/µl, Toyobo Co., Ltd.) | 1 µl |
| (g) | Sterile distilled water | 29 µl |

PCR was conducted by heating the reaction solution at 94°C for 2 minutes before 30 cycles of denaturation at 98°C for 10 seconds, annealing and extension at 68°C for 6 minutes. The amplified DNA of approximately 4.1 kb was isolated by agarose gel electrophoresis, and then extracted and purified from the gel with MagExtractor PCR&Gel Clean up (Toyobo Co., Ltd.) The resulting DNA was cut with the restriction enzymes SphI and BamHI, and then purified using MagExtractor PCR&Gel Clean up (Toyobo Co., Ltd.)
pUC18 Plasmid (Toyobo Co., Ltd.) was cut with the restriction enzymes SphI and BamHI, and then purified using MagExtractor PCR&Gel Clean up Toyobo Co., Ltd.)
The about 4.1 kb and the plasmid were Ligated, and the resulting ligation solution was used for the transformation of E. coli (Competent high DH5α, Toyobo Co., Ltd.). For the ligation, Ligation high (Toyobo Co., Ltd.) was used.
On LB agar medium containing 50 µg/ml of ampicillin, the transformed E. coli was inoculated to cultivate it. A colony formed on the agar medium was inoculated into sterile LB medium containing 50 µg/ml of ampicillin and incubated in the test tube with shaking (37°C, 17 hours). Then, using MagExtractor plasmid (Toyobo Co., Ltd.), the plasmid into which the DNA encoding human collagen Type I α2 was inserted (hereinafter, it may be referred to as pUC18-HsCOL1A2) was isolated from the cultivated bacterial cells to give pUC18― HsCOL1A2.

### Example 2 (Construction of Gene Transfer Plasmid)

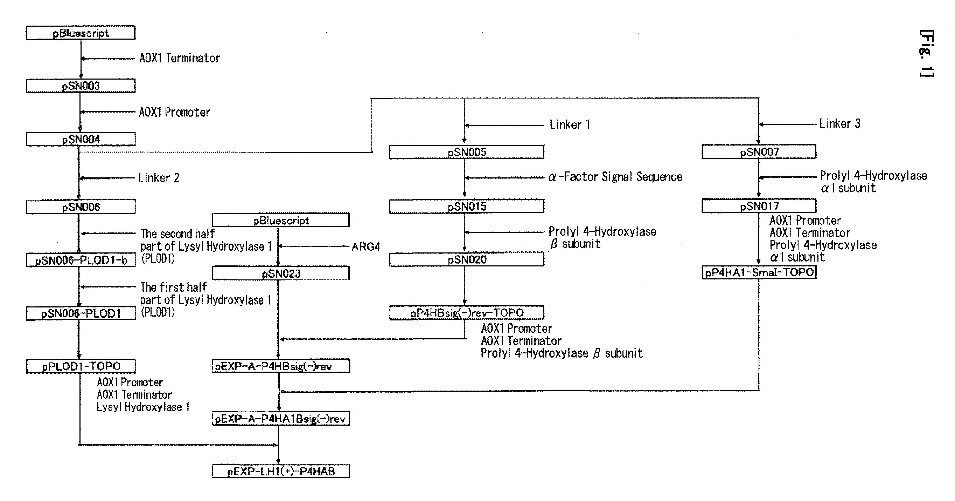

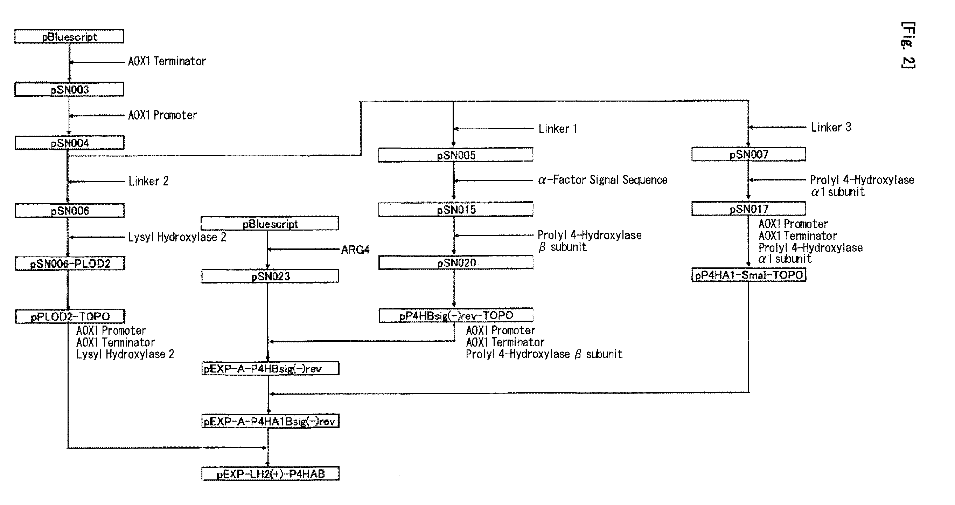

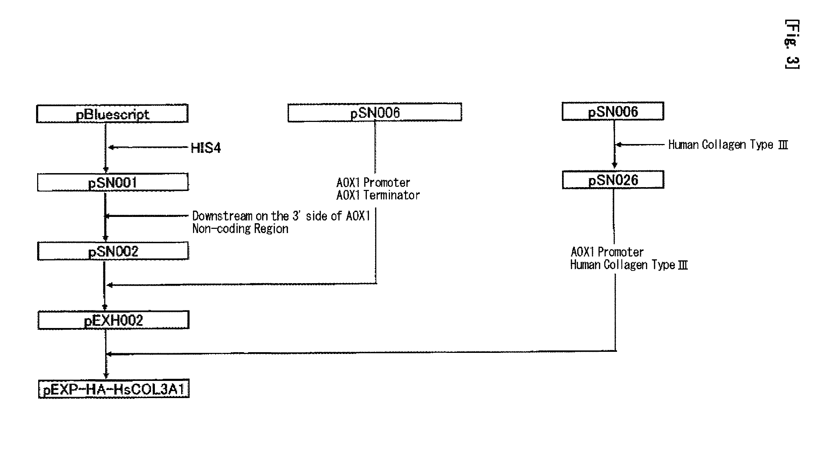

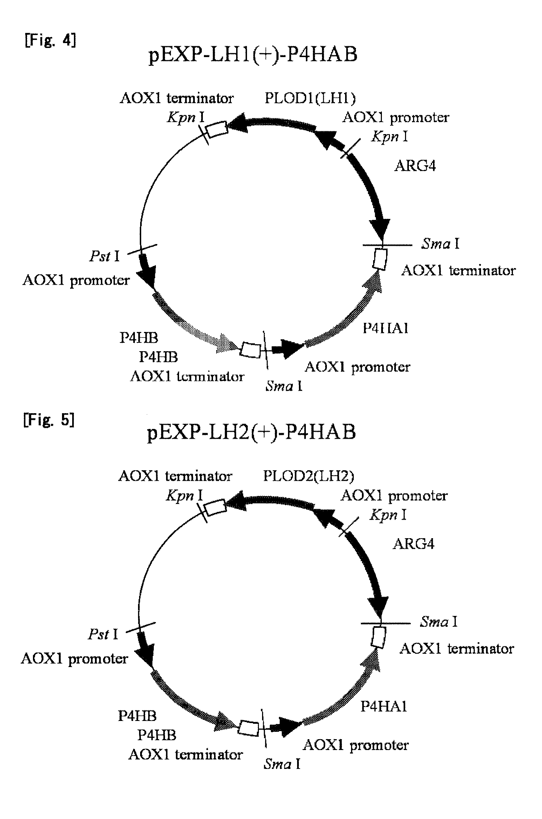

The details of a method for producing a Gene Transfer Plasmid will be described below. Fig. 1 shows a flow chart schematically illustrating a construction process of a plasmid for transfection of a prolyl 4-hydroxylase α1 subunit gene, a β subunit of a prolyl 4―hydroxylase gene and a lysyl Hydroxylase 1 gene; Fig. 2 shows a flow chart schematically illustrating a construction process of a plasmid for transfection of a prolyl 4-hydroxylase αl subunit gene, a β subunit of a prolyl 4-hydroxylase gene and lysyl hydroxylase 2 gene; and Fig. 3 shows a flow chart schematically illustrating a construction process of a plasmid for transfection of a human collagen Type III gene. Further, Fig. 4 illustrates a structure of the plasmid for transfection of a prolyl 4-hydroxylase α1 subunit gene, a β subunit of a prolyl 4-hydroxylase gene and a lysyl hydroxylase 1 gene; and Fig. 5 illustrates a structure of the plasmid for transfection of a prolyl 4-hydroxylase α1 subunit gene, a β subunit of a prolyl 4-hydroxylase gene and lysyl hydroxylase 2 gene.

### (2―1) Construction of pSN003

Oligonucleotides 19 and 20 were synthesized. The oligonucleotides 19 and 20 are oligonucleotides consisting of the nucleotide sequence of AOX1 terminator and the nucleotide sequence of the restriction enzyme site. The double-stranded DNA fragment was amplified by PCR using the following oligonucleotides 19 and 20 as primers and the pAOX1term―TOPO as a template.
(a) Oligonucleotide 19: TCGACTAGTTTAGACATGACTGTTCCTCAGTTCAA (SEQ ID NO: 19)
(b) Oligonucleotide 20: AACTGCAGGCACAAACGAACGTCTCACTTA (SEQ ID NO: 20)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM―mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD―plus― DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 2 minutes followed by 25 cycles of denaturation at 94°C for 15 seconds, annealing at 60°C for 30 seconds and extensione at 68°C for 1 minute, and then maintaining the reaction solution at 68°C for 5 minutes.
The resulting double-stranded DNA fragment from the PCR was cut with the restriction enzymes SpeI and PstI to isolate and purify the about 300 bp double-stranded DNA fragment comprising AOX1 terminator by means of agarose gel electrophoresis.
A pBluescriptII SK(+) plasmid purchased from Stratagene was cut with the restriction enzymes SpeI and PstI, and then subjected to agarose gel electrophoresis to isolate and purify the about 3000bp double-stranded DNA fragment.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN003 hereinafter.

### (2-2) Construction of pSN004

The pAOX1pro―TOPO was cut with the restriction enzyme Eco52I to isolate and purify the about 1000bp double-stranded DNA fragment comprising AOX1 promoter by means of agarose gel electrophoresis.
The pSN003 was cut with the restriction enzyme Eco52I and then dephosphorylated using alkaline phosphatase.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN004 hereinafter.

### (2-3) Construction of pSN005

Oligonucleotides 21 and 22 shown by the following nucleotide sequences which are complementary each other were synthesized:
(a) Oligonucleotide 21: TATTCGAAACGCATATGTGACCGGCAGACTAGTGG (SEQ ID NO: 21)
(b) Oligonucleotide 22: CCACTAGTCTGCCGGTCACATATGGGTTTCGAATA (SEQ ID NO: 22)
These oligonucleotides 21 and 22 were mixed as shown in the following composition, and then the mixed solution was incubated at 98°C for 5 minutes, 50°C for 50 minutes and then 37°C for 1 hour.

| | | |
|---|---|---|
| | Oligonucleotide 21 (50 pmol/ul) | 5 µl |
| | Oligonucleotide 22 (50 pmol/µl) | 5 µl |
| (a) | Tris-HCl (100 mM) | 10 µl |
| (b) | MgCl₂ (100 mM) | 10 µl |
| (c) | Dithiothreitol (10 mM) | 10 µl |
| (d) | Sterile distilled water | 60 µl |

The double-stranded DNA fragment prepared by annealing the oligonucleotides 21 and 22 was cut with the restriction enzymes BspT104I and SpeI. Next, the mixed solution was extracted with phenol: chloroform: isoamyl alcohol (25:24:1) and then subjected to ethanol precipitation to purify double-stranded DNA fragment (linker―1).
Also, pSN004 was cut with the restriction enzymes BspT104I and SpeI to isolate and purify the about 4300bp double-stranded DNA fragment by means of agarose gel electrophoresis.
The double-stranded DNA fragment and the plasmid were ligated, and the resulting ligation solution was used for the transformation of E. coli (Competent high PH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN005 hereinafter.

### (2-4) Construction of pSN006

Oligonucleotides 23 and 24 shown by the following nucleotide sequences which are complementary each other were synthesized:
(a) Oligonucleotide 23: TATTCGAAACGCATATGGTACCGGCAGACTAGTGG (SEQ ID NO: 23)
(b) Oligonucleotide 24: CCACTAGTCGCCTAGGCGACATATGGTTTCGAATA (SEQ ID NO: 24)
These oligonucleotides 23 and 24 were mixed as shown in the following composition, and then the mixed solution was incubated at 98°C for 5 minutes, 50°C for 50 minutes and then 37°C for 1 hour.

| | | |
|---|---|---|
| (a) | Oligonucleotide 23 (50 pmol/µl) | 5 µl |
| (b) | Oligonucleotide 24 (50 pmol/µl) | 5 µl |
| (c) | Tris-HCl (100 mM) | 10 µl |
| (d) | MgCl₂ (100 mM) | 10 µl |
| (e) | Dithiothreitol (10 mM) | 10 µl |
| (f) | Sterile distilled water | 60 µl |

The aoubi-e-stranded DNA fragment prepared by annealing the oligonucleotides 23 and 24 was cut with the restriction enzymes 3spT104I and SpeI. Next, the mixed solution was extracted with phenol: chloroform: isoamyl alcohol (25:24:1) and then subjected to ethanol precipitation to purify double-stranded DNA fragment (linker―2).
Also, pSN004 was cut with the restriction enzymes BspT104I and SpeI to isolate and purify the about 4300bp double-stranded DNA fragment by means of agarose gel electrophoresis.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN006 hereinafter.

### (2-5) Construction of pSN007

Oligonucleotides 25 and 26 shown by the following nucleotide sequences which are complementary each other were synthesized:
(a) Oligonucleotide 25: TATTCGAAACGACGCGTGTCAGCTAGCACTAGTGC (SEQ ID NO: 25)
(b) Oligonucleotide 26: GCACTAGTGCTAGCTGACACGCGTCGTTTCGAATA (SEQ ID NO: 26)
These oligonucleotides 25 and 26 were mixed as shown in the following composition, and then the mixed solution was incubated at 98°C for 5 minutes, 50°C for 50 minutes and 37°C for 1 hour.

| | | |
|---|---|---|
| (a) | Oligonucleotide 25 (50 pmol/µl) | 5 µl |
| (b) | Oligonucleotide 26 (50 pmol/µl) | 5 µl |
| (c) | Tris-HCl (100 mM) | 10 µl |
| (d) | MgCl₂ (100 mM) | 10 µl |
| (e) | Dithiothreitol (10 mM) | 10 µl |
| (f) | Sterile distilled water | 60 µl |

The double-stranded DNA fragment prepared by annealing the oligonucleotides 25 and 26 was cut with the restriction enzymes BspT104I and SpeI. Next, the mixed solution was extracted with phenol: chloroform: isoamyl alcohol (25:24:1) and then subjected to ethanol precipitation to purify double-stranded DNA fragment (linker-3).
Also, pSN004 was cut with the restriction enzymes BspT104I and SpeI to isolate and purify the 4,300 bp double-stranded DNA fragment by means of agarose gel electrophoresis.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN007 hereinafter.

### (2-6) Construction of a Plasmid for transfection of Prolyl 4-Hydroxylase α1 Subunit Gene and Prolyl 4-Hydroxylase β Subunit Gene (pEXP-A-P4HBsig(―)Alrev)

### (2-6-1) Construction of a Plasmid comprising Prolyl 4-Hydroxylase α1 Subunit Expression Construct (pSN017)

cDNA Clone (Clone ID: 4797051) of human prolyl 4-hydroxylase α1 subunit gene was purchased from Invitrogen. Oligonucleotides 27 and 28 were synthesized. The oligonucleotides 27 and 28 are Oligonucleotides consisting of the nucleotide sequence of the prolyl 4-hydroxylase α1 subunit and the nucleotide sequence of the restriction enzyme site.
The double-stranded DNA fragment was amplified by PCR using the following oligonucleotides 27 and 28 as primers and the cDNA clone as a template.
(a) Oligonucleotide 27: TATTCGAAACGATGATCTGGTATATATTAATTATA (SEQ ID NO: 27)
(b) Oligonucleotide 28: TTGCTAGCTCATTCCAATTCTGACAACGTACAAGG (SEQ ID NO: 28)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD―plus― DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 10 cycles of denaturation at 94°C for 15 seconds, annealing at 60°C for 30 seconds and extension at 68°C for 2 minutes, and 15 cycles of denaturation at 94°C for 15 seconds, annealing and extension at 68°C for 2 minutes, and then maintaining the reaction solution at 68°C for 5 minutes.
The resulting double-stranded DNA fragment from the PCR was cut with the restriction enzymes BspT104I and NheI, and subjected to agarose gel electrophoresis to isolate and purify the target DNA fragment.
The pSN007 was cut with the restriction enzymes BspT104I and NheI, and then subjected to agarose gel electrophoresis to isolate and purify the about 4200bp double-stranded DNA fragment.
The double―stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 ug/ml of ampicillin, the transformed E. coli was inoculated to cultivate it. Ten (10) colonies were selected randomly from colonies formed on the agar medium, and E. coli forming each of them was inoculated into sterile LB medium (2 ml) containing 50 µg/ml of ampicillin and then incubated in the test tube with shaking (37°C, 17 hours). After that, using QIAprep Spin Miniprep Kit (Qiagen), the plasmid into which the target DNA fragment was inserted was isolated from the cultivated bacterial cells. The plasmid will be referred to as pSN017 hereinafter.

### (2-6-2) Construccion of Plasmid comprising ARG4 (pSN023)

ARG4 to which the restriction enzyme sites were ligated was cloned into a plasmid. Oligonucleotides 29 and 30 were synthesized. The oligonucleotides 29 and 30 are Oligonucleotides consisting of the nucleotide sequence of the ARG4 and the nucleotide sequence of the restriction enzyme site.
The double-stranded DNA fragment was amplified by PCR using the following oligonucleotides 29 and 30 as primers and the pARG4-TOPO as a template.
(a) Oligonucleotide 29: AACTCGAGACGAAAATATGGTACCTGCCCT (SEQ ID NO: 29)
(b) Oligonucleotide 30: CCATCGATACAGAGGTATCATCCAATGATTCC (SEQ ID NO: 30)
As a polymerase for the PCR, KOD―Plus― PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM―mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 10 cycles of denaturation at 94°C for 15 seconds, annealing at 60°C for 30 seconds and extension at 68°C for 2 minutes, and 15 cycles of denaturation at 94°C for 15 seconds, annealing and extension at 68°C for 2 minutes, and then maintaining the reaction solution at 68°C for 5 minutes.
The resulting double-stranded DNA fragment from the PCR was cut with the restriction enzymes XhoI and ClaI, and subjected to agarose gel electrophoresis to isolate and purify the about 2200bp double-stranded DNA fragment comprising ARG4.
A pBluescriptII SK(―) plasmid purchased from Stratagene was cut with the restriction enzymes XhoI and ClaI, and then subjected to agarose gel electrophoresis to isolate and purify the about 3000bp double-stranded DNA fragment.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 ug/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN023 hereinafter.

### (2-6-3) Construction of pSN015

Oligonucleotides 31 and 32 were synthesized. The oligonucleotides 31 and 32 are oligonucleotides consisting of the nucleotide sequence of the α―factor and the nucleotide sequence of the restriction enzyme site.
The double-stranded DNA fragment was amplified by PCR using the following oligonucleotides 31 and 32 as primers and the pα―factor―TOPO as a template.
(a) Oligonucleotide 31: GGTTCGAAACGATGAGATTTCCTTCAATTTTTACT (SEQ ID NO: 31)
(b) Oligonucleotide 32: TCGACTAGTAGCTTCAGCCTCTCTTTTATCC (SEQ ID NO: 32)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM―mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 10 cycles of denaturation at 94°C for 15 seconds, annealing at 60°C for 30 seconds and extension at 68°C for 1 minute, and 15 cycles of denaturation at 94°C for 15 seconds, annealing and extension at 68°C for 1 minute, and then maintaining the reaction solution at 68°C for 5 minutes.
The resulting double-stranded DNA fragment from the PCR was cut with the restriction enzymes BspT104I and SpeI, and subjected to agarose gel electrophoresis to isolate and purify the target DNA fragment.
The pSN005 was cut with the restriction enzymes BspT104I and SpeI, and then subjected to agarose gel electrophoresis to isolate and purify the about 4200bp double-stranded DNA fragment.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN015 hereinafter.

### (2-6-4) Construction of Plasmid comprising Prolyl 4-Hydroxylase β Subunit Expression Construct with α―Factor Signal (pSN020)

cDNA Clone (Clone ID: 3848651) of human β subunit of prolyl 4-hydroxylase gene was purchased from. Invitrogen. Oligonucleotides 33 and 34 were synthesized. The oligonucleotides 33 and 34 are oligonucleotides consisting of the nucleotide sequence of the β subunit of prolyl 4-hydroxylase gene and the nucleotide sequence of the restriction enzyme site.
The double-stranded DNA fragment was amplified by PCR using the following oligonucleotides 33 and 34 as primers and the cDNA clone as a template.
(a) Oligonucleotide 33: TTACTAGTGACGCCCCCGAGGAGGA (SEQ ID NO: 33)
(b) Oligonucleotide 34: TTACTAGTTTACAGTTCATCTTTCACAGCTTTCTG (SEQ ID NO: 34)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | DNTP (2 mM―mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 10 cycles of denaturation at 94°C for 15 seconds, annealing at 60°C for 30 seconds and extension at 68°C for 2 minutes, and 15 cycles of denaturation at 94°C for 15 seconds, annealing and extension at 68°C for 2 minutes, and then maintaining the reaction solution at 68°C for 5 minutes.
The resulting double-stranded DNA fragment from the PCR was cut with the restriction enzyme SpeI, and subjected to agarose gel electrophoresis to isolate and purify the target DNA fragment.
The pSN015 was cut with the restriction enzyme SpeI and then dephosphorylated by the use of alkaline phosphatase.
The double-stranded DNA fragment and the plasmid were ligated, and the resulting ligation solution was used for the transformation of E. coli (Competent high JM109, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN020 hereinafter.

### (2―6―5) Construction of a Plasmid for transfection of a Prolyl 4-Hydroxylase β Subunit gene (pEXP-A-P4HBsig(―)rev)

Oligonucleotides 35 and 36 were synthesized. The oligonucleotide 35 is the oligonucleotide consisiting of the nucleotide sequence of the AOX1 promoter and the nucleotide sequence of the restriction enzyme site, and the oligonucleotide 36 is the oligonucleotide consisting the nucleotide sequence of the AOX1 terminator and the nucleotide sequence of the restriction enzyme site.
The double―stranded DNA fragment was amplified by PCR using the following oligonucleotides 35 and 36 as primers and the pSN020 as a template.
(a) Oligonucleotide 35: AACCGCGGTCTAACATCCAAAGACGAAAGGTTGAA (SEQ ID NO: 35)
(b) Oligonucleotide 36: AACCCGGGGCACAAACGAACGTCTCACTTAATCTT (SEQ ID NO: 36)
As a polymerase for the PCR, KOD―Plus― PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM―mix each) | 5 µl |
| (c) | MgSO₄ (25 mM) | 2 µl |
| (d) | Primers (10 pmol/µl) | 1.5 µl each |
| (e) | 10 × PCR buffer for KOD―plus― | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 10 cycles of denaturation at 94°C for 15 seconds, annealing at 60°C for 30 seconds and extension at 68°C for 3.5 minutes, and 15 cycles of denaturation at 94°C for 15 seconds, annealing and extension at 68°C for 3.5 minutes, and then maintaining the reaction solution at 68°C for 5 minutes.
The double-stranded DNA fragment prepared by the PCR was ligated to the "PCR Product insertion site" of pCR-BluntII―TOPO plasmid, and the resulting ligation solution was used for the transformation of E. coli (Competent high JM109, Toyobo Co., Ltd.). For the ligation, Invitrogen ZERO-BluntII TOPO PCR cloning kit was used.
On LB agar medium containing 50 ug/ml of kanamycin, the transformed E. coli was inoculated to cultivate it. A colony formed on the agar medium was inoculated into sterile LB medium (2 ml) containing 50 µg/ml of kanamycin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pP4HBsig(―)rev―TOPO hereinafter.
The plasmid was cut with the restriction enzymes SacII and Cfr91, and subjected to agarose gel electrophoresis to isolate and purify the DNA fragment of the β subunit of prolyl 4-hydroxylase expression construct.
Further, the pSN0023 was cut with the restriction enzymes SacII and Cfr9I, and then subjected to agarose gel electrophoresis to isolate and purify the about 5200bp double-stranded DNA fragment.
The double-stranded DNA fragment and the plasmid were ligated, and the resulting ligation solution was used for the transformation of E. coli (Competent high JM109, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours) . Then, using QIAprep Spin Miniprep Kit (Qiagen), the plasmid into which the target DNA fragment was inserted was isolated from the cultivated bacterial cells. The plasmid will be referred to as pEXP-A-P4HBsig(-)rev hereinafter.

### (2-6-6) Introduction of Prolyl 4-Hydroxylase α1 Subunit Expression Construct into pEXP-A-P4HBsig(-)rev

Oligonucleotides 37 and 36 were synthesized. The oligonucleotide 37 is the oligonucleotide consisting of the nucleotide sequence of the AOX1 promoter and the nucleotide sequence of the restriction enzyme site, and the oligonucleotide 36 is the oligonucleotide consisting of the nucleotide sequence of the AOX1 terminator and the nucleotide sequence of the restriction enzyme site.
The double-stranded DNA fragment was amplified by PCR using the following oligonucleotides 37 and 36 as primers and the pSN017 as a template.
(a) Oligonucleotide 37: AACCCGGGTCTAACATCCAAAGACGAAAGGTTGAA (SEQ ID NO: 37)
(b) Oligonucleotide 36: AACCCGGGGCACAAACGAACGTCTCACTTAATCTT (SEQ ID NO: 36)
As a polymerase for the PCR, KOD-Plus- PCR polymerase, manufactured by Toyobo Co., Ltd., was used. The details of composition, of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 10 cycles of denaturation at 94°C for 15 seconds, annealing at 60°C for 30 seconds and extension at 68°C for 3.5 minutes, and 15 cycles of denaturation at 94°C for 15 seconds, annealing and extension at 68°C for 3.5 minutes, and then maintaining the reaction solution at 68°C for 5 minutes.
The double-stranded DNA fragment prepared by the PCR was ligated to the "PCR Product insertion site" of pCR-BluntII-TOPO plasmid, and the resulting ligation solution was used for the transformation of E. coli (Competent high DH5α, Toyobo Co., Ltd.). For the ligation, Invitrogen ZERO-BluntII TOPO PCR cloning kit was used.
On LB agar medium containing 50 µg/ml of kanamycin, the transformed E. coli was inoculated to cultivate it. A colony formed on the agar medium was inoculated into sterile LB medium (2 ml) containing 50 µg/ml of kanamycin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pP4HA1-SmaI-TOPO hereinafter.
The plasmid was cut with the restriction enzyme Cfr9I, and subjected to agarose gel electrophoresis to isolate and purify the DNA fragment of the prolyl 4-hydroxylase α1 subunit expression construct.
Further, the pEXP-A-P4HBsig(-)rev was cut with the restriction enzyme Cfr9I and then dephosphorylated by the use of alkaline phosphatase.
The double-stranded DNA fragment and the plasmid were ligated, and the resulting ligation solution was used for the transformation of E. coli (Competent high JM109, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pBXP-A-P4HBsig(-)A1rev hereinafter.

### (2-7) Construction of a Plasmid for transfection of Prolyl 4-Hydroxylase α1 Subunit Gene, Prolyl 4-Hydroxylase β Subunit Gene and Lysyl Hydroxylase 1 Gene (pEXP-LH1 (+) -P4HAB)

A method for producing a plasmid pEXP-LH1 (+) - P4HAB into which prolyl 4-hydroxylase α1 subunit gene, β subunit of prolyl 4-Hydroxylase gene and a lysyl hydroxylase 1 gene (PLOD1) were inserted will be described below.

### (2-7-1) Construction of Plasmid comprising Lysyl Hydroxylase 1 Expression Construct (pSN006-PLOD1)

A DNA fragment encoding the second half part of lysyl hydroxylase 1 was introduced into the pSN006. Oligonucleotides 38 and 39 were synthesized. The oligonucleotides 38 and 39 are oligonucleotides consisting of the nucleotide sequence of the lysis hydroxylase 1 and the nucleotide sequence of the restriction enzyme site.
The double-stranded DNA fragment was amplified by PCR using the following oligonucleotides 38 and 39 as primers and the pPLOD1-TOPO as a template.
(a) Oligonucleotide 38: GACCTTCGAAACAGGCTGCACCGT (SEQ ID NO: 38)
(b) Oligonucleotide 39: CGACTAGTTTAGGGATCGACGAA (SEQ ID NO: 39)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distillled water | 33 µl |

PCR was conducted using PERKIN ELMER GeneAmp PCR System 9700. This reaction was conducted under the following conditions: heating the reaction solution at 94°C for 2 minutes followed by 35 cycles of denaturation at 94°C for 15 seconds, annealing at 60°C for 30 seconds and extension at 68°C for 1.5 minutes, and then maintaining the reaction solution at 68°C for 5 minutes.
The resulting double-stranded DNA fragment from the PCR was cut with the restriction enzymes BspT104I and SpeI, and subjected to agarose gel electrophoresis to isolate and purify the target DNA fragment.
The pSN006 was cut with the restriction enzymes BspT104I and SpeI, and then subjected to agarose gel Electrophoresis to isolate and purify the about 4200bp double-stranded DNA fragment.
The double-stranded DNA fragment and the plasmid were ligated, and the resulting ligation solution was used for the transformation of E. coli (Competent high JM109, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN006-PLOD1-b hereinafter.

Then, a DNA fragment encoding the first half part of lysyl hydroxylase 1 was introduced into the pSN006-PLOD1-b. Oligonucleotides 40 and 41 were synthesized. The oligonucleotides 40 and 41 are oligonucleotides consisting of the nucleotide sequence of the lysis hydroxylase 1 and the nucleotide sequence of the restriction enzyme site. The double-stranded DNA fragment was amplified by PCR using the following oligonucleotides 40 and 41 as primers and the pPLOD1-TOPO as a template.
(a) Oligonucleotide 40: TATTCGAAACGATGCGGCCCCTGCTGCTAC (SEQ ID NO: 40)
(b) Oligonucleotide 41: CAGCCTGTTTCGAAGGTCCAGAAGCGCG (SEQ ID NO: 41)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 2 minutes followed by 35 cycles of denaturation at 94°C for 15 seconds, annealing at 60°C for 30 seconds and extension at 68°C for 1 minute, and then maintaining the reaction solution at 68°C for 5 minutes.
The resulting double-stranded DNA fragment from the PCR was cut with the restriction enzyme BspT104I, and subjected to agarose gel electrophoresis to isolate and purify the about 800bp target DNA fragment.
The pSN006-PLOD1-b was cut with the restriction enzyme BspT104I, and then the plasmid was dephosphorylated by the use of alkaline phosphatase.
The double-stranded DNA fragment and the plasmid were ligated, and the resulting ligation solution was used for the transformation of E. coli (Competent high JM109, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN006-PLOD1 hereinafter.

### (2-7-2) Construction of pEXP-LH1(+)-P4HAB

Oligonucleotides 42 and 43 were synthesized. The oligonucleotide 43 is the oligonucleotide consisting of the nucleotide sequence of the AOX1 promoter and the nucleotide sequence of the restriction enzyme site, and the oligonucleotide is the oligonucleotide consisting of the nucleotide sequence of the AOX1 terminator and the nucleotide sequence of the restriction enzyme site.
The double-stranded DNA fragment was amplified by PCR using the following oligonucleotides 42 and 43 as primers and the pSN006-PLOD1 as a template.
(a) Oligonucleotide 42: AAGGTACCTCTAACATCCAAAGACGAAAGGT (SEQ ID NO: 42)
(b) Oligonucleotide 43: AAGGTACCGCACAAACGAACGTCTCACTTA (SEQ ID NO: 43)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 35 cycles of denaturation at 94°C for 15 seconds, annealing at 60°C for 30 seconds and extension at 68°C for 3.5 minutes, and then maintaining the reaction solution at 68°C for 5 minutes.
The double-stranded DNA fragment prepared by the PCR was ligated to the "PCR Product insertion site" of pCR-BluntII-TOPO plasmid, and the resulting ligation solution was used for the transformation of E. coli (Competent high JM105, Toyobo Co., Ltd.). For the ligation, Invitrogen ZERO-BluntII TOPO PCR cloning kit was used.
On LB agar medium containing 50 µg/ml of kanamycin, the transformed E. coli was inoculated to cultivate it. A colony formed on the agar medium was inoculated into sterile LB medium (2 ml) containing 50 µg/ml of kanamycin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pPLOD1-TOPO hereinafter.

The plasmid was cut with the restriction enzyme KpnI, and subjected to agarose gel electrophoresis to isolate and purify the DNA fragment of the lysyl hydroxylase 1 expression construct.
And, the pEXP-A-P4HBsig(-)A1rev was cut with the restriction enzyme KpnI and then dephosphorylated by the use of alkaline phosphatase.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells.
The isolated plasmid was cut with the restriction enzyme BlnI and subjected to agarose gel electrophoresis, and then, it was found that the target fragment was inserted into the resulting plasmid. The plasmid in which the lysyl hydroxylase 1 expression construct is suited to the same direction as the selective marker ARG4 gene will be referred to as pEXP-LH1(+) -P4HAB.

### (2-8) Construction of Plasmid obtained by expressing Prolyl 4-Hydroxylase α1 Subunit Gene, Prolyl 4-Hydroxylase β Subunit Gene and Lysyl Hydroxylase 2 Gene (pEXP-LH2 (+) -P4HAB)

A method for producing a plasmid pEXP-LH2 (+) -P4HAB into which prolyl 4-hydroxylase α1 subunit gene, β subunit of prolyl 4-hydroxylase gene and lysyl hydroxylase 2 gene (PLOD2) were inserted will be described below.

### (2-8-1) Construction of Plasmid comprising Lysyl Hydroxylase 2 Expression Construct (pSN006-PLOD2)

A DNA fragment encoding lysyl hydroxylase 2 was introduced into the pSN006. Oligonucleotides 44 and 45 were synthesized. The oligonucleotides 44 and 45 are oligonucleotides consisting of the nucleotide sequence of the lysis hydroxylase 2 and the nucleotide sequence of the restriction enzyme site.
The double-stranded DNA fragment was amplified by PCR using the following oligonucleotides 44 and 45 as primers and the pPLOD2-TOPO as a template.
(a) Oligonucleotide 44: TATTCGAAACGATGGGGGGATGCACGGTG (SEQ ID NO: 44)
(b) Oligonucleotide 45: CGACTAGTTTAGGGATCTATAAATGCACTG (SEQ ID NO: 45)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 2 minutes followed by 35 cycles of denaturation at 94°C for 15 seconds, annealing at 60°C for 30 seconds and extension at 68°C for 2.5 minutes, and then maintaining the reaction solution at 68°C for 5 minutes.
The resulting double-stranded DNA fragment from the PCR was cut with the restriction enzymes BspT104I and SpeI, and subjected to agarose gel electrophoresis to isolate and purify the about 2300bp target DNA fragment.
The plasmid pSN006 was also cut with the restriction enzymes BspT104I and SpeI, and then subjected to agarose gel electrophoresis to isolate and purify the about 4200bp double-stranded DNA fragment.
The double-stranded DNA fragment and the plasmid were ligated, and the resulting ligation solution was used for the transformation of E. coli (Competent high JM109, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN006-PLOD2 hereinafter.

### (2-8-2) Construction of pEXP-LH2(+)-P4HAB

Oligonucleotides 42 and 43 were synthesized. The oligonucleotide 42 is the oligonucleotide consisting of the nucleotide sequence of the AOX1 promoter and the nucleotide sequence of the restriction enzyme site, and the oligonucleotide 43 is the oligonucleotide consisting of the nucleotide sequence of the AOX1 terminator and the nucleotide sequence of the restriction enzyme site.
The double-stranded DNA fragment was amplified by PCR using the following oligonucleotides 42 and 43 as primers and the pSN006-PLOD2 as a template.
(a) Oligonucleotide 42: AAGGTACCTCTAACATCCAAAGACGAAAGGT (SEQ ID NO: 42)
(b) Oligonucleotide 43: AAGGTACCGCACAAACGAACGTCTCACTTA (SEQ ID NO: 43)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PER System 9700. This reaction was conducted under the following conditions: heating the reaction solution at 94°C for 3 minutes followed by 10 cycles of denaturation at 94°C for 15 seconds, annealing at 60°C for 30 seconds and extension at 68°C for 3.5 minutes, and 15 cycles of denaturation at 94°C for 15 seconds, annealing and extension at 68°C for 3.5 minutes, and then maintaining the reaction solution at 68°C for 5 minutes.
The double-stranded DNA fragment prepared by the PCR was ligated to the "PCR Product insertion site" of pCR-BluntII-TOPO plasmid, and the resulting ligation solution was used for the transformation of E. coli (Competent high DH5α, Toyobo Co., Ltd.). For the ligation, Invitrogen ZERO-BluntII TOPO PCR cloning kit was used.
On LB agar medium containing 50 µg/ml of kanamycin, the transformed E. coli was inoculated to cultivate it. A colony formed on the agar medium was inoculated into sterile LB medium (2 ml) containing 50 µg/ml of kanamycin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pPLOD2-TOPO hereinafter.

The plasmid was cut with the restriction enzyme KpnI, and subjected to agarose gel electrophoresis to isolate and purify the DNA fragment of the lysyl hydroxylase 2 expression construct.
And, the pEXP-A-P4HBsig(-)Alrev was cut with the restriction enzyme KpnI and then dephosphorylated by the use of alkaline phosphatase.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells.
The isolated plasmid was cut with the restriction enzyme EcoRV and subjected to agarose gel electrophoresis, and then, it was found that the target fragment was inserted into the resulting plasmid. The plasmid in which the lysyl hydroxylase 2 expression construct is suited to the same direction as the selective marker ARG4 gene will be referred to as pEXP-LH2(+)-P4HAB.

### (2-9) Construction of a Plasmid for transfection of Human Collagen Type III Gene

### (2-9-1) Construction of pSN001

HIS4 to which the restriction enzyme sites were ligated was cloned into plasmid. Oligonucleotides 46 and 47 were synthesized. The oligonucleotides 46 and 47 are oligonucleotides consisting of the nucleotide sequence of the HIS4 and the nucleotide sequence of the restriction enzyme site.
The double-stranded DNA fragment was amplified by PCR using then following oligonucleotides 46 and 47 together with the pHIS4-TOPO as a template.
(a) Oligonucleotide 46: GGAAGCTTGATCTCCTGATGACTGACTCACTG (SEQ ID NO: 46)
(b) Oligonucleotide 47: CCCTGCAGTAATTAAATAAGTCCCAGTTTCTCCA (SEQ ID NO: 47)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 5 cycles of denaturation at 94°C for 15 seconds, annealing at 60°C for 30 seconds and extension at 68°C for 3 minutes, and 20 cycles of denaturation at 94°C for 15 seconds, annealing and extension at 68°C for 3 minutes, and then maintaining the reaction solution at 68°C for 5 minutes.
The resulting double-stranded DNA fragment from the PCR was cut with the restriction enzymes HindIII and PstI, and subjected to agarose gel electrophoresis to isolate and purify the about 2600bp double-stranded DNA fragment.
A pBluescriptII SK(+) plasmid purchased from Stratagene was cut with the restriction enzymes HindIII and PstI, and then subjected to agarose gel electrophoresis to isolate and purify the about 3000bp double-stranded DNA fragment.
The double-stranded DNA fragment and the plasmid were ligated, and the resulting ligation solution was used for the transformation of E. coli (Competent high DH5α, (Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN001 hereinafter.
The nucleotide sequence of the DNA fragment which was inserted into the plasmid was analyzed in accordance with the known techniques, and then, it was found to be in good agreement with the nucleotide sequence of HIS4 on a database.

### (2-9-2) Construction of pSN002

Oligonucleotide 48 and oligonucleotide 49 were synthesized. The oligonucleotides 48 and 49 are oligonucleotides consisting of the nucleotide sequence of the 3'-downstream non-coding region of AOX1 and the nucleotide sequence of the restriction enzyme site.
The double-stranded DNA fragment was amplified by PCR using the following oligonucleotides 48 and 49 as primers and the pAOX1-3'-TOPO as a template.
(a) Oligonucleotide 48: GCATCGATTCGAGTATCTATGATTGGAAGTATGG (SEQ ID NO: 48)
(b) Oligonucleotide 49: AAGGGCCCGATCTTGAGATAAATTTCACGTTTAAA (SEQ ID NO: 49)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP ·(2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 5 cycles of denaturation at 94°C for 15 seconds, annealing at 60°C for 30 seconds and extension at 68°C for 1 minute, and 20 cycles of denaturation at 94°C for 15 seconds, annealing and extension at 68°C for 1 minutes, and then maintaining the reaction solution at 68°C for 5 minutes.
The resulting double-stranded DNA fragment from the PCR was cut with the restriction enzymes ApaI and ClaI, and subjected to agarose gel electrophoresis to isolate and purify the about 700bp double-stranded DNA fragment.
The plasmid pSN001 was cut with the restriction enzymes ApaI and ClaI, and then subjected to agarose gel electrophoresis to isolate and purify the about 5600bp double-stranded DNA fragment.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN002 hereinafter.

### (2-9-3) Construction of pEXH002

The pSN006 was cut with the restriction enzymes PstI and Eco52I, and subjected to agarose gel electrophoresis to isolate and purify the about 1300bp double-stranded DNA fragment encoding AOX1 promoter/terminator expression units.
The SN002 was cut with the restriction enzymes PstI and Eco52I, and subjected to agarose gel electrophoresis to isolate and purify the about 6300bp double-stranded DNA fragment.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pEXH002 hereinafter.

### (2-9-4) Construction of pSN026

Oligonucleotides 50 and 51 were synthesized. The oligonucleotides 50 and 51 are oligonucleotides consisting of the nucleotide sequence of human collagen Type III and the nucleotide sequence of the restriction enzyme site.
The double-stranded DNA fragment was amplified by PCR using the following oligonucleotides 50 and 51 as primers and the pUC19-COL3A1 as a template.
(a) Oligonucleotide 50: TATTCGAAACGATGATGAGCTTTGTGCAAAAGGGG (SEQ ID NO: 50)
(b) Oligonucleotide 51: TTACTAGTTTATAAAAAGCAAACAGGGCCAACGT (SEQ ID NO: 51)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 94°C for 3 minutes followed by 5 cycles of denaturation at 94°C for 15 seconds, annealing at 60°C for 30 seconds and extension at 68°C for 5 minutes, and 20 cycles of denaturation at 94°C for 15 seconds, annealing and extension at 68°C for 5 minutes, and then maintaining the reaction solution at 68°C for 5 minutes.
The resulting double-stranded DNA fragment from the PCR was cut with the restriction enzymes BspT104I and SpeI, and subjected to agarose gel electrophoresis to isolate and purify the about 4400bp double-stranded DNA fragment encoding human Type III collagen.
The pSN006 was cut with the restriction enzymes BspT104I and ClaI, and then subjected to agarose gel electrophoresis to isolate and purify the about 4200bp double-stranded DNA fragment.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (374°C, 17 hours). Then, using QIAprep Spin Miniprep Kit (Qiagen), the plasmid into which the target DNA fragment was inserted was isolated from the cultivated bacterial cells. The plasmid will be referred to as pSN026 hereinafter.

### (2-9-5) Construction of pEXE-HA-HsCOL3A1

The pSN026 was cut with the restriction enzymes Eco52I and SpeI, and then subjected to agarose gel electrophoresis to isolate and purify the about 5300bp DNA fragment encoding both human Type III collagen and AOX1 promoter.
The pEXH002 was cut with the restriction enzymes Eco52I and SpeI, and then subjected to agarose gel electrophoresis to isolate and purify the about 6700bp double-stranded DNA fragment.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pEXP-HA-HsCOL3A1.

### (2-10) Construction of a Plasmid for Transfection of Human Collagen Type I α1 Gene and Human Collagen Type I α2 Gene (pEXP-HA-HsCOL1A2-1A1)

### (2-10-1) Construction of pSN025

Oligonucleotides 56 and 57 were synthesized. The oligonucleotides 56 and 57 are oligonucleotides consisting of the nucleotide sequence of the human collagen Type I α1 and the nucleotide sequence of the restriction enzyme site. The double-stranded DNA fragment was amplified by PCR using the following oligonucleotides 56 and 57 as primers and the pBlue-HsCOL1A1 (see Example (1-10)) as a template.
(a) Oligonucleotide 56: TATTCGAAACGATGTTCAGCTTTGTGGACCTCCG (SEQ ID NO: 76)
(b) Oligonucleotide 57: TTACTAGTTTACAGGAAGCAGACAGGGCCAA (SEQ ID NO: 77)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 98°C for 2 minutes followed by 5 cycles of denaturation at 98°C for 15 seconds, annealing at 60°C for 30 seconds and extension at 68°C for 5 minutes, and 23 cycles of denaturation at 98°C for 15 seconds, annealing and extension at 68°C for 5 minutes, and then maintaining the reaction solution at 68°C for 5 minutes.
The resulting double-stranded DNA fragment from the PCR was cut with the restriction enzymes BspT104I and SpeI, and subjected to agarose gel electrophoresis to isolate and purify the about 4400bp double-stranded DNA fragment encoding human Type I α1.
A pSN006 (see Example (2-4)) was cut with the restriction enzymes BspT104I and SpeI, and then subjected to agarose gel electrophoresis to isolate and purify the about 4200bp double-stranded DNA fragment.
The double-stranded DNA fragment and the plasmid were ligated, and the resulting ligation solution was used for the transformation of E. coli (Competent high DH5α, (Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN025 hereinafter.

### (2-10-2) Construction of pEXP-HA-HsCOL1A1

The pSN025 (see Example (2-10-1)) was cut with the restriction enzymes EcoT22I, SspI and SpeI, and subjected to agarose gel electrophoresis to isolate approximately 4.5 kb of AOX1 promoter and the DNA encoding human collagen Type I α1, and then extracted and purified from the gel with MinElute Gel Extraction Kit (QIAGEN).
The plasmid named as pEXH002 (see Example (2-9-3)) was cut with the restriction enzymes EcoT22I and SpeI to isolate approximately 7.5 kb of DNA using agarose gel electrophoresis, and then extracted and purified from the gel using MinElute Gel Extraction Kit (QIAGEN).
The about 4.5kb DNA and the about 7.5kb DNA were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium containing 50 µg/ml of ampicillin and incubated in the test tube with shaking (37°C, 17 hours). Then, using QIAprep Spin Miniprep Kit (Qiagen), the plasmid into which the AOX1 promoter and the DNA encoding human collagen Type I α1 were inserted (which may be referred to as pEXP-HA-HsCOL1A1 hereinafter) was isolated from the cultivated bacterial cells to give pEXP-HA-HsCOL1A1.

### (2-10-3) Construction of pSN029

Oligonucleotides 58 and 59 were synthesized. The oligonucleotides 58 and 59 are Oligonucleotides consisting of the nucleotide sequence of the human collagen Type I α2 and the nucleotide sequence of the restriction enzyme site. The double-stranded DNA fragment was amplified by PCR using the following oligonucleotides 58 and 59 as primers and the pUC18-HsCOL1A2 (see Example (1-11)) as a template.
(a) Oligonucleotide 58: TATTCGAAACGATGCTCAGCTTTGTGGATACGCG (SEQ ID NO: 78)
(b) Oligonucleotide 59: TTACTAGTTTATTTGAAACAGACTGGGCCAATGTC (SEQ ID NO: 79)
As a polymerase for the PCR, KOD-Plus- PCR polymerase, manufactured by Toyobo Co., Ltd., was used. The details of composition of the reaction solutions are given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted using PERKIN ELMER GeneAmp PCR System 9700. This reaction was conducted under the following conditions: heating the reaction solution at 98°C for 2 minutes followed by 5 cycles of denaturation at 98°C for 15 seconds, annealing at 60°C for 30 seconds and extension at 68°C for 5 minutes, and 23 cycles of denaturation at 98°C for 15 seconds, annealing and extension at 68°C for 5 minutes, and then maintaining the reaction solution at 68°C for 5 minutes.
The resulting doublestranded DNA fragment from the PCR was cut with the restriction enzymes BspT104I and SpeI, and subjected to agarose gel electrophoresis to isolate and purify the about 4100bp double-stranded DNA fragment encoding human Type I α1.
The pSN006 (see Example (2-4)) was cut with the restriction enzymes BspT104I and SpeI, and then subjected to agarose gel electrophoresis to isolate and purify the about 4200bp double-stranded DNA fragment.
The double-stranded DNA fragment and the plasmid were ligated, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pSN029 hereinafter.

### (2-10-4) Construction of pHsCOL1A2 Exp unit-Eco52I

Oligonucleotides 60 and 61 were synthesized. PCR was conducted using the following oligonucleotides 60 and 61 as primers and the plasmid named as pSN029 (see Example (2-10-3)) as a template to amplify the DNA wherein the sequence of restriction enzyme sites was added to both end of the human collagen Type I α2 expression cassette.
(a) Oligonucleotide 60: AACGGCCGTCTAACATCCAAAGACGAAAGGTTGAA (SEQ ID NO: 80)
(b) Oligonucleotide 61: AACGGCCGGCACAAACGAACGTCTCACTTAATCTT (SEQ ID NO: 81)
The composition of the reaction solutions is given as follows:

| | | |
|---|---|---|
| (a) | Plasmid solution (10 ng/µl) | 1 µl |
| (b) | dNTP (2 mM-mix each) | 5 µl |
| (c) | MgSO₄ (25 mM) | 2 µl |
| (d) | Primers (10 pmol/µl) | 1.5 µl each |
| (e) | 10 × PCR buffer for KOD-plus- | 5 µl |
| (f) | KOD-plus- DNA polymerase (1 U/µl) | 1 µl |
| (g) | Sterile distilled water | 33 µl |

PCR was conducted under the following conditions: heating the reaction solution at 94°C for 3 minutes followed by 5 cycles of denaturation at 94°C for 15 seconds, annealing at 60°C for 30 seconds and extension at 68°C for 6 minutes, and 18 cycles of denaturation at 94°C for 15 seconds, annealing and extension at 68°C for 6 minutes, and then maintaining the reaction solution at 68°C for 5 minutes.
Approximately 5.4 kb of the double-stranded DNA fragment resulted from the PCR was isolated by agarose gel electrophoresis, and then extracted and purified from the gel using MinElute Gel Extraction Kit (QIAGEN). The about 5.4kb of DNA was ligated to the "PCR Product insertion site" of pCR-BluntII-TOPO plasmid (Invitrogen), and the resulting ligation solution was used for the transformation of E. coli (Competent high DH5α, Toyobo Co., Ltd.). For the ligation, Invitrogen Zero-Blunt TOPO PCR cloning kit was used.
On LB agar medium containing 50 µg/ml of kanamycin, the transformed E. coli was inoculated to cultivate it. A colony formed on the agar medium was inoculated into LB medium containing 50 µg/ml of kanamycin and incubated in the test tube with shaking (37°C, 17 hours). Then, using QIAprep Spin Miniprep Kit (Qiagen), the plasmid (which may be referred to as pHsCOL1A2 Exp unit-Eco52I hereinafter) to which the DNA with addition of the recognition sequences of restriction enzyme site on both ends of the human collagen Type I α2 expression cassette was inserted was isolated to give the pHsCOL1A2 Exp unit-Eco52I.

### (2-10-5) Construction of pEXP-HA-HsCOL1A2-1A1

The plasmid named as pHsCOL1A2 Exp unit-Eco52I (see Example (2-10-4)) was cut with the restriction enzymes Eco52I, and subjected to agarose gel electrophoresis to isolate approximately 5.4 kb of DNA wherein the recognition sequences of restriction enzyme site were added to both ends of the human collagen Type I α2 expression cassette, and then extracted and purified from the gel using MinElute Gel Extraction Kit (QIAGEN).
The plasmid named as pEXP-HA-HsCOL1A1 (see Example (2-10-2)) was cut with the restriction enzymes Eco52I and dephosphorylated by the use of alkaline phosphatase, followed by purification using MinElute Reaction Cleanup Kit (QIAGEN).
The about 5.4kb of DNA was ligated to the plasmid, and the resulting ligation solution was used for the transformation of E. coli (Competent high DH5α, Toyobo Co., Ltd.). For the ligation, Takara Ligation Kit ver2.1 was used.
On LB agar medium containing 50 µg/ml of ampicillin, the transformed E. coli was inoculated to cultivate it. A colony formed on the agar medium was inoculated into LB medium containing 50 µg/ml of ampicillin and incubated in the test tube with shaking (37°C, 17 hours). Then, using QIAprep Spin Miniprep Kit (Qiagen), the plasmid into which the DNA with the addition of the sequence of restriction enzyme sites to both ends of the human collagen Type I α2 expression cassette was inserted (which may be referred to as pEXP-HA-HsCOL1A2-1A1 hereinafter) was isolated from the cultivated bacterial cells to give the pEXP-HA-HsCOL1A2-1A1.

### Example 3 (Production of Anti-lysyl Hydroxylase 1 Antibody)

The lysyl hydroxylase 1 was prepared using recombinants E. coli., and the resulting lysyl hydroxylase 1 was used as an antigen to prepare anti-lysyl hydroxylase 1 antibody. The details of the procedures for preparation will be described below.

### (3-1) Construction of Expression Plasmid for E. coli

The pPLOD1-TOPO was cut with the restriction enzymes NdeI and XhoI, and then subjected to agarose gel electrophoresis to isolate and purify the about 2200bp double-stranded DNA fragment encoding lysyl hydroxylase 1.
After pET-16b (Novagen) was cut with the restriction enzymes NdeI and XhoI, the about 5700bp double-stranded DNA fragment was isolated and purified by agarose gel electrophoresis.
The double-stranded DNA fragment and the vector were ligated and using the resulting solution, E. coli (Competent high JM109, Toyobo Co., Ltd.) was transformed. For the ligation, Takara Ligation Kit ver2.1 was used.
On LB agar medium containing 50 µg/ml of ampicillin, the transformed E. coli was inoculated to cultivate it. A colony formed on the agar medium was inoculated into sterile LB medium (2 ml) containing 50 µg/ml of ampicillin and incubated in the test tube with shaking (37°C, 17 hours). Then, using QIAprep Spin Miniprep Kit (Qiagen), the plasmid into which the target DNA fragment was inserted was isolated from the cultivated bacterial cells. The plasmid will be referred to as pET16b-PLOD1 hereinafter.
Next, the pET16b-PLOD1 was used to transform E. coli (BL21(DE3)Competent cells, Takara Bio Inc.), and then the resulting transformant was used as antigen-preparation E. coli.

### (3-2) Determination of Expression of Lysyl Hydroxylase 1

The transformed E. coli with pET16b-PLOD1 was inoculated into 5 ml of LB liquid medium containing 50 µg/ml of ampicillin to cultivate it at 37°C for 17 hours. Also, the untransformed E. coli (i.e., BL21 (DE3)) was inoculated into 5 ml of LB medium to cultivate it at 37°C for 17 hours.
The culture solution of the transformant was inoculated into 50 ml of LB liquid medium containing 50 µg/ml of ampicillin to cultivate it at 37°C for 9 hours in such a way that its turbidity (OD₆₀₀) was equal to 0.1. Three hours after the inoculation, 150 µl of 0.1 M isopropyl-β-thiogalactopyranoside was added thereto. Also, the culture solution of the untransformed E. coli was inoculated into 50 ml of LB liquid medium to cultivate it at 37°C for 9 hours in such a way that its turbidity (OD₆₀₀) was equal to 0.1. Three hours after the inoculation, 150 µl of 0.1 M isopropyl-β-thiogalactopyranoside was added thereto.
Each 20 ml of the culture solutions was centrifuged at 3,000 rpm, 4°C for 10 minutes. The supernatant was removed therefrom, and then, the precipitate was suspended in 5 ml of sterile distilled water. This suspension was centrifuged at 3,000 rpm, 4°C for 10 minutes. The supernatant was removed therefrom, and then, the precipitate was suspended in 0.7 ml of 200 mM potassium phosphate buffer (pH 7.5).

The suspension was subjected to disruption using Multi-beads Shocker (manufactured by Yasui Kikai Corporation). The disruption was performed under the conditions of E. coli disruption (0.1 mmΦ glass beads, 2,500 rpm, 10 minutes). The resulting solution was centrifuged at 13,000 rpm, 4°C for 10 minutes and a supernatant was collected.
The supernatant was subjected to SDS polyacrylamide gel electrophoresis in according with the procedures described in Laemmli, U. K., Nature, 227, 680 (1970). The electrophoresed gel was stained with Page Blue 83 (manufactured by Cosmo Bio Co., Ltd.)
On the electrophoresed gel, a distinct band was found to correspond to about 80 kDa. Molecular weight of lysyl hydroxylase 1 is 81.6 kDa, and so, the molecular weight of the observed band was an equivalent size to the lysyl hydroxylase 1. Further, for E. coli used as a host of the transformation, there was no band at about 80 kDa. These results show the expression of lysyl hydroxylase 1 in the transformant.

### (3-3) Purification of PLOD1

The E. coli transformed with the pET16b-PLOD1 was inoculated into 5 ml of LB liquid medium containing 50 µg/ml of ampicillin to cultivate it at 37°C for 17 hours.
The culture solution of the transformant was inoculated into 100 ml of LB liquid medium containing 50 µg/ml of ampicillin to cultivate it at 37°C for 9 hours in such a way that its turbidity (OD₆₀₀) was equal to 0.1. Three hours after the inoculation, 150 µl of 0.1 M isopropyl-β-thiogalactopyranoside was added thereto.
The culture solution was centrifuged at 5,000 rpm, 4°C for 10 minutes to collect bacterial cells. The collected bacterial cells were resuspended in 0.2 M phosphate buffer (pH 7.0) and then centrifuged at 5,000 rpm, 4°C for 10 minutes to collect bacterial cells.
A tag (His-tag) comprising repeating sequence of 10 histidine residues may be integrated into the N-terminal end of the pET16b-PLOD1, and the PLOD1 may express as a protein wherein the His-tag is fused on the N-terminal end. The PLOD1 was purified using an affinity column for the His-tag (Ni-NTA Fast Start kit, Quiagen). The PLOD1 was purified under the degenerative conditions.
The resulting protein was subjected to SDS polyacrylamide gel electrophoresis in according with the procedures described in Laemmli, U. K., Nature, 227, 680 (1970). The electrophoresed gel was stained with a Coomassie brilliant blue G250 staining fluid (Bio-Rad).
As a result of the electrophoresis, a distinct band was observed at about 80 kDa on the gel. The detected band was a single band, and the molecular weight was an equivalent size to the lysyl hydroxylase 1.

### (3-4) Preparation of Anti-lysyl Hydroxylase 1 Antibody

The purified PLOD1 was used along with adjuvants for the subcutaneous immunization of a rabbit in accordance with the known techniques. After the immunization, the rabbit was subjected to the check for antibody titers in blood and then the collection of whole blood to isolate IgG in accordance with the known techniques. The anti-lysyl hydroxylase 1 antibody (polyclonal antibody) obtained after the isolation will be referred to as TAL061025 hereinafter.

### Example 4 (Preparation of Lysyl Hydroxylase 1, Prolyl 4-Hydroxylase α1 Subunit and Prolyl 4-hydroxylase β Subunit Expression Yeasts)

### (4-1) Transfection of Lysyl Hydroxylase 1 Gene, Prolyl 4-Hydroxylase α1 Subunit Gene and Prolyl 4-Hydroxylase β Subunit Gene into Yeast

The Gene Transfer Plasmid pEXP-LH1(+)-P4HAB was transfected into yeast Komagataella pastoris PPY12 strain (purchased from ATCC, ATCC204163) so as to be chromosomally integrated through homologous recombination. The gene transfer procedures will be described below.
Into 100 ml of YPD liquid medium (prepared by dissolving 1 g of Yeast Extract and 2 g of Bacto Peptone into ion exchanged water to be made up to 90 ml and then autoclave sterilizing, followed by mixing the medium with 10 ml of 20% glucose which was mechanically sterilized separately), Komagataella pastoris PPY12 strain was inoculated to cultivate them at 30°C until the turbidity (OD₆₀₀) reached approximately 10. After 80 ml of the culture solution was cooled in an ice bath, it was centrifuged at 3,000 g, 4°C for 10 minutes to precipitate the bacterial cells. After removing the supernatant, the bacterial cells were washed with equal, 1/2 and then 1/4 parts of ice-chilled sterile distilled water in turn. After that, the bacterial cells were suspended in 1/4 parts of ice-chilled 1M sorbitol and then centrifuged at 3,000 g, 4°C for 10 minutes to collect them. The resulting bacterial cells were suspended in ice-chilled 1M sorbitol to adjust OD₆₀₀ into approximately 150.
Ten (10) µg of pEXP-LH1(+)-P4HAB were cut with the restriction enzyme Bsp1407I. The DNA was collected through ethanol precipitation and dissolved into 5 µl of 10 mM Tris-HCl to prepare DNA solution.
Into a sterile test tube, 100 µl of the bacterial cell suspension was added to be mixed with 5 µl of the DNA solution. After a gene transfer device (ECM630, manufactured by BTX) was set to the specific yeast conditions (1,500 V/2 mm, 25 µF, 200 Ω of parallel resistance), and the mixture was transfered into a cuvette with electrode, the cuvette was placed in the device to apply pulse voltage. After that, into the resulting mixture, 1 ml of 1M sorbitol was added and mixed. The mixture was inoculated in an amount of 200 µl on a MD agar medium (final concentration: 1.34% Yeast Nitrogen Base, 4 × 10⁻⁵% biotin, 2% glucose, 0.005% of histidine, 2% agar) to cultivate them at 30°C for 48 hours. A colony formed on the MD agar medium was isolated as a transformed yeast.

### (4-2) Determination of Trabsfection of Lysyl Hydroxylase 1 Gene

PCR was conducted using the chromosomal DNA of the transformed yeast as a template to determine integration of a lysyl hydroxylase 1 gene into the chromosome.
A small number of bacterial cells were taken from the colony formed on the MD agar medium to suspend them in 10 µl of sterile distilled water. Into the suspension, 5 µl of 2,000 u/µl Lyticase (purchased from Sigma) was added and incubated at 30°C for 20 minutes. After that, the suspension was incubated at -80°C for 10 minutes and then at 98°C for 5 minutes before centrifugation at 12,000 rpm, 4°C for 10 minutes. A supernatant was collected to use it as a chromosomal DNA extract.
Oligonucleotides 40 and 39 were synthesized. The oligonucleotide 40 is oligonucleotide located on the 5' end of a lysyl hydroxylase 1 gene, and the oligonucleotide 39 is oligonucleotide located on the 3' end of the gene, respectively.
The double-stranded DNA fragment of a lysyl hydroxylase 1 gene was amplified by PCR using the following oligonucleotides 40 and 39 as primers and the chromosomal DNA extract as a template.
(a) Oligonucleotide 40: TATTCGAAACGATGCGGCCCCTGCTGCTAC (SEQ ID NO: 40)
(b) Oligonucleotide 39: CGACTAGTTTAGGGATCGACGAA (SEQ ID NO: 39)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 60°C for 30 seconds and extension at 68°C for 4 minutes.
The solution resulted from the PCR was subjected to agarose gel electrophoresis to lead to observation of a single band at about 2,200 bp on the gel. This obviously shows that a lysyl hydroxylase 1 gene has been transfected into the chromosome.

### (4-3) Determination of Transfection of Prolyl 4-Hydroxylase α1 Subunit Gene

PCR was conducted using the chromosomal DNA of the transformed yeast as a template to determine integration of lysyl 4-hydroxylase α1 subunit gene into the chromosome.
A small number of bacterial cells were taken from the colony formed on the MD agar medium to suspend them in 10 µl of sterile distilled water. Into the suspension, 5 µl of 2,000 u/µl Lyticase (purchased from Sigma) was added and incubated at 30°C for 20 minutes. After that, the suspension was incubated at -80°C for 10 minutes and then at 98°C for 5 minutes before centrifugation at 12,000 rpm, 4°C for 10 minutes. A supernatant was collected to use it as a chromosomal DNA extract.
Oligonucleotides 27 and 28 were synthesized. The oligonucleotide 27 is oligonucleotide located on the 5' end of prolyl 4-hydroxylase α1 subunit gene, and the oligonucleotide 28 is oligonucleotide located on the 3' end of the gene, respectively.
The double-stranded DNA fragment of prolyl 4-hydroxylase α1 subunit gene was amplified by PCR using the following oligonucleotides 27 and 28 as primers and the chromosomal DNA extract as a template.
(a) Oligonucleotide 27: TATTCGAAACGATGATCTGGTATATATTAATTATA (SEQ ID NO: 27)
(b) Oligonucleotide 28: TTGCTAGCTCATTCCAATTCTGACAACGTACAAGG (SEQ ID NO: 28)

As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 604°C for 30 seconds and extension at 68°C for 4 minutes.
The solution resulted from the PCR was subjected to agarose gel electrophoresis, and therefore, a single band was observed at about 1,600 bp on the gel. This obviously shows that prolyl 4-hydroxylase α1 subunit gene has been transfected into the chromosome.

### (4-4) Determination of Transfection of Prolyl 4-Hydroxylase β Subunit Gene

PCR was conducted using the chromosomal DNA of the transformed yeast as a template to determine integration of β subunit of prolyl 4-hydroxylase gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. After that, the suspension was incubated at -80°C for 10 minutes and then at 98°C for 5 minutes before centrifugation at 12,000 rpm, 4°C for 10 minutes. A supernatant was collected to use it as a chromosomal DNA extract.
Oligonucleotides 33 and 34 were synthesized. The oligonucleotide 33 is oligonucleotide located on the 5' end of β subunit of prolyl 4-hydroxylase gene, and the oligonucleotide 34 is oligonucleotide located on the 3' end of the gene, respectively.
The double-stranded DNA fragment of β subunit of prolyl 4-hydroxylase gene was amplified by PCR using the following oligonucleotides 33 and 34 as primers and the chromosomal DNA extract as a template.
(a) Oligonucleotide 33: TTACTAGTGACGCCCCCGAGGAGGA (SEQ ID NO: 33)
(b) Oligonucleotide 34: TTACTAGTTTACAGTTCATCTTTCACAGCTTTCT (SEQ ID NO: 34)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 60°C for 30 seconds and extension at 68°C for 4 minutes.
The solution resulted from the PCR was subjected to agarose gel electrophoresis, and therefore, a single band was observed at about 1,500 bp on the gel. This obviously shows that the β subunit of prolyl 4-hydroxylase gene has been transfected into the chromosome.
In conclusion, as the result of determination of gene transfer in 4-2, 4-3 and 4-4, TT061018-1-13 strain as the recombinant yeast obtained by transfecting all of the genes was provided.

### (4-5) Determination of expression of Lysyl Hydroxylase 1

The TT061018-1-13 strain was inoculated into 100 ml of BMGY medium (1% Yeast Extract, 2% peptone, 100 mM phosphate buffer, 1.34% Yeast Nitrogen Base, 4 × 10⁻⁵% biotin, 1% glycerol, 0.005% of histidine) to cultivate it at 30°C for 26 hours. The resulting culture solution was then centrifuged at 5,000 g, room temperature for 10 minutes to collect bacterial cells.
The collected bacterial cells were inoculated into 50 ml of BMM medium (100 mM phosphate buffer, 1.34% Yeast Nitrogen Base, 4 × 10⁻⁵% biotin, 0.5% methanol, 0.005% of histidine) in such a way that the turbidity (OD₆₀₀) was equal to approximately 10, and cultivated at 30°C for 60 hours. Every about 12 hours during the cultivation, 50% methanol was added in an amount of 0.5 ml each.

After 4 ml of the culture solution was centrifuged at 5,000 g, 4°C for 10 minutes, the bacterial cells were collected therefrom and then suspended in 2 ml of ice-chilled distilled water. The suspension was centrifuged at 5,000 g, 4°C for 10 minutes, and then, the resulting bacterial cells were resuspended in 0.7 ml of 100 mM of potassium phosphate buffer, pH 7.0.
The suspension was subjected to disruption using Multi-beads Shocker (manufactured by Yasui Kikai Corporation). The disruption was performed under the conditions for yeast disruption (0.5 mmΦ glass beads, 2,500 rpm, 40 minutes). The resulting solution was centrifuged at 10,000 rpm, 4°C for 10 minutes to collect the supernatant.
The supernatant was subjected to SDS polyacrylamide gel electrophoresis in according with the procedures described in Laemmli, U. K., Nature, 227, 680 (1970). The electrophoresed acrylamide gel was blotted to PVDF membrane (Immobilon-P, manufactured by Millipore Corp.).
The resulting membrane was shaken in 25 ml of Blocking one (purchased from Nacalai Tesque., Inc.) for 1 hour, and then washed with PBS-T (PBS solution containing 0.5% Tween-20) twice.

Ten (10) µl of anti-lysyl hydroxylase 1 antibody (TAL160025) was diluted with 10 ml of Can Get Signal solution 1 (manufactured by Toyobo Co., Ltd.) to prepare a diluted solution, in which the membrane was then soaked and shaken for 1 hour. After shaking, the membrane was washed with each 25 ml of PBS-T three times. Next, 10 µl of secondary antibody (anti-rabbit IgG - HRP-Linked donkey antibody NA-934, manufactured by GE Healthcare Bio Science) was diluted with 10 ml of Can Get Signal solution 2 (manufactured by Toyobo Co., Ltd.) to prepare a diluted solution, in which the membrane was further soaked and shaken for 1 hour. After shaking, the membrane was washed with each 25 ml of PBS-T three times. The luminescence generated from the washed membrane with a chemiluminescence detection reagent (ECL Western Blotting Detection System, manufactured by GE Healthcare Bio Science) was detected by means of an image analyzer (LAS-3000UVmini, manufacturted by Fujifilm Corparation). As a result, the band of molecular weight equivalent to LH1 was detected in the TT061018-1-13 strain, and therefore, it was found that the strain produced LH1.

### (4-5) Determination of expression of Lysyl Hydroxylase α1 Subunit

The TT061018-1-13 strain was inoculated into 100 ml of BMGY medium (1% Yeast Extract, 2% peptone, 100 mM phosphate buffer, 1.34% Yeast Nitrogen Base, 4 × 10⁻⁵% biotin, 1% glycerol, 0.005% of histidine) to cultivate it at 30°C for 26 hours. The resulting culture solution was then centrifuged at 5,000 g, room temperature for 10 minutes to collect bacterial cells.
The collected bacterial cells were inoculated into 50 ml of BMM medium (100 mM phosphate buffer, 1.34% Yeast Nitrogen Base, 4 × 10⁻⁵% biotin, 0.5% methanol, 0.005% of histidine) in such a way that the turbidity (OD₆₀₀) was equal to approximately 10, and cultivated at 30°C for 60 hours. Every about 12 hours during the cultivation, 50% methanol was added in an amount of 0.5 ml each.

After 4 ml of the culture solution was centrifuged at 5,000 g, 4°C for 10 minutes, the bacterial cells were collected and then suspended in 2 ml of ice-chilled distilled water. The suspension was centrifuged at 5,000 g, 4°C for 10 minutes, and the resulting bacterial cells were resuspended in 0.7 ml of 100 mM of potassium phosphate buffer, pH 7.0.
The suspension was subjected to disruption using Multi-beads Shocker (manufactured by Yasui Kikai Corporation). The disruption was performed under the conditions for yeast disruption (0.5 mmΦ glass beads, 2,500 rpm, 40 minutes). After the resulting solution was centrifuged at 10,000 rpm, 4°C for 10 minutes, a supernatant was collected.
The supernatant was subjected to SDS polyacrylamide gel electrophoresis in according with the procedures described in Laemmli, U. K., Nature, 227, 680 (1970) . The electrophoresed acrylamide gel was blotted to PVDF membrane (Immobilon-P, manufactured by Millipore Corp.).
The resulting membrane was shaken in 25 ml of Blocking one (purchased from Nacalai Tesque., Inc.) for 1 hour, and then washed with PBS-T (PBS solution containing 0.5% Tween-20) twice.

Five (5) µl of anti-prolyl hydroxylase α1 antibody (63167, purchased from MP Biomedicals) was diluted with 10 ml of Can Get Signal solution 1 (manufactured by Toyobo Co., Ltd.) to prepare a diluted solution, and then, the membrane was soaked in the diluted solution and shaken for 1 hour. After shaking, the membrane was washed with each 25 ml of PBS-T three times. Then, 10 µl of secondary antibody (anti-mouse IgG - HRP-Linked sheep antibody NA-931, manufactured by GE Healthcare Bio Science) was diluted with 10 ml of Can Get Signal solution 2 (manufactured by Toyobo Co., Ltd.) to prepare a diluted solution, and then, the membrane was soaked in this diluted solution and shaken for 1 hour. After shaking, the membrane was washed with each 25 ml of PBS-T three times. The luminescence generated from the washed membrane with a chemiluminescence detection reagent (ECL Western Blotting Detection System, manufactured by GE Healthcare Bio Science) was detected by means of an image analyzer (LAS-3000UVmini, manufacturted by Fujifilm Corporation).
As a result, the band equivalent to prolyl hydroxylase α1 subunit was detected in the TT061018-1-13 strain, and therefore, it was found that the strain produced prolyl hydroxylase α1 subunit.

### (4-6) Determination of expression of Prolyl Hydroxylase β Subunit

The TT061018-1-13 strain was inoculated into 100 ml of BMGY medium (1% Yeast Extract, 2% peptone, 100 mM phosphate buffer, 1.34% Yeast Nitrogen Base, 4 × 10⁻⁵% biotin, 1% glycerol, 0.005% of histidine) to cultivate it at 30°C for 26 hours. The resulting culture solution was then centrifuged at 5,000 g, room temperature for 10 minutes to collect bacterial cells.
The collected bacterial cells were inoculated into 50 ml of BMM medium (100 mM phosphate buffer, 1.34% Yeast Nitrogen Base, 4 × 10⁻⁵% biotin, 0.5% methanol, 0.005% of histidine) in such a way that the turbidity (OD₆₀₀) was equal to approximately 10, and cultivated at 30°C for 60 hours. Every about 12 hours during the cultivation, 50% methanol was added in an amount of 0.5 ml each.

After 4 ml of the culture solution was centrifuged at 5,000 g, 4°C for 10 minutes, the bacterial cells were collected therefrom and then suspended in 2 ml of ice-chilled distilled water. The suspension was centrifuged at 5,000 g, 4°C for 10 minutes, and then, the resulting bacterial cells were resuspended in 0.7 ml of 100 mM of potassium phosphate buffer, pH 7.0.
The suspension was subjected to disruption using Multi-beads Shocker (manufactured by Yasui Kikai Corporation). The disruption was performed under the conditions for yeast disruption (0.5 mmΦ glass beads, 2,500 rpm, 40 minutes). The resulting solution was centrifuged at 10,000 rpm, 4°C for 10 minutes before a supernatant was collected.
The supernatant was subjected to SDS polyacrylamide gel electrophoresis in according with the procedures described in Laemmli, U. K., Nature, 227, 680 (1970) . The electrophoresed acrylamide gel was blotted to PVDF membrane (Immobilon-P, manufactured by Millipore Corp.).
The resulting membrane was shaken in 25 ml of Blocking one (purchased from Nacalai Tesque., Inc.) for 1 hour, and then washed with PBS-T (PBS solution containing 0.5% Tween-20) twice.

Two (2) µl of anti-prolyl hydroxylase β antibody (SPA-890, purchased from Stressgen) was diluted with 10 ml of Can Get Signal solution 1 (manufactured by Toyobo Co., Ltd.) to prepare a diluted solution, and then, the membrane was soaked in the diluted solution and shaken for 1 hour. After shaking, the membrane was washed with each 25 ml of PBS-T three times. Next, 1 µl of secondary antibody (anti-rabbit IgG - HRP-Linked donkey antibody NA-934, manufactured by GE Healthcare Bio Science) was diluted with 10 ml of Can Get Signal solution 2 (manufactured by Toyobo Co., Ltd.) to prepare a diluted solution, and then the membrane was soaked in this diluted solution and shaken for 1 hour. After shaking, the membrane was washed with each 25 ml of PBS-T three times. The luminescence generated from the washed membrane with a chemiluminescence detection reagent (ECL Western Blotting Detection System, manufactured by GE Healthcare Bio Science) was detected by means of an image analyzer (LAS-3000UVmini, manufacturted by Fujifilm Corporation).
As a result, the band of molecular weight equivalent to prolyl hydroxylase β subunit was detected in the TT061018-1-13 strain, and therefore, it was found that the strain produced prolyl hydroxylase subunit.

### Example 5 (Preparation of Human Collagen Type III Expression Yeast)

### (5-1) Transfection of Human Collagen Type III Gene into Yeast

In order to be chromosomally integrated through homologous recombination, the Gene Transfer Plasmid pEXP-HA-HsCOL3A1 was transfected into the yeast TT061018-1-13 strain in which a lysyl hydroxylase 1 gene, prolyl 4-hydroxylase α1 subunit gene and β subunit of propyl 4-hydroxylase gene has been transfected. The gene transfer procedures will be described below.
Into 100 ml of YPD liquid medium, the yeast TT061018-1-13 strain was inoculated to cultivate them at 30°C until the turbidity (OD₆₀₀) reached approximately 10. The culture solution of 80 ml was cooled in an ice bath, and then centrifuged at 3,000 g, 4 °C for 10 minutes to precipitate the bacterial cells. After removing the supernatant, the bacterial cells were washed with equal, 1/2 and then 1/4 parts of ice-chilled sterile distilled water in turn. After that, the bacterial cells were suspended in 1/4 parts of ice-chilled 1M sorbitol and then centrifuged at 3,000 g, 4°C for 10 minutes to collect the bacterial cells. The resulting bacterial cells were suspended in ice-chilled 1M sorbitol to adjust OD₆₀₀ into approximately 150.

Ten (10) µg of pEXP-HA-HsCOL3A1 were cut with the restriction enzyme XbaI. The DNA was collected through ethanol precipitation and dissolved into 5 µl of 10 mM Tris-HCl to prepare DNA solution.
Into a sterile test tube, 100 µl of the bacterial cell suspension was added to be mixed with 5 µl of the DNA solution. After a gene transfer device (ECM630, manufactured by BTX) was set to the specific yeast conditions (1,500 V/2 mm, 25 µF, 200 Q of parallel resistance), and the mixture was transfered into a cuvette with electrode, the cuvette was placed in the device to be subjected to pulses. After that, into the resulting mixture, 1 ml of 1M sorbitol was added and mixed. Two hundreds (200) µl of the resulting mixture was inoculated on a MD agar medium to cultivate it at 30°C for 48 hours. A colony formed on the MD agar medium was isolated as a transformed yeast.

### (5-2) Determination of Ttransfection of Human Collagen Type III Gene

PCR was conducted using the chromosomal DNA of the transformed yeast as a template to determine integration of human collagen Type III gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, the suspension was incubated at -80°C for 10 minutes and then at 98°C for 5 minutes before centrifugation at 12,000 rpm, 4°C for 10 minutes. A supernatant was collected to use it as a chromosomal DNA extract.
Oligonucleotides 50 and 51 were synthesized. The oligonucleotide 50 is oligonucleotide located on the 5' end of human collagen Type III gene, and the oligonucleotide 51 is oligonucleotide located on the 3' end of the gene, respectively.
The double-stranded DNA fragment of human collagen Type III gene was amplified by PCR using the following oligonucleotides 50 and 51 as primers and the chromosomal DNA extract as a template.
(a) Oligonucleotide 50: TATTCGAAACGATGATGAGCTTTGTGCAAAAGGGG (SEQ ID NO: 50)
(b) Oligonucleotide 51: TTACTAGTTTATAAAAAGCAAACAGGGCCAACGT (SEQ ID NO: 51)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 60°C for 30 seconds and extension at 68°C for 5 minutes.
The solution resulted from the PCR was subjected to agarose gel electrophoresis, and then a single band was found to correspond to about 4,400 bp on the gel. This obviously shows that the β subunit of prolyl 4-hydroxylase gene has been transfected into the chromosome.
As to the yeast, the strain obtained by transfecting human collagen Type III gene into the TT061018-1-13 strain will be referred to as TT061226-1-3 strain hereinafter.

### (5-3) Determination of Expression of Human Collagen Type III

The TT061226-1-3 strain was inoculated into 100 ml of BMGY medium to cultivate it at 30°C for 26 hours. The resulting culture solution was then centrifuged at 5,000 g, room temperature for 10 minutes to collect bacterial cells.
The collected bacterial cells were inoculated into 50 ml of BMM medium in such a way that the turbidity (OD₆₀₀) was equal to approximately 10, and cultivated at 30°C for 60 hours. Every about 12 hours during the cultivation, 50% methanol was added in an amount of 0.5 ml each.
After 4 ml of the culture solution was centrifuged at 5,000 g, 4°C for 10 minutes, the bacterial cells were collected therefrom and suspended in 2 ml of ice-chilled distilled water. The suspension was centrifuged at 5,000 g, 4°C for 10 minutes, and then, the resulting bacterial cells were resuspended in 0.7 ml of 100 mM of potassium phosphate buffer, pH 7.0.

The suspension was subjected to disruption using Multi-beads Shocker (manufactured by Yasui Kikai Corporation). The disruption was performed under the conditions for yeast disruption (0.5 mmΦ glass beads, 2,500 rpm, 40 minutes). The resulting solution was centrifuged at 10,000 rpm, 4°C for 10 minutes before a supernatant was collected.
The supernatant was subjected to SDS polyacrylamide gel electrophoresis in according with the procedures described in Laemmli, U. K., Nature, 227, 680 (1970). The electrophoresed acrylamide gel was stained with Page Blue 83 (manufactured by Cosmo Bio Co., Ltd.)
As a result of the electrophoresis, a distinct band was found to correspond to at about 100 kDa or more on the gel. Molecular weight of procollagen of human collagen Type III is approximately 140 kDa, and so, the molecular weight of the observed band was an equivalent size to the human collagen Type III procollagen. From these results, it was found that the human collagen Type III expressed in the TT061226-1-3 strain.

### Example 6 (Analysis of Collagen)

### (6-1) Cultivation of Yeast

To conduct preculture, the TT061226-1-3 strain was inoculated into 100 ml of BMGY medium and incubated it at 30°C for 20 hours. Into 1 L volume of Basal salt medium prepared in 3 L jar fermentor (B. E. Marubisi Co., Ltd), the culture solution was inoculated in such a way that the turbidity (OD₆₆₀) was equal to approximately 1.25, and then agitated with aeration to conduct main culture.
The main culture was conducted with setting the culture temperature at 30°C, maximum air flow rate at 1.0 vvm (1 L). After all available glycerol in the medium was consumed, feeding of glycerol feed medium (50% glycerol, 1.2% PMT1 solution) was started. The glycerol fed-batch culture was stopped when OD₆₆₀ of the culture solution achieved approximately 140. Then, feeding of methanol feed medium (99.8%(w/v) methanol, 1.2% PMT1) was started to conduct the methanol fed-batch culture.
For the methanol fed-batch culture, culture temperature was changed to 32°C, and dissolved oxygen concentration was controlled to be approximately 8 ppm. During the cultivation, the culture solution was maintained constant pH of approximately 5.0 using 28% ammonia water and 4M phosphoric acid. After approximately 140 hours in culture, approximately 2L of the culture solution were obtained. The composition of the reaction solutions used herein is given as follows:

| | | |
|---|---|---|
| (a) | (Composition of Basal salt medium) | |
| (b) | 85% H₃PO₄ | 26.7 mL/L |
| (c) | CaSO₄-2H₂O | 0.93 g/L |
| (d) | K₂SO₄ | 18.2 g/L |
| (e) | MgSO₄-7H₂O | 14.9 g/L |
| (f) | KOH | 4.13 g/L |
| (g) | Glycerol | 40 g/L |

After mixing the above components, it was subjected to autoclave sterilization. After adjusted to pH 5.0 with 28% ammonia water, 2 ml/L of PMT1 solution and 1 ml/L of a solution of defoaming agent in methanol (12.5% Adekanol LG295S) were further added.

### (Composition of PMT1 Solution)

| | | |
|---|---|---|
| (a) | CuSO₄-5H₂O | 6.0 g/L |
| (b) | KI | 0.8 g/L |
| (c) | MnSO₄-H₂O | 3.0 g/L |
| (d) | Na₂MoO₄-2H₂O | 0.2 g/L |
| (e) | H₃BO₄ | 0.2 g |
| (f) | CaSO₄-2H₂O | 0.5 g/L |
| (g) | ZnCl₂ | 20 g/L |
| (h) | FeSO₄-7H₂O | 65 g/L |
| (i) | Biotin | 0.2 g/L |
| (j) | Conc. sulfuric acid | 5 mL/L |

### (6-2) Crude Purification of Collagen

The bacterial cells obtained from the culture were subjected to disruption, and then, collagen was purified from the resulting solution. The disruption of the bacterial cells was conducted using DYNO-MILL Type KDL-A (Willy A. Bachofen AG). The culture solution was centrifuged at 5,000 rpm, 4°C for 10 minutes to collect the bacterial cells. The obtained bacterial cells were suspended in potassium phosphate buffer (50 mM, pH6.0) and then centrifuged to collect the bacterial cells. These procedures were repeated once again to remove the medium components, and then, 600 g of wet bacterial cells were suspended in 1500 ml of potassium phosphate buffer (50 mM, pH6.0). Using a feed pump, the suspension was applied into the DYNO-MILL which was set to the conditions for yeast disruption. Completion of disruption was determined by examining the configuration of the bacterial cells under a microscope
The solution of the disrupted bacterial cells was 2-fold diluted with potassium phosphate buffer (50 mM, pH6.0). Conc. hydrochloric acid was used to adjust pH to approximately 2.0. Pepsin (purchased from Sigma) was added to be 5 mg/ml of final concentration. After incubating at 4°C for 96 hours, the solution was centrifuged at 9,000 rpm, 4°C for 30 minutes to collect the supernatant. Into the resulting supernatant, 10 N NaOH was added to adjust pH to approximately 10. The supernatant was incubated at 4°C with stirring. Approximately 16 hours later, the supernatant was centrifuged at 9,000 rpm, 4°C for 30 minutes to collect a supernatant.

Into the resulting supernatant, acetic acid was added so that the final concentration could be 0.5 M. Conc. hydrochloric acid was used to adjust pH to approximately 3.0. The supernatant was centrifuged at 9,000 rpm, 4°C for 30 minutes to collect the supernatant. Into the supernatant, NaCl was added so that the final concentration could be 1 M and then incubated at 4°C with stirring. Approximately 16 hours later, the supernatant was centrifuged at 9,000 rpm, 4°C for 30 minutes to collect precipitate. The resulting precipitate was dissolved in 0.1 N HCl.
Into the dissolved solution, 1M potassium phosphate buffer (pH 7.4) was added so that the final concentration could be 0.05 M. and then adjusted to pH of approximately 7.4 using 10 N NaOH. NaCl was added so that the final concentration could be 2 M, and the solution was incubated at 4°C with stirring. Approximately 16 hours later, the solution was centrifuged at 9,000 rpm, 4°C for 30 minutes to collect precipitate. The resulting precipitate was dissolved in 0.1 N HCl and then centrifuged at 9,000 rpm, 4°C for 30 minutes to collect the supernatant.
Into the resulting supernatant, acetic acid was added so that the final concentration could be 0.5 M. Conc. hydrochloric acid was used to adjust pH to approximately 3.0. NaCl was added so that the final concentration could be 1 M, and the supernatant was incubated at 4°C with stirring. Approximately 16 hours later, the supernatant was centrifuged at 9,000 rpm, 4°C for 30 minutes to collect precipitate. The resulting precipitate was dissolved in 0.1 N HCl. The dissolved solution was centrifuged at 40,000 rpm, 4°C for 60 minutes to collect a supernatant.
The resulting supernatant was added into a dialysis tube (Spectra/Por, SPECTUM), dialyzed three times with ten-fold volume of 1 mM HCl solution more than the volume of the supernatant, and then filtered with 0.22 µm filter (Millex GP, manufactured by Millipore Corp.) to give crude collagen.

### (6-3) Purification of Collagen

Into the crude collagen, 1M potassium phosphate buffer (pH7.4) was added so that the final concentration could be 0.05 M, and then adjusted to pH of approximately 7.4 using 10 N NaOH. NaCl was added so that the final concentration could be 2 M, and the crude collagen solution was incubated at 4°C with stirring. Approximately 16 hours later, the crude collagen solution was centrifuged at 9,000 rpm, 4°C for 30 minutes to collect precipitate. The resulting precipitate was dissolved in 5-fold weight of 0.1 N HCl more than the weight of the precipitate, and then centrifuged at 9,000 rpm, 4°C for 30 minutes to collect a precipitate.
Into the resulting supernatant, acetic acid was added so that the final concentration could be 0.5 M. Conc. hydrochloric acid was used to adjust pH to approximately 3.0. NaCl was added so that the final concentration could be 1 M, and the supernatant was incubated at 4°C with stirring. Approximately 16 hours later, it was centrifuged at 9,000 rpm, 4°C for 30 minutes to collect a precipitate. The resulting precipitate was dissolved in 0.1 N HCl. The dissolved solution was centrifuged at 40,000 rpm, 4°C for 60 minutes to collect a supernatant.
The resulting supernatant was added into a dialysis tube (Spectra/Por, SPECTUM), dialyzed three times with ten-fold volume of 1 mM HCl solution more than the volume of the supernatant, and then filtered with 0.22 µm filter (Millex GP, manufactured by Millipore Corp.) to give purified collagen.

### (6-4) Electrophoresis of Collagen

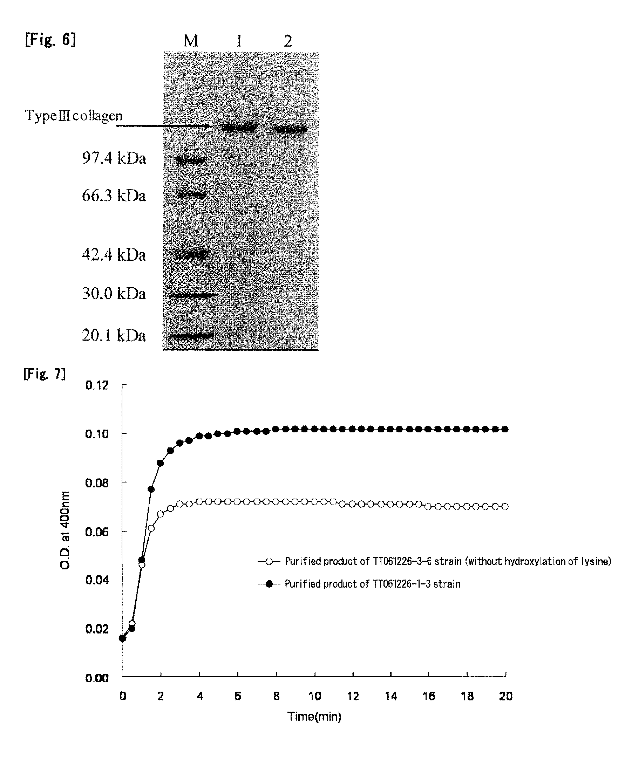

The purified collagen was subjected to SDS polyacrylamide gel electrophoresis in according with the procedures described in Laemmli, U. K., Nature, 227, 680 (1970). The electrophoresed acrylamide gel was stained with a Coomassie brilliant blue G250 staining fluid (Bio-Rad). From the result of the electrophoresis, a distinct single band was observed on the gel. Further, molecular weight of the band was an equivalent size to the collagen (see Fig. 6). Lane 1 in Fig. 6 shows an electrophoretic profile of the purified collagen obtained from the TT061226-1-3 strain.

### (6-5) Analysis of Amino Acid

A 0 . 1M hydrochloric acid solution containing 1 mg of the purified recombinant human collagen was added into a hydrolysis tube and dried under reduced pressure before the addition of 500 µl of 6M hydrochloric acid solution. Air in the hydrolysis tube was purged with nitrogen and then sealed under reduced pressure.
The sealed hydrolysis tube was incubated at 110°C for 24 hours to hydrolyze the purified recombinant human collagen. After the hydrolysis tube was opened, the hydrochloric acid solution therein was dried under reduced pressure.
The hydrolyzed human collagen was dissolved in 2 ml of 20 mM HCl, and 10 µg of the resulting hydrolysate were examined by ninhydrin coloring method (on Hitachi Model L-8800 high performance amino acid analyzer).
As a column, ion-exchang resin column (#2622) was used, and as eluents, commercially available products: L-8500 Buffer Solution PH-1 (021-09111, Wako Pure Chemical Industries, Ltd.), L-8500 Buffer Solution PH-2 (028-09121, Wako Pure Chemical Industries, Ltd.), L-8500 Buffer Solution PH-3 (025-09131, Wako Pure Chemical Industries, Ltd.), L-8500 Buffer Solution PH-4 (022-09141, Wako Pure Chemical Industries, Ltd.) and L-8500 Column Regenerating Solution PH (PH-RG) (036-12531, Wako Pure Chemical Industries, Ltd.) were used. For color development of the amino acid by ninhydrin coloring method, commercially available Ninhydrin Solution Set (201-06251, Wako Pure Chemical Industries, Ltd.) was used. The amino acid standard mixture was prepared and used by adding trans-4-Hydroxy-L-PROLINE (H-6002, Sigma) and DL-plus allo-δ Hydroxylysine, HCl (3920, CALBIO. CHEM. ) into commercially available AMINO ACID CALIBRATION MIXTURE (782-3140, Hitachi High-Tech Fielding Corporation) and then adjusting the amino acid level to be 100 nmol/mL, wherein each concentration of proline and hydroxyproline was 200 nmol/mL.
As shown in Table 1, it was found that in this example, a composition ratio of hydroxylated lysine to the total lysine residues was increased up to 36.9%.

**Table 1: Amino acid composition of purified collagen**

| | Purified product of TT061226-3-6 strain*2 | Purified product of TT061226-1-3 strain*3 | Wild type human Type III collagen*1 |
|---|---|---|---|
| Asp | 48.8 | 49.2 | 42.0 |
| Thr | 15.7 | 16.2 | 13.0 |
| Ser | 40.7 | 41.4 | 39.0 |
| Glu | 69.0 | 69.1 | 71.0 |
| Gly | 349.8 | 348.3 | 350.0 |
| Ala | 88.9 | 88.8 | 96.0 |
| Cys | 1.5 | 1.6 | 2.0 |
| Vat | 13.1 | 13.7 | 14.0 |
| Met | 8.5 | 8.7 | 8.0 |
| Ile | 13.8 | 13.9 | 13.0 |
| Leu | 22.4 | 22.5 | 22.0 |
| Tyr | 2.3 | 2.5 | 3.0 |
| Phe | 8.2 | 8.2 | 8.0 |
| Hyl | 0.0 | 13.2 | 5.0 |
| Lys | 36.3 | 24.1 | 30.0 |
| His | 6.8 | 6.8 | 6.0 |
| Arg | 46.5 | 46.4 | 46.0 |
| Hyp | 116.8 | 116.6 | 125.0 |
| Pro | 110.3 | 108.8 | 107.0 |
| Total | 1000 | 1000 | 1000 |

| | | | |
|---|---|---|---|
| *1 Literature data: Chung, F., and Miller, E. J., Science 183, 1200-1201; *2 Recombinant human collagen Type III (without hydroxylation of lysine) *3 Recombinant human collagen Type III (with coexpression of lysyl hydroxylase 1) | | | |

### (6-6) Measurement of Ability for Fibril Formation

The purified recombinant human collagen was dialyzed with 1 mM HCl and then diluted with the external solution derived from dialysis to be 0.06% of protein concentration. Next, 6-fold concentrated D-PBS(-) (which was obtained by dissolving 8 g of sodium chloride, 1.15 g of disodium hydrogen phosphate, 0.2 g of potassium chloride and 0.2 g of potassium hydrogen phosphate in water to be made up to 1000 mL) was added thereto to adjust the collagens concentration to be final 0.05% and the D-PBS(-) concentration to be 1-fold. The pH after preparation was between 7.3 and 7.4. After the purified recombinant human collagen was added into a quartz glass cell surrounded by a jacket, the cell was deaerated under reduced pressure using an oil-sealed rotary pump and then placed on the cell stand in spectrophotometer (U-2000, Hitachi). Absorbance at 400 nm of wave length was measured with time while allowing the circulating water at 37°C to flow (sampling points: every 30 second).
As shown in Fig. 7, it was found that ability for fibril formation of the collagen prepared from TT061226-1-3 strain was enhanced.
Since the present invention is not intended to be limited to any of the constitutions as mentioned above, but various modifications can be made within the scope of the appended claims, any other embodiments and examples provided by appropriately combining technical means discretely-disclosed in different embodiments and examples are also intended to be encompassed within the technical scope of the present invention.

### Example 7 (Preparation of Lysyl Hydroxylase 2, Prolyl 4-Hydroxylase α1 Subunit and Prolyl 4-Hydroxylase β Subunit Expression Yeast)

### (7-1) Transfecttion of Lysyl Hydroxylase 2 Gene, Prolyl 4-Hydroxylase α1 Subunit Gene and Prolyl 4-Hydroxylase β Subunit Gene into Yeast

The Gene Transfer Plasmid pEXP-LH2(+)-P4HAB was transfected into yeast Komagataella pastoris PPY12 strain (purchased from ATCC, ATCC204163) to be chromosomally integrated through homologous recombination. The gene transfer procedures will be described below.
Into 100 ml of YPD liquid medium (prepared by dissolving 1 g of Yeast Extract and 2 g of Bacto Peptone into ion exchanged water to be made up to 90 ml, followed by autoclave sterilization, and then the medium was mixed with 10 ml of 20% glucose which was subjected to filter sterilization), Komagataella pastoris PPY12 strain was inoculated to cultivate them at 30°C until the turbidity (OD₆₀₀) reached approximately 10. 80 ml of the culture solution was cooled in an ice bath, and then centrifuged at 3,000 g, 4°C for 10 minutes to precipitate the bacterial cells. After removing the supernatant, the bacterial cells were washed with equal, 1/2 and then 1/4 parts of ice-chilled sterile distilled water in turn. After that, the bacterial cells were suspended in 1/4 parts of ice-chilled 1M sorbitol and then centrifuged at 3,000 g, 4°C for 10 minutes to collect them. The resulting bacterial cells were suspended in ice-chilled 1M sorbitol to adjust OD₆₀₀ to approximately 150.
Ten (10) µg of pEXP-LH2(+)-P4HAB were cut with the restriction enzyme AatII. The DNA was collected through ethanol precipitation and dissolved into 5 µl of 10 mM Tris-HCl to prepare DNA solution.
Into a sterile test tube, 100 µl of the bacterial cell suspension was added to be mixed with 5 µl of the DNA solution.
After a gene transfer device (ECM630, manufactured by BTX) was set to the specific yeast conditions (1,500 V/2 mm, 25 µF, 200 Ω of parallel resistance), and the mixture was transfered into a cuvette with electrode, the cuvette was placed in the device to apply pulse voltage. After that, into the resulting mixture, 1 ml of 1M sorbitol was added and mixed. Two hundreds (200) µl of the resulting mixture was inoculated on a MD agar medium (final concentration: 1.34% Yeast Nitrogen Base, 4 × 10⁻⁵% biotin, 2% glucose, 0.005% of histidine, 2% agar) to cultivate it at 30°C for 48 hours. A colony formed on the agar medium was isolated as a transformed yeast.

### (7-2) Determination of Transfecttion of Lysyl Hydroxylase 2 Gene

PCR was conducted using the chromosomal DNA of the transformed yeast as a template to determine integration of lysyl hydroxylase 2 gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, the bacterial cells were incubated at -80°C for 10 minutes and then at 98°C for 5 minutes before centrifugation at 12,000 rpm, 4°C for 10 minutes. A supernatant was collected to be used as a chromosomal DNA extract.
Oligonucleotides 44 and 45 were synthesized. The oligonucleotide 44 is oligonucleotide located on the 5' end of lysyl hydroxylase 2 gene, and the oligonucleotide 45 is oligonucleotide located on the 3' end of the gene, respectively.
The double-stranded DNA fragment of a lysyl hydroxylase 2 gene was amplified by PCR using the following oligonucleotides 44 and 45 as primers and the chromosomal DNA extract as a template.
(a) Oligonucleotide 44: TATTCGAAACGATGGGGGGATGCACGGTG (SEQ ID NO: 44)
(b) Oligonucleotide 45: CGACTAGTTTAGGGATCTATAAATGACACTG (SEQ ID NO: 45)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 60°C for 30 seconds and extension at 68°C for 4 minutes.
The solution obtained from the PCR was subjected to agarose gel electrophoresis, and then a distinct single band was found to correspond to about 2,200 bp on the gel. This obviously shows that the lysyl hydroxylase 2 gene has been transfected into the chromosome.

### (7-3) Determination of Transfecttion of Prolyl 4-hydroxylase α1 Subunit Gene

PCR was conducted using the chromosomal DNA of the transformed yeast as a template to determine integration of lysyl 4-hydroxylase α1 subunit gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, bacterial cells were incubated at -80°C for 10 minutes and then at 98°C for 5 minutes before centrifugation at 12,000 rpm, 4°C for 10 minutes. A supernatant was collected to be used as a chromosomal DNA extract.
Oligonucleotides 27 and 28 were synthesized. The oligonucleotide 27 is oligonucleotide located on the 5' end of prolyl 4-hydroxylase α1 subunit gene, and the oligonucleotide 28 is oligonucleotide located on the 3' end of the gene, respectively.
The double-stranded DNA fragment of prolyl 4-hydroxylase α1 subunit gene was amplified by PCR using the following oligonucleotides 27 and 28 as primers and the chromosomal DNA extract as a template.
(a) Oligonucleotide 27: TATTCGAAACGATGATCTGGTATATATTAATTATA (SEQ ID NO: 27)
(b) Oligonucleotide 28: TTGCTAGCTCATTCCAATTCTGACAACGTACAAGG (SEQ ID NO: 28)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 60°C for 30 seconds and extension at 68°C for 4 minutes.
The solution obtained from the PCR was subjected to agarose gel electrophoresis, and then a distinct single band was observed at about 1,600 bp on the gel. This obviously shows the prolyl 4-hydroxylase α1 subunit gene has been transfected into the chromosome.

### (7-4) Determination of Transfecttion of Prolyl 4-Hydroxylase β Subunit Gene

PCR was conducted using the chromosomal DNA of the transformed yeast as a template to determine integration of β subunit of prolyl 4-hydroxylase gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, bacterial cells were incubated at -80°C for 10 minutes and then at 98°C for 5 minutes before centrifugation at 12,000 rpm, 4°C for 10 minutes. A supernatant was collected to be used as a chromosomal DNA extract.
Oligonucleotides 33 and 34 were synthesized. The oligonucleotide 33 is oligonucleotide located on the 5' end of β subunit of prolyl 4-hydroxylase gene, and the oligonucleotide 34 is oligonucleotide located on the 3' end of the gene, respectively.
The double-stranded DNA fragment of β subunit, of prolyl 4-hydroxylase gene was amplified by PCR using the following oligonucleotides 33 and 34 as primers and the chromosomal DNA extract as a template.
(a) Oligonucleotide 33: TTACTAGTGACGCCCCCGAGGAGGA (SEQ ID NO: 33)
(b) Oligonucleotide 34: TTACTAGTTTACAGTTCATCTTTCACAGCTTTCT (SEQ ID NO: 34)
As a polymerase for the PCR, BlendTaq PCR polymerase, manufactured by Toyobo Co., Ltd., was used. The details of composition, of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 x PCR buffer for BlendTaq | 5 µl |
| (e) | BlendTaq DNA polymerase (1 U/µl) | 0.5 µl |
| (f) | Sterile distilled water | 32.5 µl |

PCR, was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 60°C for 30 seconds and extension at 68°C for 4 minutes.
The solution obtained from the PCR was subjected to agarose gel electrophoresis, and therefore, a distinct single band was observed at about 1,500 bp on the gel. This obviously shows the transfecttion of β subunit of prolyl 4-hydroxylase gene into the chromosome.
In conclusion, as the result of determination of gene transfer in 7-2, 7-3 and 7-4, TT061018-3-5 strain was obtained as a recombinant yeast into which all of the genes have been transfected.

### (7-5) Determination of Expression of Lysyl Hydroxylase 2

The TT061018-3-5 strain was inoculated into 100 ml of BMGY medium (1% Yeast Extract, 2% peptone, 100 mM phosphate buffer, 1.34% Yeast Nitrogen Basle, 4 × 10⁻⁵% biotin, 1% glycerol, 0.005% of histidine) to cultivate it at 30°C for 26 hours. The resulting culture solution was then centrifuged at 5,000 g, room temperature for 10 minutes to collect bacterial cells.
The collected bacterial cells were inoculated into 50 ml of BMM medium (100 mM phosphate buffer, 1.34% Yeast Nitrogen Base, 4 × 10⁻⁵% biotin, 0.5% methanol, 0.005% of histidine) so that the OD₆₀₀ could be approximately 10 of turbidity, followed by cultivation at 30°C for 60 hours. Every about 12 hours during the cultivation, 50% methanol was added in an amount of 0.5 ml each.
After 0.7 ml of the culture solution was centrifuged at 5,000 g, 4°C for 10 minutes, the bacterial cells were collected to be suspended in 0.7 ml of ice-chilled distilled water. The resulting suspension was centrifuged at 5,000 g, 4°C for 10 minutes, and then, the collected bacterial cells were resuspended in 0.7 ml of sterile distilled water.
From the bacterial cells, total DNA was extracted with RNeasy Kit (QIAGEN), and then reverse transcription reaction was conducted using the total DNA as a template to synthesize cDNA. The reverse transcription reaction was performed using Takara RNA PCR Kit (AMV) ver.3.0 and Random 9mer as a reverse transcription reaction primer.
Oligonucleotides 62 and 63 were synthesized. The oligonucleotides 62 and 63 are primers which amplify an about 600-bp product corresponding to the N-terminal end of PLOD2 gene.
The double-stranded DNA fragment derived from the transcription product of PLOD2 gene was amplified by PCR using the following oligonucleotides 62 and 63 as primers and the resulting cDNA from the reverse transcription reaction as a template.
(a) Oligonucleotide 62: TGGAGAGGTGGTGATGGAATT (SEQ ID NO: 82)
(b) Oligonucleotide 63: AAAGAGTGCAGCCATTATCCTG (SEQ ID NO: 83)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted by 30 cycles of denaturation at 94°C for 30 seconds, annealing at 60°C for 30 seconds and extension at 68°C for 1 minute.
The solution obtained from the PCR was subjected to agarose gel electrophoresis, and therefore, a distinct single band was observed at about 600 bp on the gel. This obviously shows the expression of PLOD2 gene transfected into the chromosome.

### (7-6) Determination of Expression of Prolyl Hydroxylase α1 Subunit

The TT061018-3-5 strain was inoculated into 100 ml of BMGY medium (1% Yeast Extract, 2% peptone, 100 mM phosphate buffer, 1.34% Yeast Nitrogen Base, 4 × 10⁻⁵% biotin, 1% glycerol, 0.005% of histidine) to cultivate it at 30°C for 26 hours. The resulting culture solution was then centrifuged at 5,000 g, room temperature for 10 minutes to collect bacterial cells.
The collected bacterial cells were inoculated into 50 ml of BMM medium (100 mM phosphate buffer, 1.34% Yeast Nitrogen Base, 4 × 10⁻⁵% biotin, 0.5% methanol, 0.005% of histidine) so that the OD₆₀₀ could be approximately 10 of turbidity, followed by cultivation at 30°C for 60 hours. Every about 12 hours during the cultivation, 50% methanol was added in an amount of 0.5 ml each.
After 4 ml of the culture solution was centrifuged at 5,000 g, 4°C for 10 minutes, bacterial cells were collected and then suspended in 2 ml of ice-chilled distilled water. The resulting suspension was centrifuged at 5,000 g, 4°C for 10 minutes, and then, the collected bacterial cells were resuspended in 0.7 ml of 200 mM potassium phosphate buffer, pH 7.4.
The resulting suspension was subjected to disruption using Multi-beads Shocker (manufactured by Yasui Kikai Corporation). The disruption was performed under the conditions for yeast disruption (0.5 mmΦ glass beads, 2,500 rpm, 40 minutes). The resulting solution was centrifuged at 10,000 rpm, 4°C for 10 minutes before a supernatant was collected.
The supernatant was subjected to SDS polyacrylamide gel electrophoresis in according with the procedures described in Laemmli, U. K., Nature, 227, 680 (1970). The electrophoresed acrylamide gel was blotted to PVDF membrane (Immobilon-P, manufactured by Millipore Corp.).
The resulting membrane was shaken in 25 ml of Blocking one (purchased from Nacalai Tesque., Inc.) for 1 hour, and then washed with PBS-T (PBS solution containing 0.5% Tween-20) twice.

Five (5) µl of anti-prolyl hydroxylase αl antibody (63167, obtained from MP Biomedicals) was diluted with 10 ml of Can Get Signal solution 1 (manufactured by Toyobo Co., Ltd.) to prepare a diluted solution, and the membrane was then soaked and shaken in the resulting diluted solution for 1 hour. After shaking, the membrane was washed with each 25 ml of PBS-T three times. Next, 10 µl of secondary antibody (anti-mouse IgG - HRP-Linked sheep antibody DNA-931, manufactured by GE Healthcare Bio Science) was diluted with 10 ml of Can Get Signal solution 2 (manufactured by Toyobo Co., Ltd.) to prepare a diluted solution, and the membrane was further soaked and shaken in this diluted solution for 1 hour. After shaking, the membrane was washed with each 25 ml of PBS-T three times. The luminescence generated from the washed membrane with a chemiluminescence detection reagent (ECL Western Blotting Detection System, manufactured by GE Healthcare Bio Science) was detected by means of an image analyzer (LAS-3000UVmini, manufacturted by Fujifilm Corporation).
From the result, it was found that since the band of molecular weight equivalent to prolyl hydroxylase α1 subunit was detected, the TT061018-3-5 strain produces prolyl hydroxylase α1 subunit.

### (7-7) Determination of Expression of Prolyl Hydroxylase β Subunit

The TT061018-3-5 strain was inoculated into 100 ml of BMGY medium (1% Yeast Extract, 2% peptone, 100 mM phosphate buffer, 1.34% Yeast Nitrogen Base, 4 × 10⁻⁵% biotin, 1% glycerol, 0.005% of histidine) to cultivate it at 30°C for 26 hours. The resulting culture solution was then centrifuged at 5,000 g, room temperature for 10 minutes to collect bacterial cells.
The collected bacterial cells were inoculated into 50 ml of BMM medium (100 mM phosphate buffer, 1.34% Yeast Nitrogen Base, 4 × 10⁻⁵% biotin, 0.5% methanol, 0.005% of histidine) so that OD₆₀₀ could be approximately 10 of turbidity, followed by cultivation at 30°C for 60 hours. Every about 12 hours during the cultivation, 50% methanol was added in an amount of 0.5 ml each.
After 4 ml of the culture solution was centrifuged at 5,000 g, 4°C for 10 minutes, the bacterial cells were collected therefrom to be suspended in 2 ml of ice-chilled distilled water. The resulting suspension was centrifuged at 5,000 g, 4°C for 10 minutes, and then, the resulting bacterial cells were resuspended in 0.7 ml of 20 mM of potassium phosphate buffer, pH 7.4.
The suspension was subjected to disruption using Multi-beads Shocker (manufactured by Yasui Kikai Corporation). The disruption was performed under the conditions for yeast disruption (0.5 mmΦ glass beads, 2,50C rpm, 40 minutes). The resulting solution was centrifuged at 10,000 rpm, 4°C for 10 minutes before a supernatant was collected.
The supernatant was subjected to SDS polyacrylamide gel electrophoresis in according with the procedures described in Laemmli, U. K., Nature, 227, 680 (1970). The electrophoresed acrylamide gel was blotted to PVDF membrane (Immobilon-P, manufactured by Millipore Corp.).
The resulting membrane was shaken in 25 ml of Blocking one (purchased from Nacalai Tesque., Inc.) for 1 hour, and then washed with PBS-T (PBS solution containing 0.5% Tween-20) twice.

Two (2) µl of anti-prolyl hydroxylase β antibody (SPA-890, purchased from Stressgen) was diluted with 10 ml of Can Get Signal solution 1 (manufactured by Toyobo Co., Ltd.) to prepare a diluted solution, and the membrane was then soaked and shaken in the diluted solution for 1 hour. After shaking, the membrane was washed with each 25 ml of PBS-T three times. Next, 1 µl of secondary antibody (anti-rabbit IgG - HRP-Linked donkey antibody NA-934, manufactured by GE Healthcare Bio Science) was diluted with 10 ml of Can Get Signal solution 2 (manufactured by Toyobo Co., Ltd.) to prepare a diluted solution, and the membrane was further soaked and shaken in this diluted solution for 1 hour. After shaking, the membrane was washed with each 25 ml of PBS-T three times. The luminescence generated from the washed membrane with a chemiluminescence detection reagent (ECL Western Blotting Detection System, manufactured by GE Healthcare Bio Science) was detected by means of an image analyzer (LAS-3000UVmini, manufacturted by Fujifilm Corporation).
From the result, it was found that since the band of molecular weight equivalent to prolyl hydroxylase β subunit was detected, TT061018-3-5 strain produces prolyl hydroxylase β subunit.

### Example 8 (Preparation of Human Collagen Type I Expression Yeast)

### (8-1) Transfecttion of Human Collagen Type I α1 Gene and Human Collagen Type I α2 Gene into Yeast

In order to be chromosomally integrated through homologous recombination, the Gene Transfer Plasmid pEXP-HA-HsCOL1A2-1A1 was transfected into the gene transfer yeast TT061018-1-13 strain into which a lysyl hydroxylase 1 gene, prolyl 4-hydroxylase α1 subunit gene and β subunit of prolyl 4-hydroxylase gene have been transfected; and the gene transfer yeast TT061018-3-5 strain into which Lysyl hydroxylase 2 gene, prolyl 4-hydroxylase α1 subunit gene and β subunit of prolyl 4-hydroxylase gene have been transfected. The gene transfer procedures will be described below.
Into 100 ml of YPD liquid medium, the yeasts TT061018-1-13 strain and TT061018-3-5 strain were individually inoculated to cultivate them at 30°C until the turbidity (OD₆₀₀) reached approximately 10. 80 ml of the culture solution was cooled in an ice bath, and then centrifuged at 3,000 g, 4°C for 10 minutes to precipitate the bacterial cells. After removing the supernatant, the bacterial cells were washed with equal, 1/2 and then 1/4 parts of ice-chilled sterile distilled water in turn. After that, the bacterial cells were suspended in 1/4 parts of ice-chilled 1M sorbitol and then centrifuged at 3,000 g, 4°C for 10 minutes to collect them. The resulting bacterial cells were suspended in ice-chilled 1M sorbitol to adjust OD₆₀₀ to approximately 150.
Ten (10) µg of pEXP-HA-HsCOL1A2-1A1 were cut with the restriction enzyme XbaI. The DNA was collected through ethanol precipitation and dissolved into 5 µl of 10 mM Tris-HCl to prepare the DNA solution.
Into a sterile test tube, 100 µl of the bacterial cell suspension was added to be mixed with 5 µl of the DNA solution. After a gene transfer device (ECM630, manufactured by BTX) was set to the specific yeast conditions (1,500 V/2 mm, 25 µF, 200 Q of parallel resistance), and the mixture was transfered into a cuvette with electrode, the cuvette was placed in the device to apply pulse voltage. After that, into the resulting mixture, 1 ml of 1M sorbitol was added and mixed. 200 µl of the resulting mixture was inoculated on a MD agar medium to cultivate it at 30°C for 48 hours. A colony formed on the MD agar medium was isolated as a transformed yeast.

### (8-2) Determination of Transfection of Human Collagen Type I α1 Gene

PCR was conducted using the chromosomal DNA of the transformed yeast as a template to determine integration of human collagen Type I α1 gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, the bacterial cells were incubated at -804°C for 10 minutes and then at 98°C for 5 minutes before centrifugation at 12,000 rpm, 4°C for 10 minutes. A supernatant was collected to be used as a chromosomal DNA extract.
Oligonucleotides 56 and 57 were synthesized. The oligonucleotide 56 is oligonucleotide located on the 5' end of human collagen Type I αl gene, and the oligonucleotide 57 is oligonucleotide located on the 3' end of the gene, respectively.
The double-stranded DNA fragment of human collagen Type I α1 gene was amplified by PCR using the following oligonucleotides 56 and 57 as primers and the chromosomal DNA extract as a template.
(a) Oligonucleotide 56: TATTCGAAACGATGTTCAGCTTTGTGGACCTCCG (SEQ ID NO: 76)
(b) Oligonucleotide 57: TTACTAGTTTACAGGAAGCAGACAGGGCCAA (SEQ ID NO: 77)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1. U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing and extension at 68°C for 5 minutes.
The solution obtained from the PCR was subjected to agarose gel electrophoresis, and then a distinct single band was observed at about 4,400 bp on the gel. This obviously shows the transfecttion of human collagen Type I α1 gene into the chromosome.

### (8-3) Determination of Expression of Human Collagen Type I α2 gene

PCR was conducted using the chromosomal DNA of the transformed yeast as a template to determine integration of human collagen Type I α2 gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) solution was added and incubated at 30°C for 20 minutes. Then, the bacterial cells were incubated at -80°C for 10 minutes and then at 98°C for 5 minutes before centrifugation at 12,000 rpm, 4°C for 10 minutes. A supernatant was collected to be used as a chromosomal DNA extract.
Oligonucleotides 58 and 59 were synthesized. The oligonucleotide 58 is oligonucleotide located on the 5' end of human collagen Type I α2 gene, and the oligonucleotide 59 is oligonucleotide located on the 3' end of the gene, respectively.
The double-stranded DNA fragment of human collagen Type I α2 gene was amplified by PCR using the following Oligonucleotides 58 and 59 as primers and the chromosomal DNA extract as a template.
(a) Oligonucleotide 58: TATTCGAAACGATGCTCAGCTTTGTGGATACGCG (SEQ ID NO: 78)
(b) Oligonucleotide 59: TTACTAGTTTATTTGAAACAGACTGGGCCAATGTC (SEQ ID NO: 79)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 60°C for 30 seconds and extension at 68°C for 5 minutes.
The solution obtained from the PCR was subjected to agarose gel electrophoresis, and then a distinct single band was observed at about 4,100 bp on the gel. This obviously shows that human collagen Type I α2 gene has been transfected into the chromosome.
For the yeasts, hereinafter, the strain obtained by transfecting the human collagen Type I αl gene and the human collagen Type I α2 gene into the TT061018-1-13 strain will be referred to as a TT080417-1-8 strain, and the strain obtained by transecting the human collagen Type I αl gene and the human collagen Type I α2 gene into the TT061018-3-5 strain will be referred to as a TT080724-1-11 strain.

### (8-4) Determination of Expression of Human Collagen Type I αl and Human Collagen Type I α2

The TT080417-1-8 and TT080724-1-11 strains were individually inoculated into 100 ml of BMGY medium to cultivate them at 30°C for 26 hours. The resulting culture solution was then centrifuged at 5,000 g, room temperature for 10 minutes to collect bacterial cells.
The collected bacterial cells were inoculated into 50 ml of BMM medium so that OD₆₀₀ could be approximately 10 of turbidity, followed by cultivation at 30°C for 60 hours. Every about 12 hours during the cultivation, 50% methanol was added in an amount of 0.5 ml each.
After 4 ml of the culture solution was centrifuged at 5,000 g, 4°C for 10 minutes, the bacterial cells were collected therefrom to be suspended in 2 ml of ice-chilled distilled water. The suspension was centrifuged at 5,000 g, 4°C for 10 minutes, and then, the resulting bacterial cells were resuspended in 0.7 ml of 200 mM of potassium phosphate buffer, pH 7.4.
The suspension was subjected to disruption using Multi-beads Shocker (manufactured by Yasui Kikai Corporation). The disruption was performed under the conditions for yeast disruption (0.5 mmΦ glass beads, 2,500 rpm, 40 minutes). The resulting solution was centrifuged at 10,000 rpm, 4°C for 10 minutes before a supernatant was collected.
The supernatant was subjected to SDS polyacrylamide gel electrophoresis in according with the procedures described in Laemmli, U. K., Nature, 227, 680 (1970). The electrophoresed gel was stained with Page Blue 83 (manufactured by Cosmo Bio Co., Ltd.)
As a result of the electrophoresis, two distinct bands were found to correspond to about 100 kDa or more on the gel. Molecular weight of procollagen of human collagen Type I αl is approximately 140 kDa, and molecular weight of procollagen of human collagen Type I α2 is approximately 130 kDa, and therefore, from these results, it was found that both human collagen Type I α1 and human collagen Type I α2 have been expressed in the TT080417-1-8 and TT080724-1-11 strains, respectively.

### Example 9 (Analysis of Collagen)

### (9-1) Cultivation of Yeasts

To conduct precultures the TT090417-1-8 and TT080724-1-11 strains were individually inoculated into 100 ml of BMGY medium and incubated them at 30°C for 20 hours.
In 3 L jar fermentors (model: BMS-03P1, Able Corporation), 0.8 L of Basal salt medium were prepared. The culture solutions were inoculated so that final concentration OD₆₆₀ could be approximately 1.25 and then agitated with aeration to conduct main culture.
The main culture was conducted with setting the culture temperature at 30°C, maximum air flow rate at 0.8 vvm (0.8 L) and agitation rate to 800 rpm. After all available glycerol in the medium was consumed and oxygen consumption was decreased, feeding of glycerol feed medium (50% glycerol, 1.2% PMT1 solution) was started at a rate of about 15 g/h. The glycerol fed-batch culture was stopped when OD₆₆₀ of the culture solution achieved approximately 130. Then, feeding of methanol feed medium (98.6%(w/v) methanol, 1.2% PMT1) was started to onduct the methanol fed-batch culture. After the methanol fed-batch culture started, the culture temperature was changed to 32°C and dissolved oxygen concentratuin was controlled to be approximately 8 ppm. During the cultivation, pH of the culture solutions were maintained to be approximately 5.0 using 28% ammonia water. After approximately 136 hours in culture, 1358 g of the culture solution of TT080417-1-8 strain and 1371 g of the culture solution of TT080724-1-11 strain were obtained. The composition of the medium used herein is given as follows:

| | | |
|---|---|---|
| (a) | (Composition of Basal salt medium) | |
| (b) | 85% H₃PO₄ | 26.7 mL/L |
| (c) | CaSO₄-2H₂O | 0.93 g/L |
| (d) | K₂SO₄ | 18.2 g/L |
| (e) | MgSO₄-7H₂O | 14.9 g/L |
| (f) | KOH | 4.13 g/L |
| (g) | Glycerol | 40 g/L |

After mixing the above components, the mixture was subjected to autoclave sterilization. After adjusted to pH 5.0 with 28% ammonia water, 2 ml/L of PUT1 solution and 1 ml/L of a solution of defoaming agent in methanol (12.5% Adekanol LG295S) were further added.

### (Composition of PMT1 Solution)

| | | |
|---|---|---|
| (a) | CuSO₄-5H₂O | 6.0 g/L |
| (b) | KI | 0.8 g/L |
| (c) | MnSO₄-H₂O | 3.0 g/L |
| (d) | Na₂MoO₄-2H₂O | 0.2 g/L |
| (e) | H₃BO₄ | 0.2 g/L |
| (f) | CaSO₄-2H₂O | 0.5 g/L |
| (g) | ZnCl₂ | 20 g/L |
| (h) | FeSO₄-7H₂O | 65 g/L |
| (i) | Biotin | 0.2 g/L |
| (j) | Conc. sulfuric acid | 5 mL/L |

The culture solution was centrifuged at 5,000 rpm, 4°C for 10 minutes to collect the bacterial cells. To remove the medium components, the bacterial cells were suspended in potassium phosphate buffer (50 mM, pH 6.0) and then centrifuged to collect the bacterial cells. These procedures were repeated total of twice to prepare the bacterial cells. 557 g of the bacterial cells of TT080417-1-8 strain and 590 g of the bacterial cells of TT080724-1-11 strain were obtained.

### (9-2) Extraction and Purification of Crude Collagen

557 g of the bacterial cells of TT080417-1-8 strain (see in Example (9-1)) and 590 g of the bacterial cells of TT080724-1-11 strain (see in Example (4-1)) were subjected to disruption to extract and purify collagens. The disruption of the bacterial cells was conducted using DYNO-MILL Type KDL-A (Willy A. Bachofen AG).
The bacterial cells were suspended at a concentration of 40%(W/W) in potassium phosphate buffer (50 mM, pH 6.0) to prepare a suspension. Using a feed pump, the suspension was applied at a flow rate of 4,000 g/hour to the DYNO-MILL which was set to conditions for yeast disruption.
Potassium phosphate buffer (50 mM, pH 6.0) was added to reduce the concentration of the disrupted solution of the bacterial cells to half of the concentration. Conc. hydrochloric acid was used to adjust the pH of the solution to strong acidity (i.e., pH 1.5 or less) at 0.2 N of the final concentration. After pepsin (P7000, SIGMA) was added until the final concentration was equal to 5 mg/ml, the disrupted solution of the bacterial cells was incubated at 4°C with stirring. Approximately 96 hours later, the disrupted solution of the bacterial cells was centrifuged at 9,000 rpm, 4°C for 30 minutes to remove the insoluble fraction so as to provide supernatant. By adding 8 N NaOH, the resulting supernatant was adjusted to pH of approximately 10 and then incubated at 4°C with stirring. Approximately 16 hours later, the supernatant was centrifuged at 9,000 rpm, 4°C for 30 minutes to remove the insoluble fraction so as to provide supernatant.
Acetic acid was added into the resulting supernatant so that the final concentration could be 0.5 M, and cone. hydrochloric acid was used to adjust pH to 3.0, and then the mixture was incubated at 4°C with stirring. Approximately 16 hours later, the supernatant was centrifuged at 9,000 rpm, 4°C for 30 minutes to remove the insoluble fraction so as to provided supernatant. Into the resulting supernatant, NaCl was added so that the final concentration could be 1 M, and then incubated at 4°C with stirring. Approximately 16 hours later, the supernatant was centrifuged at 9,000 rpm, 4°C for 30 minutes to collect the insoluble fraction. Into the insoluble fraction, 0.1 N HCl was added and then incubated at 4°C with stirring to prepare a dissolved solution of the insoluble fraction. Approximately 16 hours later, the solution was centrifuged at 9,000 rpm, 4°C for 30 minutes to remove the insoluble fraction so as to provide supernatant.
Into the resulting supernatant, 1M potassium phosphate buffer (pH 7.4) was added so that the final concentration could be 0.05 M, and then, pH of the supernatant was adjusted to approximately 7.4 using 8 N NaOH. After NaCl was dissolved therein at the final concentration of 4 M, the supernatant was incubated at 4°C with stirring. Approximately 16 hours later, the supernatant was centrifuged at 9,000 rpm, 4°C for 30 minutes to collect an insoluble fraction. 0.1 N HCl was added into the insoluble fraction so that the weight of added 0.1 N HCl could be 2.8-fold higher compared to the weight of the insoluble fraction and then incubated at 4°C with stirring. Approximately 16 hours later, the insoluble fraction was centrifuged at 9,000 rpm, 4°C for 30 minutes to collect the insoluble fraction. Into the insoluble fraction, 0.1 N HCl was added, followed by incubation at 4°C with stirring to prepare a dissolved solution of the insoluble fraction. Approximately 16 hours later, the solution was centrifuged at 9,000 rpm, 4°C for 30 minutes to remove the insoluble fraction to prepare supernatant.
Into the resulting supernatant, acetic acid was added so that the final concentration could be 0.5 M, and then, conc. hydrochloric acid was used to adjust the pH of the supernatant to 3.0. Into the supernatant, NaCl was dissolved at the final concentration of 2 M, followed by incubation at 4°C with stirring. Approximately 16 hours later, the supernatant was centrifuged at 9,000 rpm, 4°C for 30 minutes to collect the insoluble fraction. After 0.1 N HCl was added into the insoluble fraction, the insoluble fraction was incubated at 4°C with stirring to prepare a dissolved solution. Approximately 16 hours later, the solution was centrifuged at 9,000 rpm, 4°C for 30 minutes to remove the insoluble fraction so as to provide supernatant.
The resulting supernatant was added into a dialysis tube (Spectra/Por 132665, SPECTUM LABORATORIES), dialyzed three times with 1 mM HCl solution, volume of which is ten-fold larger than the volume of the supernatant, and then filtered with 0.22 µm filter (Millex GP, manufactured by Millipore Corp.). 67.6 g, 25.3 g and 34.7 g of the purified collagen solutions were obtained from TT070327-1-11 strain, from TT080417-1-8 strain and from TT080724-1-1 strain, respectively.

### (9-3) Electrophoresis of Collagen

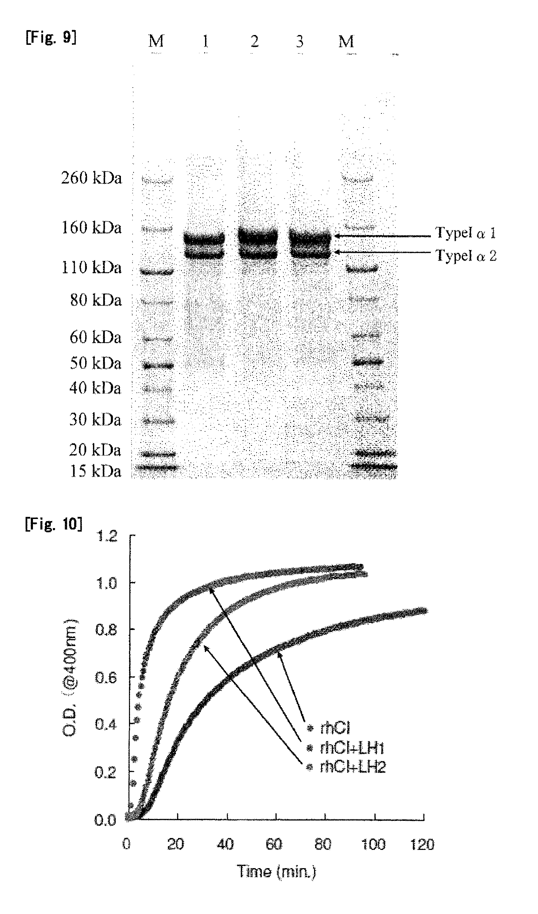

The purified collagen products were subjected to SDS polyacrylamide gel electrophoresis in according with the procedures described in Laemmli, U. K., Nature, 227, 680 (1970). Each electrophoresed gel was stained with a Page Blue 83 staining fluid (manufactured by Nacalai Tesque., Inc.) In Fig. 9, Lane 2 shows an electrophoretic profile of the purified collagen product from the TT080417-1-8 strain, and Lane 3 shows an electrophoretic profile of the purified collagen product from the TT080724-1-11 strain. As a result from electrophoresis (see in Fig. 9), in each lane, two distinct bands were observed, wherein on the gel, the higher molecular band was the one derived from Type I α1 and the lower molecular band was the one derived from Type I α2.

### (9-4) Analysis of Amino Acid

A 0.1 M hydrochloric acid solution containing 1 mg of the recombinant human collagen isolated from TT070327-1-11 strain was added into a hydrolysis tube and dried under reduced pressure before the addition of 500 µl of 6M hydrochloric acid solution. Air in the hydrolysis tube was purged with nitrogen and then sealed under reduced pressure.
The sealed hydrolysis tube was incubated at 110°C for 24 hours to hydrolyze the purified recombinant human collagen. After the hydrolysis tube was opened, the hydrochloric acid solution therein was dried under reduced pressure.
The hydrolyzed human collagen was dissolved in 2 ml of 20 mM HCl, and 10 µg of the resulting hydrolysate were examined by ninhydrin coloring method (on Hitachi Model L-8800 high performance amino acid analyzer).
As a column, ion-exchang resin column (#2622) was used, and as eluents, commercially available products: L-8500 Buffer Solution PH-1 (021-09111, Wako Pure Chemical Industries, Ltd.), L-8500 Buffer Solution PH-2 (028-09121, Wako Pure Chemical Industries, Ltd.), L-8500 Buffer Solution PH-3 (025-09131, Wako Pure Chemical Industries Ltd.), L-8500 Buffer Solution PH-4 (022-09141, Wako Pure Chemical Industries, Ltd.) and L-8500 Column Regenerating Solution PH (PH-RG) (036-12531, Wako Pure Chemical Industries, Ltd.) were used. For color development of the amino acid by ninhydrin coloring method, commercially available Ninhydrin Solution Set (201-06251, Wako Pure Chemical Industries, Ltd.) was used. The amino acid standard mixture was prepared and used by adding trans-4-Hydroxy-L-PROLINE (H-6002, Sigma) and DL-plus allo-δ Hydroxylysine, HCl (3920, CALBIO. CHEM.) into commercially available AMINO ACID CALIBRATION MIXTURE (782-3140, Hitachi High-Tech Fielding Corporation) and then adjusting the amino acid level to be 100 nmol/mL, wherein each concentration of proline and hydroxyproline was 200 nmol/mL.
As shown in Table 2, it was found that in this example, a composition ratio of the hydroylated lysine to the total lysine residues was increased up to 50.1%.

**Table 2: Amino acid composition of purified collagen**

| | Purified product of 060713-1-3 strain*1 | Purified product of TT080417-1-8 strain*2 | Wild type human Type I collagen*3 |
|---|---|---|---|
| Asp | 42.6 | 42.0 | 43 |
| Ser | 34.4 | 34.2 | 33 |
| Thr | 17.1 | 17.0 | 17 |
| Glu | 69.1 | 68.6 | 71 |
| Gly | 332.4 | 332.1 | 335 |
| Ala | 116.8 | 116.0 | 111 |
| Cys | ND | ND. | ND |
| Val | 25.1 | 24.8 | 26 |
| Met | 6.4 | 6.5 | 6 |
| Ile | 10.1 | 10.0 | 9 |
| Leu | 23.5 | 23.7 | 23 |
| Tyr | 1.8 | 1.7 | 2 |
| Phe | 12.3 | 12.3 | 12 |
| Hyl | 0.0 | 17.6 | 10 |
| Lys | 34.2 | 17.5 | 23 |
| His | 4.9 | 4.9 | 6 |
| Arg | 49.8 | 49.3 | 50 |
| Hyp | 100.8 | 102.0 | 103 |
| Pro | 119.2 | 119.7 | 120 |
| Total | 1000 | 1000 | 1000 |

| | | | |
|---|---|---|---|
| *1 Recombinant human collagen Type I (without hydroxylation of lysine) *2 Recombinant human collagen Type I (with coexpression of lysyl hydroxylase 1) *3 Literature data: Miller E.J., Gay S., Methods in Enzymology 82, 3-32 | | | |

### (9-5) Measurement of Ability for Fibril Formation

The purified recombinant human collagen was dialyzed with 1 mM HCl and then diluted with the external solution derived from dialysis to be 0.24% of collagen concentration. Next, 6-food concentrated D-PBS(-) (which was obtained by dissolving 8 g of sodium chloride, 1.15 g of disodium hydrogen phosphate, 0.2 g of potassium chloride and 0.2 g of potassium hydrogen phosphate in water to be made up to 1000 mL) was added thereto to adjust the collagen concentration to be final 0.20% and the D-PBS(-) concentration to be 1-fold. The pH after preparation was between 7.3 and 7.4. After the purified recombinant human collagen was added into a quartz glass cell surrounded by a jacket, the cell was deaerated under reduced pressure using an oil-sealed rotary pump and then placed on the cell stand in the spectrophotometer (U-2000, Hitachi). Absorbance at 400 nm of wave length was measured with time while allowing the circulating water at 37°C to flow (sampling points: every 30 second).
As shown in Fig. 10, it was found that regarding both of the collagen prepared from TT080417-1-8 strain and collagen obtained from TT080724-1-11 strain, ability for fibril formation was enhanced.

### Reference Example 1 (Preparation of Prolyl 4-Hydroxylase α1 Subunit and Propyl 4-Hydroxylase β Subunit Expression Yeast)

### (1-1) Transfecttion of Prolyl 4-Hydroxylase α1 Subunit Gene and Prolyl 4-Hydroxylase β Subunit Gene into Yeast

The Gene Transfer Plasmid pEXP-A-P4HBsig(-)Alrev was transfected into the Komagataella pastoris PPY12 strain so as to be chromosomally integrated through homologous recombination. The gene transfer procedures will be described below.
Into 100 ml of YPD liquid medium (which was prepared by dissolving 1 g of Yeast Extract and 2 g of Bacto Peptone into ion exchanged water to be made up to 90 ml; and autoclave sterilization of it; mixing the medium with 10 ml of 20% glucose which was subjected to filter sterilization separately), Komagataella pastoris PPY12 strain was inoculated to cultivate them at 30°C until the turbidity (OD₆₀₀) reached approximately 10. 80 ml of the culture solution was cooled in an ice bath, and then centrifuged at 3,000 g, 4°C for 10 minutes to precipitate bacterial cells. After removing the supernatant, the bacterial cells were washed with equal, 1/2 and then 1/4 parts of ice-chilled sterile distilled water in turn. After that, the bacterial cells were suspended in 1/4 parts of ice-chilled 1M sorbitol and then centrifuged at 3,000 g, 4°C for 10 minutes to collect them. The resulting bacterial cells were suspended in ice-chilled 1M sorbitol to adjust OD₆₀₀ into approximately 150.

Ten (10) µg of pEXP-A-P4HBsig(-)Alrev were cut with the restriction enzyme AatII. The DNA was collected through ethanol precipitation and dissolved into 5 µl of 10 mM Tris-HCl to prepare DNA solution.
Into a sterile test tube, 100 µl of the bacterial cell suspension was added to be mixed with 5 µl of the DNA solution. After a gene transfer device (ECM630, manufactured by BTX) was set to the specific yeast conditions (1,500 V/2 mm, 25 µF, 200 Ω of parallel resistance), and the mixture was transfered into a cuvette with electrode, the cuvette was placed in the device to apply pulse voltage. After that, into the resulting mixture, 1 ml of 1M sorbitol was added and mixed. The mixture was inoculated in an amount of 200 µl on a MD agar medium (final concentration: 1.34% Yeast Nitrogen Base, 4 × 10⁻⁵% biotin, 2% glucose, 0.005% of histidine, 2% agar) to cultivate them at 30°C for 48 hours. A colony formed on the MD agar medium was isolated as a transformed yeast.

### (1-2) Determination of Transfection of Prolyl 4-hydroxylase α1 Subunit Gene

PCR was conducted using the chromosomal DNA of the transformed yeast as a template to determine the integration of prolyl 4-hydroxylase α1 subunit gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, it was incubated at -80°C for 10 minutes and then at 98°C for 5 minutes before centrifugation at 12,000 rpm, 4°C for 10 minutes. The supernatant was collected to be used as a chromosomal DNA extract.
Oligonucleotides 27 and 28 were synthesized. The oligonucleotide 27 is oligonucleotide located on the 5' end of prolyl 4-hydroxylase α1 subunit gene, and the oligonucleotide 28 is oligonucleotide located on the 3' end of the gene, respectively.
The double-stranded DNA fragment of prolyl 4-hydroxylase α1 subunit gene was amplified by PCR using the following Oligonucleotides 27 and 28, as well as the chromosomal DNA extract as a template.
(a) Oligonucleotide 27: TATTCGAAACGATGATCTGGTATATATTAATTATA (SEQ ID NO: 27)
(b) Oligonucleotide 28: TTGCTAGCTCATTCCAATTCTGACAACGTACAAGG (SEQ ID NO: 28)

As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 99°C for 2 minutes followed by 40 cycles of denaturation at 94°C for 15 seconds, annealing at 60°C for 30 seconds and extension at 68°C for 4 minutes.
The solution obtained from the PCR was subjected to agarose gel electrophoresis, and then a distinct single band was observed at about 1,600 bp on the gel. This obviously shows that prolyl 4-hydroxylase α1 subunit gene has been transfected into the chromosome.

### (1-3) Determination of Transfecttion of Prolyl 4-Hydroxylase β Subunit Gene

PCR was conducted using the chromosomal DNA of the transformed yeast as a template to determine the integration of β subunit of prolyl 4-hydroxylase gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, the bacterial cells were incubated at -80°C for 10 minutes and then at 98°C for 5 minutes before centrifugation at 12,000 rpm, 4°C for 10 minutes. A supernatant was collected to be used as a chromosomal DNA extract.
Oligonucleotides 33 and 34 were synthesized. The oligonucleotide 33 is oligonucleotide located on the 5' end of β subunit of propyl 4-Hydroxylase gene, and the oligonucleotide 34 is oligonucleotide located on the 3' end of the gene, respectively.
The double-stranded DNA fragment of β subunit of prolyl 4-hydroxylase gene was amplified by PCR was conducted using the following Oligonucleotides 33 and 34 as primers and the chromosomal DNA extract as a template.
(a) Oligonucleotide 33: TTACTAGTGACGCCCCCGAGGAGGA (SEQ ID NO: 33)
(b) Oligonucleotide 34: TTACTAGTTTACAGTTCATCTTTCACAGCTTTCT (SEQ ID NO: 34)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR, buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 60°C for 30 seconds and extension at 68°C for 4 minutes.
The solution obtained from the PCR was subjected to agarose gel electrophoresis, and then a distinct single band was observed at about 1,500 bp on the gel. This obviously shows that β subunit of prolyl 4-hydroxylase gene has been transfected into the chromosome.
In conclusion, as the result of determination of gene transfer in 1-2 and 1-3, 050518-3-1 strain into which α subunit of prolyl 4-hydroxylase gene and β subunit of prolyl 4-hydroxylase gene have been transfected was obtained.

### Reference Example 2 (Preparation of Prolyl 4-Hydroxylase α1 Subunit, Prolyl 4-Hydroxylase β Subunit and Human Collagen Type III Expression Yeast)

### (2-1) Transfecttion of Human Collagen Type III Gene into Yeast

In order to be chromosomally integrated through homologous recombination, the Gene Transfer Plasmid pEXP-HA-HsCOL3A1 was transfected into the yeast 050518-3-1 strain into which prolyl 4-hydroxylase α1 subunit gene and β subunit of prolyl 4-hydroxylase gene have been transfected. The gene transfer procedures will be described below.
Into 100 ml of YPD liquid medium, the yeast 050518-3-1 strain was inoculated to cultivate them at 30°C so that OD₆₀₀ could be approximately 10 of turbidity. 80 ml of the culture solution was cooled in an ice bath, and then centrifuged at 3,000 g, 4°C for 10 minutes to precipitate bacterial cells. After removing the supernatant, the bacterial cells were washed with equal, 1/2 and then 1/4 parts of ice-chilled sterile distilled water in turn. After that, the bacterial cells were suspended in 1/4 parts of ice-chilled 1M sorbitol and then centrifuged at 3,000 g, 4°C for 10 minutes to collect the bacterial cells. The resulting bacterial cells were suspended in ice-chilled 1M sorbitol to adjust OD₆₀₀ into approximately 150.
Ten (10) µg of pEXP-HA-HsCOL3A1 were cut with the restriction enzyme XbaI. The DNA was collected through ethanol precipitation and dissolved into 5 µl of 10 mM Tris-HCl to give DNA solution.
Into a sterile test tube, 100 µl of the bacterial cell suspension was added to be mixed with 5 µl of the DNA solution. After a gene transfer device (ECM630, manufactured by BTX) was set to the specific yeast conditions (1,500 V/2 mm, 25 µF, 200 Ω of parallel resistance), and the mixture was transfered into a cuvette with electrode, the cuvette was placed in the device to apply pulse voltage. After that, into the resulting mixture, 1 ml of 1M sorbitol was added and mixed. Two hundreds (200) µl of the resulting mixture was inoculated on a MD agar medium to cultivate it at 30°C for 48 hours. A colony formed on the MD agar medium was isolated as a transformed yeast.

### (2-2) Determination of Transfecttion of Human Collagen Type III Gene

PCR was conducted using the chromosomal DNA of the transformed yeast as a template to determine integration of the human collagen Type III gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, the bacterial cells were incubated at -80°C for 10 minutes and then at 98°C for 5 minutes before centrifugation at 12,000 rpm, 4 °C for 10 minutes. A supernatant was collected to be used as a chromosomal DNA extract.
Oligonucleotides 50 and 51 were synthesized. The oligonucleotide 50 is oligonucleotide located on the 5' end of human collagen Type III gene, and the oligonucleotide 51 is oligonucleotide located on the 3' end of the gene, respectively.
The double-stranded DNA fragment of human collagen Type III gene was amplified by PCR, using the following oligonucleotides 50 and 51 as primers and the chromosomal DNA extract as a template.
(a) Oligonucleotide 50: TATTCGAAACGATGATGAGCTTTGTGCAAAAGGGG (SEQ ID NO: 50)
(b) Oligonucleotide 51: TTACTAGTTTATAAAAAGCAAACAGGGCCAACGT (SEQ ID NO: 51)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was condacted under the following conditions: heating the reaction solution at 94°C for 2 minutes followed by 40 cycles of denaturation at 94°C for 15 seconds, annealing at 60°C for 30 seconds and extension at 68°C for 5 minutes.
The solution obtained from the PCR was subjected to agarose gel electrophoresis, and then a distinct single band was observed at about 4,400 bp on the gel. This obviously shows that β subunit of prolyl 4-hydroxylase gene has been transfected into the chromosome.
For the yeast, the strain obtained by transfecting human collagen Type III gene into the 050518-3-1 strain will be referred to as TT061226-3-6 strain hereinafter.

### Reference Example 3 (Analysis of Collagen)

### (3-1) Cultivation of Yeast

To conduct preculture, the TT061226-3-6 strain was inoculated into 100 ml of BMGY medium and incubated it at 30°C for 20 hours.
Into 1 L of Basal salt medium prepared in 3 L jar fermentor (B. E. Marubisi Co., Ltd), the culture solution was inoculated so that OD₆₀₀ could be approximately 1.25, and then agitated with aeration to conduct main culture.
The main culture was conducted at 30°C, with setting maximum air flow rate 1.0 vvm (1 L). After the culture was used all availableglycerol in the medium, feeding of glycerol feed medium (50% glycerol, 1.2% PMT1 solution) was started. The glycerol fed-batch culture was stopped when OD₆₆₀ of the culture solution achieved approximately 140. Then, feeding of methanol feed culture (99.8% (w/v) methanol, 1.2% PMT1) was started to conduct the methanol fed-bafich culture. For the methanol fed-batch culture, culture temperature was changed to 32°C and dissolved oxygen concentration was controlled to approximately 8 ppm. During the cultivation, pH of the culture solution was maintained to be approximately 5.0 using 28% ammonia water and 4M phosphoric acid. After approximately 140 hours in culture, approximately 2 L of the culture solution were obtained. The composition of the reaction solutions used herein is given as follows:

| | | |
|---|---|---|
| (a) | (Composition of Basal salt medium) | |
| (b) | 85% H₃PO₄ | 26.7 mL/L |
| (c) | CaSO₄-2H₂O | 0.93 g/L |
| (d) | K₂SO₄ | 18.2 g/L |
| (e) | MgSO₄-7H₂O | 14.9 g/L |
| (f) | KOH | 4.13 g/L |
| (g) | Glycerol | 40 g/L |

After mixing the above components, the mixture was subjected to autoclave sterilization. After adjusted to pH 5.0 with 28% ammonia water, 2 ml/L of PMT1 solution and 1 ml/L of a solution of defoaming agent in methanol (12.5% AdeKanol LG295S) were further added.

### (Composition of PMT1 Solution)

| | | |
|---|---|---|
| (a) | CuSO₄-5H₂O | 6.0 g/L |
| (b) | KI | 0.8 g/L |
| (c) | MnSO₄-H₂O | 3.0 g/L |
| (d) | Na₂MoO₄-2H₂O | 0.2 g/L |
| (e) | H₃BO₄ | 0.2 g |
| (f) | CaSO₄₋2H₂O | 0.5 g/L |
| (g) | ZnCl₂ | 20 g/L |
| (h) | FeSO₄-7H₂O | 65 g/L |
| (i) | Biotin | 0.2 g/L |
| (j) | Conc. sulfuric acid | 5 mL/L |

### (3-2) Crude Purification of Collagen

The bacterial cells obtained from the cultivation were subjected to disruption, and then, collagen was purified from the resulting solution. The disruption of the bacterial cells was conducted using DYNO-MILL Type KDL-A (Willy A. Bachofen AG). The culture solution was centrifuged at 5,000 rpm, 4°C for 10 minutes to collect the bacterial cells. The obtained bacterial cells were suspended in potassium phosphate buffer (50 mM, pH 6.0) and then centrifuged to collect the bacterial cells. These procedures were repeated once more to remove the medium components, and then, 600 g of the wet bacterial cells were suspended in 1500 ml of potassium phosphate buffer (50 mM, pH6.0). Using a feed pump, the suspension was applied into the DYNO-MILL which was set to the conditions for yeast disruption. Completion of disruption was determined by examining the configuration of the bacterial cells under a microscope.
The solution of the disrupted bacterial cells was 2-fold diluted with potassium phosphate buffer (50 mM, pH 6.0). Conc. hydrochloric acid was used to adjust pH to approximately 2.0. Pepsin (purchased from Sigma) was added so that the final concentration could be 5 mg/ml. After incubation at 4°C for approximately 96 hours, the disrupted bacterial cells were centrifuged at 9,000 rpm, 4°C for 30 minutes to collect a supernatant. Into the resulting supernatant, 10 N NaOH was added to adjust pH to approximately 10. The supernatant was incubated at 4°C with stirring. Approximately 16 hours later, the supernatant was centrifuged at 9,000 rpm, 4°C for 30 minutes to collect a supernatant.

Into the resulting supernatant, acetic acid was added so that the final concentration was 0.5 M. Conc. hydrochloric acid was used to adjust pH to 3.0. The supernatant was then centrifuged at 9,000 rpm, 4°C for 30 minutes to collect a supernatant. Into the resulting supernatant, NaCl was added so that the final concentration was 1 M and then incubated at 4°C with stirring. Approximately 16 hours later, the supernatant was centrifuged at 9,000 rpm, 4°C for 30 minutes to collect a precipitate. The precipitate was dissolved in 0.1 N HCl.
Into the dissolved solution, 1M potassium phosphate buffer (pH 7.4) was added so that the final concentration was 0.05 M, and then, 10 N NaOH was used to adjust pH to approximately 7.4. After NaCl was added so that the final concentration was 2 M, the dissolved solution was incubated at 4°C with stirring. Approximately 16 hours later, the dissolved solution was centrifuged at 9,000 rpm, 4°C for 30 minutes to collect a precipitate. The resulting precipitate was dissolved in 0.1 N HCl and then centrifuged at 9,000 rpm, 4°C for 30 minutes to collect a supernatant.
Into the resulting supernatant, acetic acid was added so that the final concentration was 0.5 M. Conc. hydrochloric acid was used to adjust pH to approximately 3.0. Into the supernatant, NaCl was dissolved so that the final concentration was 1 M and then incubated at 4°C with stirring. Approximately 16 hours later, the supernatant was centrifuged at 9,000 rpm, 4°C for 30 minutes to collect a precipitate. The resulting precipitate was dissolved in 0.1 N HCl. The dissolved solution was centrifuged at 40,000 rpm, 4°C for 60 minutes to collect a supernatant.
The resulting supernatant was added into a dialysis tube (Spectra/Por, SPECTUM), dialyzed three times with 1 mM HCl solution, the volume of which was ten-fold larger than the volume of the supernatant, and then filtered with 0.22 µm filter (Millex GP, manufactured by Millipore Corp.) to give crude collagen product.

### (3-3) Purification of Collagen

Into the crude collagen product, 1M potassium phosphate buffer (pH7.4) was added so that the final concentration was 0.05 M, and then, the crude collagen product was adjusted to pH of approximately 7.4 by using 10 N NaOH. After NaCl was added so that the final concentration was 2 M, the crude collagen product was incubated at 4°C with stirring. Approximately 16 hours later, the crude collagen product was centrifuged at 9,000 rpm, 4°C for 30 minutes to collect a precipitate. The resulting precipitate was dissolved in 5-fold volume of 0.1 N HCl of the volume of the resulting precipitate and then centrifuged at 9,000 rpm, 4°C for 30 minutes to collect a precipitate.
Into the resulting supernatant, acetic acid was added so that the final concentration was 0.5 M. Conc. hydrochloric acid was used to adjust pH to approximately 3.0. After NaCl was added so that the final concentration was 1 M, the supernatant was incubated at 4°C with stirring. Approximately 16 hours later, the supernatant was centrifuged at 9,000 rpm, 4°C for 30 minutes to collect a precipitate. The resulting precipitate was dissolved in 0.1 N HCl. The dissolved solution was centrifuged at 40,000 rpm, 4°C for 60 minutes to collect a supernatant.
The resulting supernatant was added into a dialysis tube (Spectra/Por, SPECTRUM), dialyzed three times with 1 mM HCl solution, the volume of which is ten-fold larger than the volume of the supernatant, and then filtered with 0.22 µm filter (Millex GP, manufactured by Millipore Corp.) to give purified collagen product.

### Reference Example 4 (Preparation of Prolyl 4-Hydroxylase α1 Subunit, Prolyl 4-Hydroxylase β Subunit and Human Collagen Type I α1 and Human Collagen Type I α2 Expression Yeast)

### (4-1) Transfecttion of Human Collagen Type I α1 Gene and Human Collagen Type I α2 into Yeast

In order to be chromosomally integrated through homologous recombination, the Gene Transfer Plasmid pEXP-HA-HsCOL1A2-1A1 was transfected into the yeast 050518-3-1 strain into which prolyl 4-hydroxylase α1 subunit gene and β subunit of prolyl 4-hydroxylase gene have been transfected. The gene transfer procedures will be described below.
Into 100 ml of YPD liquid medium, the yeast 050518-3-1 strain was inoculated to cultivate it at 30°C until the turbidity (OD₆₀₀) reached approximately 10. 80 ml of the culture solution was cooled in an ice bath and then centrifuged at 3,000 g, 44°C for 10 minutes to precipitate bacterial cells. After removing the supernatant, the bacterial cells were washed with equal, 1/2 and then 1/4 parts of ice-chilled sterile distilled water in turn. After that, the bacterial cells were suspended in 1/4 parts of ice-chilled 1M sorbitol and then centrifuged at 3,000 g, 4°C for 10 minutes to collect the bacterial cells. The obtained bacterial cells were suspended in ice-chilled 1M sorbitol so that OD₆₀₀ was approximately 150.
Ten (10) µg of pEXP-HA-HSCOL1A2-1A1 were cut with the restriction enzyme XbaI. The DNA was collected through ethanol precipitation and dissolved into 5 µl of 10 mM Tris-HCl to give DNA solution.
Into a sterile test tube, 100 µl of the bacterial cell suspension was added to be mixed with 5 µl of the DNA solution. After a gene transfer device (ECM630, manufactured by BTX) was set to the specific yeast conditions (1,500 V/2 mm, 25 µF, 200 Ω of parallel resistance), and the mixture was transfered into a cuvette with electrode, the cuvette was placed in the device to apply pulse voltage. After that, into the resulting mixture, 1 ml of 1M sorbitol was added and mixed. Two hundreds (200) µl of the resulting mixture was inoculated on a MD agar medium to cultivate it at 30°C for 48 hours. A colony formed on the MD agar medium was isolated as a transformed yeast.

### (4-2) Determination of Transfection of Human Collagen Type I α1 Gene

PCR was conducted using the chromosomal DNA of the transformed yeast as a template to determine integration of the human collagen Type I α1 gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, the bacterial cells were incubated at -80°C for 10 minutes and then at 98°C for 5 minutes before centrifugation at 12,000 rpm, 4°C for 10 minutes. A supernatant was collected to be used as a chromosomal DNA extract.
Oligonucleotides 56 and 57 were synthesized. The oligonucleotide 56 is oligonucleotide located on the 5' end of human collagen Type I α1 gene, and the oligonucleotide 57 is oligonucleotide located on the 3' end of the gene, respectively.
The double-stranded DNA fragment of human collagen Type I α1 gene was amplified by PCR using the following oligonucleotides 56 and 57 as primers and the chromosomal DNA extract as a template.
(a) Oligonucleotide 56: TATTCGAAACGATGTTCAGCTTTGTGGACCTCCG (SEQ ID NO: 76)
(b) Oligonucleotide 57: TTACTAGTTTACAGGAAGCAGACAGGGCCAA (SEQ ID NO: 77)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 4°C for 15 seconds, annealing and extension at 68°C for 5 minutes.
The solution obtained from the PCR was subjected to agarose gel electrophoresis, and then a distinct single band was observed at about 4,400 bp on the gel. This obviously shows that human collagen Type I α1 gene has been transfected into the chromosome.

### (4-3) Determination of Transfecttion of Human Collagen Type I α2 Gene

PCR was conducted using the chromosomal DNA of the transformed yeast as a template to determine integration of the human collagen Type I α2 gene into the chromosome.
A small number of bacterial cells were taken from the colony formed on the MD agar medium, and suspended in 10 µl of sterile distilled water. Into the suspension, 5 µl of 2,000 u/µl Lyticase (purchased from Sigma) was added and incubated at 30°C for 20 minutes. Then, the bacterial cells was incubated at -80°C for 10 minutes and then at 98°C for 5 minutes before centrifugation at 12,000 rpm, 4°C for 10 minutes. A supernatant was collected to be used as a chromosomal DNA extract.
Oligonucleotides 58 and 59 were synthesized. The oligonucleotide 58 is oligonucleotide located on the 5' end of human collagen Type I α2 gene, and the oligonucleotide 59 is oligonucleotide located on the 3' end of the gene, respectively.
The double-stranded DNA fragment of human collagen Type I α2 gene was amplified by PCR using the following Oligonucleotides 58 and 59 as primers and the chromosomal DNA extract as a template.
(a) Oligonucleotide 58: TATTCGAAACGATGCTCAGCTTTGTGGATACGCG (SEQ ID NO: 78)
(b) Oligonucleotide 59: TTACTAGTTTATTTGAAACAGACTGGGCCAATGTC (SEQ ID NO: 79)
As a polymerase for the PCR, BlendTaq PCR polymerase, manufactured by Toyobo Co., Ltd., was used. The details of composition of the reaction solutions are given as follows:

| | | |
|---|---|---|
| (a) | Chromosomal DNA extract | 5 µl |
| (b) | dNTP (2 mM-mix each) | 5 µl |
| (c) | Primers (10 pmol/µl) | 1 µl each |
| (d) | 10 × PCR buffer for BlendTaq | 5 µl |
| (e) | BlendTaq DNA polymerase (1 U/µl) | 0.5 µl |
| (f) | Sterile distilled water | 32.5 µl |

PCR was conducted using PERKIN ELMER GeneAmp PCR System 9700. This reaction was conducted under the following conditions: heating the reaction solution at 94°C for 2 minutes followed by 40 cycles of denaturation at 94°C for 15 seconds, annealing at 60°C for 30 seconds and extension at 68°C for 5 minutes.
The solution obtained from the PCR was subjected to agarose gel electrophoresis, and therefore, a distinct single band was observed at about 4,100 bp on the gel. This obviously shows that human collagen Type I α2 gene has been transfected into the chromosome.
For the yeast, the strains obtained by transfecting both human collagen Type I α1 gene and human collagen Type I α2 gene into the 050518-3-1 strain will be referred to as 060713-1-3 strain hereinafter.

### Example 5 (Analysis of Collagen)

### (5-1) Cultivation of Yeast

To conduct preculture, the 060713-1-3 strain was inoculated into 100 ml of BMGY medium,and incubated it at 30°C for 20 hours.
In 3 L jar fermentor (B. E. Marubisi), 0.8 L of Basal salt medium was prepared. The culture solution was inoculated into the medium so that final concentration OD₆₆₀ was equal to approximately 1.25, and then agitated with aeration to conduct main culture.
The main culture was conducted with setting the culture temperature at 30°C, maximum air flow rate at 0.8 vvm (0.8 L) and agitation rate to 800 rpm. After all available glycerol in the medium was consumed and oxygen consumption was decreased, feeding of glycerol feed medium (50% glycerol, 1.2% PMT1 solution) was started at a flow rate of approximately 15 g/hour. The glycerol fed-batch culture was stopped when OD₆₆₀ of the culture solution achieved approximately 130. Then, feeding of methanol feed culture medium (98.6%(w/v) methanol, 1.2% PMT1 solution) was started to conduct the methanol fed-batch culture. After the start of the methanol fed-batch culture, its culture temperature was changed to 32°C and its dissolved oxygen concentration was controlled to be approximately 8 ppm. During the cultivation, pH of the culture solution was maintained to be approximately 5.0 using 28% ammonia water. After approximately 136 hours in culture, 1415 g of the culture solution of 060713-1-3 strain were obtained. The composition of the reaction solutions used herein is given as follows:

| | | |
|---|---|---|
| (a) | (Composition of Basal salt medium) | |
| (b) | 85% H₃PO₄ | 26.7 mL/L |
| (c) | CaSO₄-2H₂O | 0.93 g/L |
| (d) | K₂SO₄ | 18.2 g/L |
| (e) | MgSO₄-7H₂O | 14.9 g/L |
| (f) | SOH | 4.13 g/L |
| (g) | Glycerol | 40 g/L |

After mixing the above components, the mixture was subjected to autoclave sterilization. After adjusted to pH 5.0 with 28% ammonia water, 2 ml/L of PMT1 solution and 1 mill of a solution of defaming agent in methanol (12.5% Adekanol LG295S) were further added.

### (Composition of PMT1 Solution)

| | | |
|---|---|---|
| (a) | CuSO₄-5H₂O | 6.0 g/L |
| (b) | KI | 0.8 g/L |
| (c) | MnSO₄-H₂O | 3.0 g/L |
| (d) | Na₂MoO₄-2H₂O | 0.2 g/L |
| (e) | H₃BO₄ | 0.2 g |
| (f) | CaSO₄-2H₂O | 0.5 g/L |
| (g) | ZnCl₂ | 20 g/L |
| (h) | FeSO₄-7H₂O | 65 g/L |
| (i) | Biotin | 0.2 g/L |
| (j) | Conc. sulfuric acid | 5 mL/L |

The culture solution was centrifuged at 5,000 rpm, 4°C for 10 minutes to collect the bacterial cells. To remove the medium components, the bacterial cells were suspended in potassium phosphate buffer (50 mM, pH6.0), and then centrifuged to collect the bacterial cells. These procedures were repeated total twice to give the bacterial cells. 588 Grams of the bacterial cells of 060713-1-3 strain were obtained.

### (5-2) Extraction and Purification of Collagen

Collagen was extracted and purified by disrupting 588 g of the bacterial cells of 060713-1-3 strain (see in Reference Example (5-1)). The disruption of the bacterial cells was conducted using DYNO-MILL Type KDL-A (Willy A. Bachofen AG) .
The bacterial cells were suspended at a concentration of 40%(W/W) in potassium phosphate buffer (50 mM, pH 6.0) to prepare the suspension. Using a feed pump, the suspension was applied at a flow rate of 4,000 g/hour into the DYNE-MILL which was set to the condition for yeast disruption.
Potassium phosphate buffer (50 mM, pH 6.0) was added to reduce the concentration of the disrupted solution of the bacterial cells to half of the concentration. Conc. hydrochloric acid was used to adjust pH to strong acidity (i.e., pH 1.5 or less) at 0.2N of the final concentration. After pepsin (P7000, SIGMA) was added so that the final concentration was 5 mg/ml, the disrupted solution of the bacterial cells was incubated at 4°C with stirring. Approximately 96 hours later, the disrupted solution of the bacterial cells was centrifuged at 9,000 rpm, 4°C for 30 minutes to remove the insoluble fraction so as to provide supernatant. Into the resulting supernatant, 8 N NaOH was added to adjust pH to approximately 10, and then incubated at 4°C with stirring. Approximately 16 hours later, the supernatant was centrifuged at 9,000 rpm, 4°C for 30 minutes to remove the insoluble fraction so as to provide supernatant.
Into the resulting supernatant, acetic acid was added so that the final concentration was 0.5 M, and conc. hydrochloric acid was used to adjust pH to 3.0, and then, the supernatant was incubated at 4°C with stirring.
Approximately 16 hours later, the supernatant was centrifuged at 9,000 rpm, 4°C for 30 minutes to remove the insoluble fraction so as to provide supernatant. Into the resulting supernatant, NaCl was added so that the final concentration was 1 M, followed by incubation at 4°C with stirring. Approximately 16 hours later, the supernatant was centrifuged at 9,000 rpm, 4 °C for 30 minutes to collect the insoluble fraction. After 0.1 N HCl was added into the insoluble fraction, it was incubated at 4°C with stirring to prepare a dissolved solution. Approximately 16 hours later, the dissolved solution was centrifuged at 9,000 rpm, 4°C for 30 minutes to remove the insoluble fraction so as to provide supernatant.
Into the resulting supernatant, 1M potassium phosphate buffer (pH 7.4) was added so that the final concentration was 0.05 M, and then, 8 N NaOH was used to adjust pH to approximately 7.4. After NaCl was dissolved therein at the final concentration of 4 M, the supernatant was incubated at 4°C with stirring. Approximately 16 hours later, the supernatant was centrifuged at 9,000 rpm, 44°C for 30 minutes to collect an insoluble fraction. 0.1 N HCl was added to the insoluble fraction so that the weight was 2.8-fold higher compared to the weight of the insoluble fraction, and then incubated at 4°C with stirring. Approximately 16 hours later, the insoluble fraction was centrifuged at 9,000 rpm, 4°C for 30 minutes to collect the insoluble fraction. Into the insoluble fraction, 0.1 N HCl was added and incubated at 4°C with stirring to prepare a dissolved solution. Approximately 16 hours later, the dissolved solution was centrifuged at 9,000 rpm, 4°C for 30 minutes to remove the insoluble fraction so as to provide a supernatant.
Into the resulting supernatant, acetic acid was added so that the final concentration was 0.5 M, and conc. hydrochloric acid was used to adjust pH to approximately 3.0. Into the supernatant, NaCl was dissolved at the final concentration of 2 M and then incubated at 4°C with stirring. Approximately 16 hours later, the supernatant was centrifuged at 9,000 rpm, 4°C for 30 minutes to collect the insoluble fraction. After 0.1 N HCl was added into the insoluble fraction, the supernatant was incubated at 4°C with stirring to prepare a dissolved solution. Approximately 16 hours later, the dissolved solution was centrifuged at 9,000 rpm, 4°C for 30 minutes to remove the insoluble fraction so as to provide a supernatant.
The resulting supernatant was added into a dialysis tube (Spectra/For 132665, SPECTRUM LABORATORIES), dialyzed three times with 1 mM HCl solution, the volume of which was ten-fold larger than the volume of the supernatant, and then filtered with 0.22 µm filter (Millex GP, manufactured by Millipore Corp.). 35.6 Grams of the purified collagen solutions were obtained from 060713-1-3 strain.

### Industrial Applicability

According to the present invention, it becomes possible to provide collagen which is usable as a high performance versatile material that is more commercially valuable for pharmaceuticals, industrial products, cosmetics and foods. Specifically, for example, it becomes possible to provide collagen having a stabilized triple-helical structure and an increased ability for fibril formation, and a process for producing the collagen.

## Claims

1. A transformant in which all of the following genes (1), (2) and (3) :
(1) a foreign gene comprising a nucleotide sequence encoding an amino acid sequence of lysyl hydroxylase,
(2) a foreign gene comprising a nucleotide sequence encoding an amino acid sequence of prolyl hydroxylase, and
(3) one or more foreign genes comprising a nucleotide sequence encoding an amino acid sequence of collagen;
are transfected into a microbial cell.

2. The transformant according to claim 1, which coexpresses all of the proteins encoded by the genes (1), (2) and (3) within the cell.

3. The transformant according to claim 1, wherein the lysyl hydroxylase in (1) is one or more Lysyl hydroxylases selected from among lysyl hydroxylase 1, lysyl hydroxylase 2 or lysyl hydroxylase 3.

4. The transformant according to claim 1, wherein the propyl hydroxylase in (2) is an enzyme composed of α subunits of propyl 4-hydroxylase and β subunits of prolyl 4-hydroxylase.

5. The transformant according to claim 4, wherein the β subunit of prolyl 4-hydroxylase fuses with a secretory signal sequence of a yeast α-factor.

6. The transformant according to claim 4, wherein the α subunit of prolyl 4-hydroxylase is an α1 subunit, an α2 subunit or an α3 subunits.

7. The transformant according to claim 1, wherein the collagen in (3) is one or more collagens selected from among Type I to Type XXIX collagens.

8. The transformant according to claim 1, wherein at least one of the nucleotide sequence encoding the amino acid sequence of the lysyl hydroxylase in (1) and the nucleotide sequence encoding the prolyl hydroxylase in (2) is linked to downstream of a promoter derived from yeast.

9. The transformant according to claim 8, wherein the promoter is an alcohol oxidase 1 gene promoter.

10. The transformant according to claim 1, wherein the microbial cell is an eukaryote cell.

11. The transformant according to claim 10, wherein the eukaryote cell is a yeast cell.

12. The transformant according to claim 11, wherein the yeast cell is a methylotrophic yeast cell.

13. The transformant according to claim 12, wherein the methylotrophic yeast cell is Komagataella pastoris.

14. Collagen which is obtainable by being produced by the transformant according to claim 1.

15. The collagen according to claim 14, wherein 15% or more of total lysine residues are hydroxylated.

16. The collagen according to claim 14, wherein the lysine residues in a telopeptide region are hydroxylated.

17. A process for producing collagen, comprising:
a first step of transfecting all of the following genes (1), (2) and (3):
(1) a foreign gene comprising a nucleotide sequence encoding an amino acid sequence of lysyl hydroxylase,
(2) a foreign gene comprising a nucleotide sequence encoding an amino acid sequence of prolyl hydroxylase, and
(3) a foreign gene comprising a nucleotide sequence encoding an amino acid sequence of collagen;
into a microbial cell;
a second step of cultivating the transformant resulting from the first step to produce the collagen; and
a third step of collecting the collagen produced in the second step.

18. Collagen produced by the process according to claim 17.


SEQUENCE LISTING

<110>  SUMITOMO CHEMICAL COMPANY, LIMITED
       KOKEN CO., LTD.
       Dainippon Sumitomo Pharma Co., Ltd.

<120>  Transformants with the ability to produce the collagen which
       contains hydroxylated lysine residues and hydroxylated proline
residues

<130>  669943

<150>  JP 2009-260721
<151>  2009-11-16

<160>  83

<170>  PatentIn version 3.1

<210>  1
<211>  25
<212>  DNA
<213>  Artificial Sequence

<220>
<223>  primer for cloning of PLOD1

<400>  1
cgtcatatga tgcggcccct gctgc                                          25


<210>  2
<211>  30
<212>  DNA
<213>  Artificial Sequence

<220>
<223>  primer for cloning of PLOD1

<400>  2
atcctcgagt tagggatcga cgaaggagac                                     30


<210>  3
<211>  26
<212>  DNA
<213>  Artificial Sequence

<220>
<223>  primer for cloning of PLOD2

<400>  3
cgagatctga tgggggatg cacggt                                          26


<210>  4
<211>  33
<212>  DNA
<213>  Artificial Sequence

<220>
<223>  primer for cloning of PLOD2

<400>  4
gcagatctcg ttagggatct ataaatgaca ctg                                 33

<210>  5
<211>  30
<212>  DNA
<213>  Artificial Sequence

<220>
<223>  primer for cloning of HIS4

<400>  5
gatctcctga tgactgactc actgataata                                        30


<210>  6
<211>  30
<212>  DNA
<213>  Artificial Sequence

<220>
<223>  primer for cloning of HIS4

<400>  6
taattaaata agtcccagtt tctccatacg                                        30


<210>  7
<211>  25
<212>  DNA
<213>  Artificial Sequence

<220>
<223>  primer for cloning of ARG4

<400>  7
acgaaaatat ggtacctgcc ctcac                                             25


<210>  8
<211>  30
<212>  DNA
<213>  Artificial Sequence

<220>
<223>  primer for cloning of ARG4

<400>  8
gttctatcta cccgaggaaa ccgatacata                                        30


<210>  9
<211>  27
<212>  DNA
<213>  Artificial Sequence

<220>
<223>  primer for cloning of AOX1 promoter

<400>  9
agatctaaca tccaaagacg aaaggtt                                           27


<210>  10
<211>  29
<212>  DNA

<213>  Artificial Sequence

<220>
<223>  primer for cloning of AOX1 promoter

<400>  10
atccaccacc tagaactagg atatcaaac                                          29


<210>  11
<211>  26
<212>  DNA
<213>  Artificial Sequence

<220>
<223>  primer for cloning of AOX1 terminator

<400>  11
ccttagacat gactgttcct cagttc                                             26


<210>  12
<211>  24
<212>  DNA
<213>  Artificial Sequence

<220>
<223>  primer for cloning of AOX1 terminator

<400>  12
gcacaaacga acgtctcact taat                                               24


<210>  13
<211>  30
<212>  DNA
<213>  Artificial Sequence

<220>
<223>  primer for cloning of AOX1 3' non coding region

<400>  13
tcgagtatct atgattggaa gtatgggaat                                         30


<210>  14
<211>  30
<212>  DNA
<213>  Artificial Sequence

<220>
<223>  primer for cloning of AOX1 3' non coding region

<400>  14
gatcttgaga taaatttcac gtttaaaatc                                         30


<210>  15
<211>  32
<212>  DNA
<213>  Artificial Sequence

<220>
<223>  primer for cloning of alpha factor

<400>  15
tcaaacaaga agattacaaa ctatcaattt ca                                    32


<210>  16
<211>  31
<212>  DNA
<213>  Artificial Sequence

<220>
<223>  primer for cloning of alpha factor

<400>  16
tttgttacat ctacactgtt gttatcagtc g                                     31


<210>  17
<211>  20
<212>  DNA
<213>  Artificial Sequence

<220>
<223>  primer for cloning of human TypeIII collagen

<400>  17
ggctgagttt tatgacgggc                                                  20


<210>  18
<211>  20
<212>  DNA
<213>  Artificial Sequence

<220>
<223>  primer for cloning of human TypeIII collagen

<400>  18
gacaagatta gaacaagagg                                                  20


<210>  19
<211>  35
<212>  DNA
<213>  Artificial Sequence

<220>
<223>  primer for cloning of AOX1 terminator with addition of restriction
       enzyme site

<400>  19
tcgactagtt tagacatgac tgttcctcag ttcaa                                 35


<210>  20
<211>  30
<212>  DNA
<213>  Artificial Sequence

<220>
<223>  primer for cloning of AOX1 terminator with addition of restriction
       enzyme site

<400>  20
aactgcaggc acaaacgaac gtctcactta                                       30

<210>  21
<211>  35
<212>  DNA
<213>  Artificial Sequence

<220>
<223>  linker 1 for construction of pSN005

<400>  21
tattcgaaac gcatatgtga ccggcagact agtgg                                35


<210>  22
<211>  35
<212>  DNA
<213>  Artificial Sequence

<220>
<223>  linker 1 for construction of pSN005

<400>  22
ccactagtct gccggtcaca tatgggtttc gaata                                35


<210>  23
<211>  35
<212>  DNA
<213>  Artificial Sequence

<220>
<223>  linker 2 for construction of pSN006

<400>  23
tattcgaaac gcatatggta ccggcagact agtgg                                35


<210>  24
<211>  35
<212>  DNA
<213>  Artificial Sequence

<220>
<223>  linker 2 for construction of pSN006

<400>  24
ccactagtcg cctaggcgac atatggtttc gaata                                35


<210>  25
<211>  35
<212>  DNA
<213>  Artificial Sequence

<220>
<223>  linker 3 for construction of pSN007

<400>  25
tattcgaaac gacgcgtgtc agctagcact agtgc                                35


<210>  26
<211>  35
<212>  DNA
<213>  Artificial Sequence

<220>
<223>  linker 3 for construction of pSN007

<400>  26
gcactagtgc tagctgacac gcgtcgtttc gaata                                35


<210>  27
<211>  35
<212>  DNA
<213>  Artificial Sequence

<220>
<223>  primer for cloning of prolyl-4-hydroxyrase alpha 1(P4HA1)
       subunit with addition of restriction enzyme site

<400>  27
tattcgaaac gatgatctgg tatatattaa ttata                                35


<210>  28
<211>  35
<212>  DNA
<213>  Artificial Sequence

<220>
<223>  primer for cloning of prolyl-4-hydroxyrase alpha 1(P4HA1)
       subunit with addition of restriction enzyme site

<400>  28
ttgctagctc attccaattc tgacaacgta caagg                                35


<210>  29
<211>  30
<212>  DNA
<213>  Artificial Sequence

<220>
<223>  primer for cloning of ARG4 with addition of restriction enzyme
       site

<400>  29
aactcgagac gaaaatatgg tacctgccct                                      30


<210>  30
<211>  32
<212>  DNA
<213>  Artificial Sequence

<220>
<223>  primer for cloning of ARG4 with addition of restriction enzyme
       site

<400>  30
ccatcgatac agaggtatca tccaatgatt cc                                   32


<210>  31
<211>  35
<212>  DNA
<213>  Artificial Sequence

<220>
<223>  primer for construction of expression vector of alpha factor signal
       sequence with addition of restriction enzyme site

<400>  31
ggttcgaaac gatgagattt ccttcaattt ttact                                35


<210>  32
<211>  31
<212>  DNA
<213>  Artificial Sequence

<220>
<223>  primer for construction of expression vector of alpha factor signal
       sequence with addition of restriction enzyme site

<400>  32
tcgactagta gcttcagcct ctcttttatc c                                    31


<210>  33
<211>  25
<212>  DNA
<213>  Artificial Sequence

<220>
<223>  primer for cloning of prolyl-4-hydroxyrase beta(P4HB) subunit
       with addition of restriction enzyme site

<400>  33
ttactagtga cgcccccgag gagga                                           25


<210>  34
<211>  35
<212>  DNA
<213>  Artificial Sequence

<220>
<223>  primer for cloning of prolyl-4-hydroxyrase beta(P4HB) subunit
       with addition of restriction enzyme site

<400>  34
ttactagttt acagttcatc tttcacagct ttctg                                 35


<210>  35
<211>  35
<212>  DNA
<213>  Artificial Sequence

<220>
<223>  primer for cloning of expression unit of P4HB with alpha signal

<400>  35
aaccgcggtc taacatccaa agacgaaagg ttgaa                                35


<210>  36
<211>  35
<212>  DNA
<213>  Artificial Sequence

<220>

<223>  primer for cloning of expression unit of P4HB with alpha signal

<400>  36
aacccggggc acaaacgaac gtctcactta atctt                                35


<210>  37
<211>  35
<212>  DNA
<213>  Artificial Sequence

<220>
<223>  primer for cloning of expression unit of P4HA1 with alpha signal

<400>  37
aacccgggtc taacatccaa agacgaaagg ttgaa                                35


<210>  38
<211>  24
<212>  DNA
<213>  Artificial Sequence

<220>
<223>  primer for cloning of  second half part of PLOD1 with addition of
       restriction enzyme site

<400>  38
gaccttcgaa acaggctgca ccgt                                            24


<210>  39
<211>  23
<212>  DNA
<213>  Artificial Sequence

<220>
<223>  primer for cloning of  second half part of PLOD1 with addition of
       restriction enzyme site

<400>  39
cgactagttt agggatcgac gaa                                             23


<210>  40
<211>  30
<212>  DNA
<213>  Artificial Sequence

<220>
<223>  primer for cloning of  first half part of PLOD1 with addition of
       restriction enzyme site

<400>  40
tattcgaaac gatgcggccc ctgctgctac                                      30


<210>  41
<211>  28
<212>  DNA
<213>  Artificial Sequence

<220>
<223>  primer for cloning of  first half part of PLOD1 with addition of
       restriction enzyme site

<400>  41
cagcctgttt cgaaggtcca gaagcgcg                                          28


<210>  42
<211>  31
<212>  DNA
<213>  Artificial Sequence

<220>
<223>  primer for cloning of expression unit of PLOD1 or PLOD2

<400>  42
aaggtacctc taacatccaa agacgaaagg t                                      31


<210>  43
<211>  30
<212>  DNA
<213>  Artificial Sequence

<220>
<223>  primer for cloning of expression unit of PLOD1 or PLOD2

<400>  43
aaggtaccgc acaaacgaac gtctcactta                                        30


<210>  44
<211>  29
<212>  DNA
<213>  Artificial Sequence

<220>
<223>  primer for construction of PLOD2 expression unit

<400>  44
tattcgaaac gatgggggga tgcacggtg                                         29


<210>  45
<211>  31
<212>  DNA
<213>  Artificial Sequence

<220>
<223>  primer for construction of PLOD2 expression unit

<400>  45
cgactagttt agggatctat aaatgacact g                                      31


<210>  46
<211>  32
<212>  DNA
<213>  Artificial Sequence

<220>
<223>  primer for cloning of HIS4 with addition of restriction enzyme site

<400>  46
ggaagcttga tctcctgatg actgactcac tg                                     32

<210>  47
<211>  34
<212>  DNA
<213>  Artificial Sequence

<220>
<223>  primer for cloning of HIS4 with addition of restriction enzyme site

<400>  47
ccctgcagta attaaataag tcccagtttc tcca                                34


<210>  48
<211>  34
<212>  DNA
<213>  Artificial Sequence

<220>
<223>  primer for cloning of AOX1 3′ non coding region with addition of
       restriction enzyme site

<400>  48
gcatcgattc gagtatctat gattggaagt atgg                                34


<210>  49
<211>  35
<212>  DNA
<213>  Artificial Sequence

<220>
<223>  primer for cloning of AOX1 3\201f non coding region with addition
of\201@
       restriction enzyme site

<400>  49
aagggcccga tcttgagata aatttcacgt ttaaa                               35


<210>  50
<211>  35
<212>  DNA
<213>  Artificial Sequence

<220>
<223>  primer for cloning of human collagen TypeIII
        with addition of restriction enzyme site

<400>  50
tattcgaaac gatgatgagc tttgtgcaaa agggg                               35


<210>  51
<211>  34
<212>  DNA
<213>  Artificial Sequence

<220>
<223>  primer for cloning of human collagen TypeIII
        with addition of restriction enzyme site

<400>  51
ttactagttt ataaaaagca aacagggcca acgt                                34

<210>  52
<211>  727
<212>  PRT
<213>  Homo sapiens

<400>  52

Met Arg Pro Leu Leu Leu Leu Ala Leu Leu Gly Trp Leu Leu Leu Ala
1               5                   10                  15

Glu Ala Lys Gly Asp Ala Lys Pro Glu Asp Asn Leu Leu Val Leu Thr
            20                  25                  30

Val Ala Thr Lys Glu Thr Glu Gly Phe Arg Arg Phe Lys Arg Ser Ala
        35                  40                  45

Gln Phe Phe Asn Tyr Lys Ile Gln Ala Leu Gly Leu Gly Glu Asp Trp
    50                  55                  60

Asn Val Glu Lys Gly Thr Ser Ala Gly Gly Gly Gln Lys Val Arg Leu
65                  70                  75                  80

Leu Lys Lys Ala Leu Glu Lys His Ala Asp Lys Glu Asp Leu Val Ile
                85                  90                  95

Leu Phe Thr Asp Ser Tyr Asp Val Leu Phe Ala Ser Gly Pro Arg Glu
            100                 105                 110

Leu Leu Lys Lys Phe Arg Gln Ser Arg Ser Gln Val Val Phe Ser Ala
        115                 120                 125

Glu Glu Leu Ile Tyr Pro Asp Arg Arg Leu Glu Thr Lys Tyr Pro Val
    130                 135                 140

Val Ser Asp Gly Lys Arg Phe Leu Gly Ser Gly Gly Phe Ile Gly Tyr
145                 150                 155                 160

Ala Pro Asn Leu Ser Lys Leu Val Ala Glu Trp Glu Gly Gln Asp Ser
                165                 170                 175

Asp Ser Asp Gln Leu Phe Tyr Thr Lys Ile Phe Leu Asp Pro Glu Lys
            180                 185                 190

Arg Glu Gln Ile Asn Ile Thr Leu Asp His Arg Cys Arg Ile Phe Gln
        195                 200                 205

Asn Leu Asp Gly Ala Leu Asp Glu Val Val Leu Lys Phe Glu Met Gly
    210                 215                 220

His Val Arg Ala Arg Asn Leu Ala Tyr Asp Thr Leu Pro Val Leu Ile
225                 230                 235                 240

His Gly Asn Gly Pro Thr Lys Leu Gln Leu Asn Tyr Leu Gly Asn Tyr
                245                 250                 255

Ile Pro Arg Phe Trp Thr Phe Glu Thr Gly Cys Thr Val Cys Asp Glu
            260                 265                 270

Gly Leu Arg Ser Leu Lys Gly Ile Gly Asp Glu Ala Leu Pro Thr Val
        275                 280                 285

Leu Val Gly Val Phe Ile Glu Gln Pro Thr Pro Phe Val Ser Leu Phe
    290                 295                 300

Phe Gln Arg Leu Leu Arg Leu His Tyr Pro Gln Lys His Met Arg Leu
305                 310                 315                 320

Phe Ile His Asn His Glu Gln His His Lys Ala Gln Val Glu Glu Phe
                325                 330                 335

Leu Ala Gln His Gly Ser Glu Tyr Gln Ser Val Lys Leu Val Gly Pro
            340                 345                 350

Glu Val Arg Met Ala Asn Ala Asp Ala Arg Asn Met Gly Ala Asp Leu
        355                 360                 365

Cys Arg Gln Asp Arg Ser Cys Thr Tyr Tyr Phe Ser Val Asp Ala Asp
    370                 375                 380

Val Ala Leu Thr Glu Pro Asn Ser Leu Arg Leu Leu Ile Gln Gln Asn
385                 390                 395                 400

Lys Asn Val Ile Ala Pro Leu Met Thr Arg His Gly Arg Leu Trp Ser
                405                 410                 415

Asn Phe Trp Gly Ala Leu Ser Ala Asp Gly Tyr Tyr Ala Arg Ser Glu
            420                 425                 430

Asp Tyr Val Asp Ile Val Gln Gly Arg Arg Val Gly Val Trp Asn Val
        435                 440                 445

Pro Tyr Ile Ser Asn Ile Tyr Leu Ile Lys Gly Ser Ala Leu Arg Gly
    450                 455                 460

Glu Leu Gln Ser Ser Asp Leu Phe His His Ser Lys Leu Asp Pro Asp
465                 470                 475                 480

Met Ala Phe Cys Ala Asn Ile Arg Gln Gln Asp Val Phe Met Phe Leu
                485                 490                 495

Thr Asn Arg His Thr Leu Gly His Leu Leu Ser Leu Asp Ser Tyr Arg
            500                 505                 510

Thr Thr His Leu His Asn Asp Leu Trp Glu Val Phe Ser Asn Pro Glu
        515                 520                 525

Asp Trp Lys Glu Lys Tyr Ile His Gln Asn Tyr Thr Lys Ala Leu Ala
    530                 535                 540

Gly Lys Leu Val Glu Thr Pro Cys Pro Asp Val Tyr Trp Phe Pro Ile
545                 550                 555                 560

Phe Thr Glu Val Ala Cys Asp Glu Leu Val Glu Glu Met Glu His Phe
                565                 570                 575

Gly Gln Trp Ser Leu Gly Asn Asn Lys Asp Asn Arg Ile Gln Gly Gly
            580                 585                 590

Tyr Glu Asn Val Pro Thr Ile Asp Ile His Met Asn Gln Ile Gly Phe
        595                 600                 605

Glu Arg Glu Trp His Lys Phe Leu Leu Glu Tyr Ile Ala Pro Met Thr
    610                 615                 620

Glu Lys Leu Tyr Pro Gly Tyr Tyr Thr Arg Ala Gln Phe Asp Leu Ala
625                 630                 635                 640

Phe Val Val Arg Tyr Lys Pro Asp Glu Gln Pro Ser Leu Met Pro His
                645                 650                 655

His Asp Ala Ser Thr Phe Thr Ile Asn Ile Ala Leu Asn Arg Val Gly
            660                 665                 670

Val Asp Tyr Glu Gly Gly Gly Cys Arg Phe Leu Arg Tyr Asn Cys Ser
        675                 680                 685

Ile Arg Ala Pro Arg Lys Gly Trp Thr Leu Met His Pro Gly Arg Leu
    690                 695                 700

Thr His Tyr His Glu Gly Leu Pro Thr Thr Arg Gly Thr Arg Tyr Ile
705                 710                 715                 720

Ala Val Ser Phe Val Asp Pro
                725

<210>  53
<211>  2184
<212>  DNA
<213>  Homo sapiens

<400> 53
atgcggcccc tgctgctact ggccctgctg ggctggctgc tgctggccga agcgaagggc 60
gacgccaagc cggaggacaa ccttttagtc ctcacggtgg ccactaagga gaccgaggga 120
ttccgtcgct tcaagcgctc agctcagttc ttcaactaca agatccaggc gcttggccta 180
ggggaggact ggaatgtgga gaaggggacg tcggcaggtg gagggcagaa ggtccggctg 240
ctgaagaaag ctctggagaa gcacgcagac aaggaggatc tggtcattct cttcacagac 300
agctatgacg tgctgtttgc atcgggcccc cgggagctcc tgaagaagtt ccggcagtcc 360
aggagccagg tggtcttctc tgctgaggag ctcatctacc cagaccgcag gctggagacc 420
aagtatccgg tggtgtccga tggcaagagg ttcctgggct ctggaggctt catcggttat 480
gcccccaacc tcagcaaact ggtggccgag tgggagggcc aggacagcga cagcgatcag 540
ctgttttaca ccaagatctt cttggacccg gagaagaggg agcagatcaa tatcaccctg 600
gaccaccgct gccgtatctt ccagaacctg gatggagcct tggatgaggt cgtgctcaag 660
tttgaaatgg gccatgtgag agcgaggaac ctggcctatg acaccctccc ggtcctgatc 720
catggcaacg ggccaaccaa gctgcagttg aactacctgg gcaactacat cccgcgcttc 780
tggaccttcg aaacaggctg caccgtgtgt gacgaaggct tgcgcagcct caagggcatt 840
ggggatgaag ctctgcccac ggtcctggtc ggcgtgttca tcgaacagcc cacgccgttt 900
gtgtccctgt tcttccagcg gctcctgcgg ctccactacc cccagaaaca catgcgactt 960
ttcatccaca accacgagca gcaccacaag gctcaggtgg aagagttcct ggcacagcat 1020
ggcagcgagt accagtctgt gaagctggtg ggccctgagg tgcggatggc gaatgcagat 1080
gccaggaaca tgggcgcaga cctgtgccgg caggaccgca gctgcaccta ctacttcagc 1140
gtggatgctg acgtggccct gaccgagccc aacagcctgc ggctgctgat ccaacagaac 1200
aagaacgtca ttgccccgct gatgacccgg catgggaggc tgtggtcgaa cttctggggg 1260
gctctcagtg cagatggcta ctatgcccgt tccgaggact acgtggacat tgtgcagggg 1320
cggcgtgttg gtgtctggaa tgtgccctat atttcaaaca tctacttgat caagggcagt 1380
gccctgcggg gtgagctgca gtcctcagat ctcttccacc acagcaagct ggaccccgac 1440
atggccttct gtgccaacat ccggcagcag gatgtgttca tgttcctgac caaccggcac 1500
acccttggcc atctgctctc cctagacagc taccgcacca cccacctgca caacgacctc 1560
tgggaggtgt tcagcaaccc cgaggactgg aaggagaagt acatccacca gaactacacc 1620
aaagccctgg ccgggaagct ggtggagacg ccctgcccgg atgtctattg gttccccatc 1680
ttcacggagg tggcctgtga tgagctggtg gaggagatgg agcactttgg ccagtggtct 1740
ctgggcaaca acaaggacaa ccgcatccag ggtggctacg agaacgtgcc gactattgac 1800
atccacatga accagatcgg ctttgagcgg gagtggcaca aattcctgct ggagtacatt 1860
gcgcccatga cggagaagct ctaccccggc tactacacca gggcccagtt tgacctggcc 1920

tttgtcgtcc gctacaagcc tgatgagcag ccctcactga tgccacacca tgatgcctcc 1980

accttcacca tcaacatcgc cctgaaccga gtcggggtgg attacgaggg cgggggctgt 2040

cggttcctgc gctacaactg ttccatccga gccccaagga agggctggac cctcatgcac 2100

cctggacgac tcacgcatta ccatgagggg ctccccacca ccaggggcac ccgctacatc 2160

gcagtctcct tcgtcgatcc ctaa 2184

<210> 54
<211> 758
<212> PRT
<213> Homo sapiens

<400> 54

<210>  55
<211>  2277
<212>  DNA
<213>  Homo sapiens

<400>  55
atggggggat gcacggtgaa gcctcagctg ctgctcctgg cgctcgtcct ccacccctgg      60

aatccctgtc tgggtgcgga ctcggagaag ccctcgagca tccccacaga taaattatta     120

gtcataactg tagcaacaaa agaaagtgat ggattccatc gatttatgca gtcagccaaa     180

tatttcaatt atactgtgaa ggtccttggt caaggagaag aatggagagg tggtgatgga     240

attaatagta ttggaggggg ccagaaagtg agattaatga aagaagtcat ggaacactat     300

gctgatcaag atgatctggt tgtcatgttt actgaatgct ttgatgtcat atttgctggt     360

ggtccagaag aagttctaaa aaaattccaa aaggcaaacc acaaagtggt ctttgcagca     420

gatggaattt tgtggccaga taaaagacta gcagacaagt atcctgttgt gcacattggg     480

aaacgctatc tgaattcagg aggatttatt ggctatgctc catatgtcaa ccgtatagtt     540

caacaatgga atctccagga taatgatgat gatcagctct tttacactaa agtttacatt     600

gatccactga aaagggaagc tattaacatc acattggatc acaaatgcaa aattttccag     660

accttaaatg gagctgtaga tgaagttgtt ttaaaatttg aaaatggcaa agccagagct     720

aagaatacat tttatgaaac attaccagtg gcaattaatg gaaatggacc caccaagatt     780

ctcctgaatt attttggaaa ctatgtaccc aattcatgga cacaggataa tggctgcact     840

ctttgtgaat tcgatacagt cgacttgtct gcagtagatg tccatccaaa cgtatcaata     900

ggtgttttta ttgagcaacc aacccctttt ctacctcggt ttctggacat attgttgaca     960

ctggattacc caaaagaagc acttaaactt tttattcata acaaagaagt ttatcatgaa    1020

aaggacatca aggtattttt tgataaagct aagcatgaaa tcaaaactat aaaaatagta    1080

ggaccagaag aaaatctaag tcaagcggaa gccagaaaca tgggaatgga cttttgccgt    1140

caggatgaaa agtgtgatta ttactttagt gtggatgcag atgttgtttt gacaaatcca    1200

aggactttaa aaattttgat tgaacaaaac agaaagatca ttgctcctct tgtaactcgt    1260

catggaaagc tgtggtccaa tttctgggga gcattgagtc ctgatggata ctatgcacga    1320

tctgaagatt atgtggatat tgttcaaggg aatagagtag gagtatggaa tgtcccatat 1380

atggctaatg tgtacttaat taaaggaaag acactccgat cagagatgaa tgaaaggaac 1440

tattttgttc gtgataaact ggatcctgat atggctcttt gccgaaatgc tagagaaatg 1500

actttacaaa gggaaaaaga ctcccctact ccggaaacat tccaaatgct cagcccccca 1560

aagggtgtat ttatgtacat ttctaataga catgaatttg gaaggctatt atccactgct 1620

aattacaata cttcccatta taacaatgac ctctggcaga tttttgaaaa tcctgtggac 1680

tggaaggaaa agtatataaa ccgtgattat tcaaagattt tcactgaaaa tatagttgaa 1740

cagccctgtc cagatgtctt ttggttcccc atattttctg aaaaagcctg tgatgaattg 1800

gtagaagaaa tggaacatta cggcaaatgg tctgggggaa aacatcatga tagccgtata 1860

tctggtggtt atgaaaatgt cccaactgat gatatccaca tgaagcaagt tgatctggag 1920

aatgtatggc ttcattttat ccgggagttc attgcaccag ttacactgaa ggtctttgca 1980

ggctattata cgaagggatt tgcactactg aattttgtag taaaatactc ccctgaacga 2040

cagcgttctc ttcgtcctca tcatgatgct tctacattta ccataaacat tgcacttaat 2100

aacgtgggag aagactttca gggaggtggt tgcaaatttc taaggtacaa ttgctctatt 2160

gagtcaccac gaaaaggctg gagcttcatg catcctggga gactcacaca tttgcatgaa 2220

ggacttcctg ttaaaaatgg aacaagatac attgcagtgt catttataga tccctaa 2277

<210> 56
<211> 738
<212> PRT
<213> Homo sapiens

<400> 56

<210>  57
<211>  2217
<212>  DNA
<213>  Homo sapiens

<400>  57
atgacctcct cggggcctgg accccggttc ctgctgctgc tgccgctgct gctgccccct      60

gcggcctcag cctccgaccg gccccggggc cgagacccgg tcaacccaga gaagctgctg     120

gtgatcactg tggccacagc tgaaaccgag gggtacctgc gtttcctgcg ctctgcggag     180

ttcttcaact acactgtgcg gaccctgggc ctgggagagg agtggcgagg gggtgatgtg     240

gctcgaacag ttggtggagg acagaaggtc cggtggttaa agaaggaaat ggagaaatac     300

gctgaccggg aggatatgat catcatgttt gtggatagct acgacgtgat tctggccggc     360

agccccacag agctgctgaa gaagttcgtc cagagtggca gccgcctgct cttctctgca     420

gagagcttct gctggcccga gtgggggctg gcggagcagt accctgaggt gggcacgggg     480

aagcgcttcc tcaattctgg tggattcatc ggttttgcca ccaccatcca ccaaatcgtg     540

cgccagtgga agtacaagga tgatgacgac gaccagctgt tctacacacg gctctacctg     600

gacccaggac tgagggagaa actcagcctt aatctggatc ataagtctcg gatctttcag     660

aacctcaacg gggctttaga tgaagtggtt ttaaagtttg atcggaaccg tgtgcgtatc     720

cggaacgtgg cctacgacac gctccccatt gtggtccatg gaaacggtcc cactaagctg     780

cagctcaact acctgggaaa ctacgtcccc aatggctgga ctcctgaggg aggctgtggc 840

ttctgcaacc aggaccggag gacactcccg ggggggcagc ctcccccccg ggtgtttctg 900

gccgtgtttg tggaacagcc tactccgttt ctgccccgct tcctgcagcg gctgctactc 960

ctggactatc cccccgacag ggtcaccctt ttcctgcaca acaacgaggt cttccatgaa 1020

ccccacatcg ctgactcctg gccgcagctc caggaccact tctcagctgt gaagctcgtg 1080

gggccggagg aggctctgag cccaggcgag gccagggaca tggccatgga cctgtgtcgg 1140

caggaccccg agtgtgagtt ctacttcagc ctggacgccg acgctgtcct caccaacctg 1200

cagaccctgc gtatcctcat tgaggagaac aggaaggtga tcgcccccat gctgtcccgc 1260

cacggcaagc tgtggtccaa cttctggggc gccctgagcc ccgatgagta ctacgcccgc 1320

tccgaggact acgtggagct ggtgcagcgg aagcgagtgg gtgtgtggaa tgtaccatac 1380

atctcccagg cctatgtgat ccggggtgat accctgcgga tggagctgcc ccagagggat 1440

gtgttctcgg gcagtgacac agacccggac atggccttct gtaagagctt tcgagacaag 1500

ggcatcttcc tccatctgag caatcagcat gaatttggcc ggctcctggc cacttccaga 1560

tacgacacgg agcacctgca ccccgacctc tggcagatct tcgacaaccc cgtcgactgg 1620

aaggagcagt acatccacga gaactacagc cgggccctgg aaggggaagg aatcgtggag 1680

cagccatgcc cggacgtgta ctggttccca ctgctgtcag aacaaatgtg tgatgagctg 1740

gtggcagaga tggagcacta cggccagtgg tcaggcggcc ggcatgagga ttcaaggctg 1800

gctggaggct acgagaatgt gcccaccgtg gacatccaca tgaagcaggt ggggtacgag 1860

gaccagtggc tgcagctgct gcggacgtat gtgggcccca tgaccgagag cctgtttccc 1920

ggttaccaca ccaaggcgcg ggcggtgatg aactttgtgg ttcgctaccg gccagacgag 1980

cagccgtctc tgcggccaca ccacgactca tccaccttca ccctcaacgt tgccctcaac 2040

cacaagggcc tggactatga gggaggtggc tgccgcttcc tgcgctacga ctgtgtgatc 2100

tcctccccga ggaagggctg ggcactcctg caccccggcc gcctcaccca ctaccacgag 2160

gggctgccaa cgacctgggg cacacgctac atcatggtgt cctttgtcga cccctga 2217

<210> 58
<211> 534
<212> PRT
<213> Homo sapiens

<400> 58

Met Ile Trp Tyr Ile Leu Ile Ile Gly Ile Leu Leu Pro Gln Ser Leu
1               5                   10                  15

Ala His Pro Gly Phe Phe Thr Ser Ile Gly Gln Met Thr Asp Leu Ile
            20                  25                  30

His Thr Glu Lys Asp Leu Val Thr Ser Leu Lys Asp Tyr Ile Lys Ala
        35                  40                  45

Glu Glu Asp Lys Leu Glu Gln Ile Lys Lys Trp Ala Glu Lys Leu Asp
    50                  55                  60

Arg Leu Thr Ser Thr Ala Thr Lys Asp Pro Glu Gly Phe Val Gly His
65                  70                  75                  80

Pro Val Asn Ala Phe Lys Leu Met Lys Arg Leu Asn Thr Glu Trp Ser
                85                  90                  95

Glu Leu Glu Asn Leu Val Leu Lys Asp Met Ser Asp Gly Phe Ile Ser
            100                 105                 110

Asn Leu Thr Ile Gln Arg Gln Tyr Phe Pro Asn Asp Glu Asp Gln Val
        115                 120                 125

Gly Ala Ala Lys Ala Leu Leu Arg Leu Gln Asp Thr Tyr Asn Leu Asp
    130                 135                 140

Thr Asp Thr Ile Ser Lys Gly Asn Leu Pro Gly Val Lys His Lys Ser
145                 150                 155                 160

Phe Leu Thr Ala Glu Asp Cys Phe Glu Leu Gly Lys Val Ala Tyr Thr
                165                 170                 175

Glu Ala Asp Tyr Tyr His Thr Glu Leu Trp Met Glu Gln Ala Leu Arg
            180                 185                 190

Gln Leu Asp Glu Gly Glu Ile Ser Thr Ile Asp Lys Val Ser Val Leu
        195                 200                 205

Asp Tyr Leu Ser Tyr Ala Val Tyr Gln Gln Gly Asp Leu Asp Lys Ala
    210                 215                 220

Leu Leu Leu Thr Lys Lys Leu Leu Glu Leu Asp Pro Glu His Gln Arg
225                 230                 235                 240

Ala Asn Gly Asn Leu Lys Tyr Phe Glu Tyr Ile Met Ala Lys Glu Lys
                245                 250                 255

Asp Val Asn Lys Ser Ala Ser Asp Asp Gln Ser Asp Gln Lys Thr Thr
            260                 265                 270

Pro Lys Lys Lys Gly Val Ala Val Asp Tyr Leu Pro Glu Arg Gln Lys
        275                 280                 285

Tyr Glu Met Leu Cys Arg Gly Glu Gly Ile Lys Met Thr Pro Arg Arg

290                 295                 300

Gln Lys Lys Leu Phe Cys Arg Tyr His Asp Gly Asn Arg Asn Pro Lys
305                 310                 315                 320

Phe Ile Leu Ala Pro Ala Lys Gln Glu Asp Glu Trp Asp Lys Pro Arg
                325                 330                 335

Ile Ile Arg Phe His Asp Ile Ile Ser Asp Ala Glu Ile Glu Ile Val
            340                 345                 350

Lys Asp Leu Ala Lys Pro Arg Leu Arg Arg Ala Thr Ile Ser Asn Pro
        355                 360                 365

Ile Thr Gly Asp Leu Glu Thr Val His Tyr Arg Ile Ser Lys Ser Ala
    370                 375                 380

Trp Leu Ser Gly Tyr Glu Asn Pro Val Val Ser Arg Ile Asn Met Arg
385                 390                 395                 400

Ile Gln Asp Leu Thr Gly Leu Asp Val Ser Thr Ala Glu Glu Leu Gln
                405                 410                 415

Val Ala Asn Tyr Gly Val Gly Gly Gln Tyr Glu Pro His Phe Asp Phe
            420                 425                 430

Ala Arg Lys Asp Glu Pro Asp Ala Phe Lys Glu Leu Gly Thr Gly Asn
        435                 440                 445

Arg Ile Ala Thr Trp Leu Phe Tyr Met Ser Asp Val Ser Ala Gly Gly
    450                 455                 460

Ala Thr Val Phe Pro Glu Val Gly Ala Ser Val Trp Pro Lys Lys Gly
465                 470                 475                 480

Thr Ala Val Phe Trp Tyr Asn Leu Phe Ala Ser Gly Glu Gly Asp Tyr
                485                 490                 495

Ser Thr Arg His Ala Ala Cys Pro Val Leu Val Gly Asn Lys Trp Val
            500                 505                 510

Ser Asn Lys Trp Leu His Glu Arg Gly Gln Glu Phe Arg Arg Pro Cys
        515                 520                 525

Thr Leu Ser Glu Leu Glu
    530

<210>  59
<211>  1605

<212>  DNA
<213>  Homo sapiens

<400>  59
atgatctggt atatattaat tataggaatt ctgcttcccc agtctttggc tcatccaggc      60

ttttttactt caattggtca gatgactgat ttgatccata ctgagaaaga tctggtgact     120

tctctgaaag attatattaa ggcagaagag gacaagttag aacaaataaa aaaatgggca     180

gagaagttag atcggctaac tagtacagcg acaaaagatc cagaaggatt tgttgggcat     240

ccagtaaatg cattcaaatt aatgaaacgt ctgaatactg agtggagtga gttggagaat     300

ctggtcctta aggatatgtc agatggcttt atctctaacc taaccattca gagacagtac     360

tttcctaatg atgaagatca ggttggggca gccaaagctc tgttacgtct ccaggatacc     420

tacaatttgg atacagatac catctcaaag ggtaatcttc caggagtgaa acacaaatct     480

tttctaacgg ctgaggactg ctttgagttg ggcaaagtgg cctatacaga agcagattat     540

taccatacgg aactgtggat ggaacaagcc ctaaggcaac tggatgaagg cgagatttct     600

accatagata aagtctctgt tctagattat ttgagctatg cggtatatca gcagggagac     660

ctggataagg cacttttgct cacaaagaag cttcttgaac tagatcctga acatcagaga     720

gctaatggta acttaaaata ttttgagtat ataatggcta aagaaaaaga tgtcaataag     780

tctgcttcag atgaccaatc tgatcagaaa actacaccaa agaaaaaagg ggttgctgtg     840

gattacctgc cagagagaca gaagtacgaa atgctgtgcc gtggggaggg tatcaaaatg     900

acccctcgga gacagaaaaa actcttttgc cgctaccatg atggaaaccg taatcctaaa     960

tttattctgg ctccagctaa acaggaggat gaatgggaca agcctcgtat tattcgcttc    1020

catgatatta tttctgatgc agaaattgaa atcgtcaaag acctagcaaa accaaggctg    1080

aggcgagcca ccatttcaaa cccaataaca ggagacttgg agacggtaca ttacagaatt    1140

agcaaaagtg cctggctctc tggctatgaa aatcctgtgg tgtctcgaat taatatgaga    1200

atacaagatc taacaggact agatgtttcc acagcagagg aattacaggt agcaaattat    1260

ggagttggag gacagtatga accccatttt gactttgcac ggaaagatga gccagatgct    1320

ttcaaagagc tggggacagg aaatagaatt gctacatggc tgttttatat gagtgatgtg    1380

tctgcaggag gagccactgt ttttcctgaa gttggagcta gtgtttggcc caaaaaagga    1440

actgctgttt tctggtataa tctgtttgcc agtggagaag gagattatag tacacggcat    1500

gcagcctgtc cagtgctagt tggcaacaaa tgggtatcca ataaatggct ccatgaacgt    1560

ggacaagaat ttcgaagacc ttgtacgttg tcagaattgg aatga                    1605


<210>  60
<211>  533
<212>  PRT
<213>  Homo sapiens

<400>  60

Met Lys Leu Trp Val Ser Ala Leu Leu Met Ala Trp Phe Gly Val Leu
1               5                   10                  15

Ser Cys Val Gln Ala Glu Phe Phe Thr Ser Ile Gly His Met Thr Asp
            20                  25                  30

Leu Ile Tyr Ala Glu Lys Glu Leu Val Gln Ser Leu Lys Glu Tyr Ile
        35                  40                  45

Leu Val Glu Glu Ala Lys Leu Ser Lys Ile Lys Ser Trp Ala Asn Lys
    50                  55                  60

Met Glu Ala Leu Thr Ser Lys Ser Ala Ala Asp Ala Glu Gly Tyr Leu
65                  70                  75                  80

Ala His Pro Val Asn Ala Tyr Lys Leu Val Lys Arg Leu Asn Thr Asp
                85                  90                  95

Trp Pro Ala Leu Glu Asp Leu Val Leu Gln Asp Ser Ala Ala Gly Phe
            100                 105                 110

Ile Ala Asn Leu Ser Val Gln Arg Gln Phe Phe Pro Thr Asp Glu Asp
        115                 120                 125

Glu Ile Gly Ala Ala Lys Ala Leu Met Arg Leu Gln Asp Thr Tyr Arg
    130                 135                 140

Leu Asp Pro Gly Thr Ile Ser Arg Gly Glu Leu Pro Gly Thr Lys Tyr
145                 150                 155                 160

Gln Ala Met Leu Ser Val Asp Asp Cys Phe Gly Met Gly Arg Ser Ala
                165                 170                 175

Tyr Asn Glu Gly Asp Tyr Tyr His Thr Val Leu Trp Met Glu Gln Val
            180                 185                 190

Leu Lys Gln Leu Asp Ala Gly Glu Glu Ala Thr Thr Thr Lys Ser Gln
        195                 200                 205

Val Leu Asp Tyr Leu Ser Tyr Ala Val Phe Gln Leu Gly Asp Leu His
    210                 215                 220

Arg Ala Leu Glu Leu Thr Arg Arg Leu Leu Ser Leu Asp Pro Ser His
225                 230                 235                 240

Glu Arg Ala Gly Gly Asn Leu Arg Tyr Phe Glu Gln Leu Leu Glu Glu
                245                 250                 255

Glu Arg Glu Lys Thr Leu Thr Asn Gln Thr Glu Ala Glu Leu Ala Thr
            260                 265                 270

Pro Glu Gly Ile Tyr Glu Arg Pro Val Asp Tyr Leu Pro Glu Arg Asp
        275                 280                 285

Val Tyr Glu Ser Leu Cys Arg Gly Glu Gly Val Lys Leu Thr Pro Arg
    290                 295                 300

Arg Gln Lys Arg Leu Phe Cys Arg Tyr His His Gly Asn Arg Ala Pro
305                 310                 315                 320

Gln Leu Leu Ile Ala Pro Phe Lys Glu Glu Asp Glu Trp Asp Ser Pro
                325                 330                 335

His Ile Val Arg Tyr Tyr Asp Val Met Ser Asp Glu Glu Ile Glu Arg
            340                 345                 350

Ile Lys Glu Ile Ala Lys Pro Lys Leu Ala Arg Ala Thr Val Arg Asp
        355                 360                 365

Pro Lys Thr Gly Val Leu Thr Val Ala Ser Tyr Arg Val Ser Lys Ser
    370                 375                 380

Ser Trp Leu Glu Glu Asp Asp Asp Pro Val Val Ala Arg Val Asn Arg
385                 390                 395                 400

Arg Met Gln His Ile Thr Gly Leu Thr Val Lys Thr Ala Glu Leu Leu
                405                 410                 415

Gln Val Ala Asn Tyr Gly Val Gly Gly Gln Tyr Glu Pro His Phe Asp
            420                 425                 430

Phe Ser Arg Arg Pro Phe Asp Ser Gly Leu Lys Thr Glu Gly Asn Arg
        435                 440                 445

Leu Ala Thr Phe Leu Asn Tyr Met Ser Asp Val Glu Ala Gly Gly Ala
    450                 455                 460

Thr Val Phe Pro Asp Leu Gly Ala Ala Ile Trp Pro Lys Lys Gly Thr
465                 470                 475                 480

Ala Val Phe Trp Tyr Asn Leu Leu Arg Ser Gly Glu Gly Asp Tyr Arg
                485                 490                 495

Thr Arg His Ala Ala Cys Pro Val Leu Val Gly Cys Lys Trp Val Ser
            500                 505                 510

Asn Lys Trp Phe His Glu Arg Gly Gln Glu Phe Leu Arg Pro Cys Gly

515                 520                 525

Ser Thr Glu Val Asp
    530

<210>  61
<211>  1602
<212>  DNA
<213>  Homo sapiens

<400>  61
atgaaactct gggtgtctgc attgctgatg gcctggtttg gtgtcctgag ctgtgtgcag      60

gccgaattct tcacctctat tgggcacatg actgacctga tttatgcaga gaaagagctg     120

gtgcagtctc tgaaagagta catccttgtg gaggaagcca agctttccaa gattaagagc     180

tgggccaaca aaatggaagc cttgactagc aagtcagctg ctgatgctga gggctacctg     240

gctcaccctg tgaatgccta caaactggtg aagcggctaa acacagactg gcctgcgctg     300

gaggaccttg tcctgcagga ctcagctgca ggttttatcg ccaacctctc tgtgcagcgg     360

cagttcttcc ccactgatga ggacgagata ggagctgcca aagccctgat gagacttcag     420

gacacataca ggctggaccc aggcacaatt tccagagggg aacttccagg aaccaagtac     480

caggcaatgc tgagtgtgga tgactgcttt gggatgggcc gctcggccta caatgaaggg     540

gactattatc atacggtgtt gtggatggag caggtgctaa agcagcttga tgccggggag     600

gaggccacca caaccaagtc acaggtgctg gactacctca gctatgctgt cttccagttg     660

ggtgatctgc accgtgccct ggagctcacc cgccgcctgc tctcccttga cccaagccac     720

gaacgagctg gagggaatct gcggtacttt gagcagttat tggaggaaga gagagaaaaa     780

acgttaacaa atcagacaga agctgagcta gcaaccccag aaggcatcta tgagaggcct     840

gtggactacc tgcctgagag ggatgtttac gagagcctct gtcgtgggga gggtgtcaaa     900

ctgacacccc gtagacagaa gaggcttttc tgtaggtacc accatggcaa cagggcccca     960

cagctgctca ttgccccctt caaagaggag gacgagtggg acagcccgca catcgtcagg    1020

tactacgatg tcatgtctga tgaggaaatc gagaggatca aggagatcgc aaaacctaaa    1080

cttgcacgag ccaccgttcg tgatcccaag acaggagtcc tcactgtcgc cagctaccgg    1140

gtttccaaaa gctcctggct agaggaagat gatgaccctg ttgtggcccg agtaaatcgt    1200

cggatgcagc atatcacagg gttaacagta aagactgcag aattgttaca ggttgcaaat    1260

tatggagtgg gaggacagta tgaaccgcac ttcgacttct ctaggcgacc ttttgacagc    1320

ggcctcaaaa cagaggggaa taggttagcg acgtttctta actacatgag tgatgtagaa    1380

gctggtggtg ccaccgtctt ccctgatctg ggggctgcaa tttggcctaa gaagggtaca    1440

gctgtgttct ggtacaacct cttgcggagc ggggaaggtg actaccgaac aagacatgct    1500

gcctgccctg tgcttgtggg ctgcaagtgg gtctccaata agtggttcca tgaacgagga    1560

caggagttct tgagaccttg tggatcaaca gaagttgact ga                    1602

<210>  62
<211>  544
<212>  PRT
<213>  Homo sapiens

<400>  62

Met Gly Pro Gly Ala Arg Leu Ala Ala Leu Leu Ala Val Leu Ala Leu
1               5                   10                  15

Gly Thr Gly Asp Pro Glu Arg Ala Ala Ala Arg Gly Asp Thr Phe Ser
            20                  25                  30

Ala Leu Thr Ser Val Ala Arg Ala Leu Ala Pro Glu Arg Arg Leu Leu
        35                  40                  45

Gly Leu Leu Arg Arg Tyr Leu Arg Gly Glu Glu Ala Arg Leu Arg Asp
    50                  55                  60

Leu Thr Arg Phe Tyr Asp Lys Val Leu Ser Leu His Glu Asp Ser Thr
65                  70                  75                  80

Thr Pro Val Ala Asn Pro Leu Leu Ala Phe Thr Leu Ile Lys Arg Leu
                85                  90                  95

Gln Ser Asp Trp Arg Asn Val Val His Ser Leu Glu Ala Ser Glu Asn
            100                 105                 110

Ile Arg Ala Leu Lys Asp Gly Tyr Glu Lys Val Glu Gln Asp Leu Pro
        115                 120                 125

Ala Phe Glu Asp Leu Glu Gly Ala Ala Arg Ala Leu Met Arg Leu Gln
    130                 135                 140

Asp Val Tyr Met Leu Asn Val Lys Gly Leu Ala Arg Gly Val Phe Gln
145                 150                 155                 160

Arg Val Thr Gly Ser Ala Ile Thr Asp Leu Tyr Ser Pro Lys Arg Leu
                165                 170                 175

Phe Ser Leu Thr Gly Asp Asp Cys Phe Gln Val Gly Lys Val Ala Tyr
            180                 185                 190

Asp Met Gly Asp Tyr Tyr His Ala Ile Pro Trp Leu Glu Glu Ala Val
        195                 200                 205

Ser Leu Phe Arg Gly Ser Tyr Gly Glu Trp Lys Thr Glu Asp Glu Ala
    210                 215                 220

Ser Leu Glu Asp Ala Leu Asp His Leu Ala Phe Ala Tyr Phe Arg Ala
225                 230                 235                 240

Gly Asn Val Ser Cys Ala Leu Ser Leu Ser Arg Glu Phe Leu Leu Tyr
                245                 250                 255

Ser Pro Asp Asn Lys Arg Met Ala Arg Asn Val Leu Lys Tyr Glu Arg
            260                 265                 270

Leu Leu Ala Glu Ser Pro Asn His Val Val Ala Glu Ala Val Ile Gln
        275                 280                 285

Arg Pro Asn Ile Pro His Leu Gln Thr Arg Asp Thr Tyr Glu Gly Leu
    290                 295                 300

Cys Gln Thr Leu Gly Ser Gln Pro Thr Leu Tyr Gln Ile Pro Ser Leu
305                 310                 315                 320

Tyr Cys Ser Tyr Glu Thr Asn Ser Asn Ala Tyr Leu Leu Leu Gln Pro
                325                 330                 335

Ile Arg Lys Glu Val Ile His Leu Glu Pro Tyr Ile Ala Leu Tyr His
            340                 345                 350

Asp Phe Val Ser Asp Ser Glu Ala Gln Lys Ile Arg Glu Leu Ala Glu
        355                 360                 365

Pro Trp Leu Gln Arg Ser Val Val Ala Ser Gly Glu Lys Gln Leu Gln
    370                 375                 380

Val Glu Tyr Arg Ile Ser Lys Ser Ala Trp Leu Lys Asp Thr Val Asp
385                 390                 395                 400

Pro Lys Leu Val Thr Leu Asn His Arg Ile Ala Ala Leu Thr Gly Leu
                405                 410                 415

Asp Val Arg Pro Pro Tyr Ala Glu Tyr Leu Gln Val Val Asn Tyr Gly
            420                 425                 430

Ile Gly Gly His Tyr Glu Pro His Phe Asp His Ala Thr Ser Pro Ser
        435                 440                 445

Ser Pro Leu Tyr Arg Met Lys Ser Gly Asn Arg Val Ala Thr Phe Met
    450                 455                 460

Ile Tyr Leu Ser Ser Val Glu Ala Gly Gly Ala Thr Ala Phe Ile Tyr
465                 470                 475                 480

Ala Asn Leu Ser Val Pro Val Val Arg Asn Ala Ala Leu Phe Trp Trp
                485                 490                 495

Asn Leu His Arg Ser Gly Glu Gly Asp Ser Asp Thr Leu His Ala Gly
            500                 505                 510

Cys Pro Val Leu Val Gly Asp Lys Trp Val Ala Asn Lys Trp Ile His
        515                 520                 525

Glu Tyr Gly Gln Glu Phe Arg Arg Pro Cys Ser Ser Ser Pro Glu Asp
    530                 535                 540

<210>  63
<211>  1635
<212>  DNA
<213>  Homo sapiens

<400>  63
atgggtcctg gggcgcggct ggcggcgctg ctggcggtgc tggcgctcgg gacaggagac      60

ccagaaaggg ctgcggctcg gggcgacacg ttctcggcgc tgaccagcgt ggcgcgcgcc     120

ctggcgcccg agcgccggct gctggggctg ctgaggcggt acctgcgcgg ggaggaggcg     180

cggctgcggg acctgactag attctacgac aaggtacttt ctttgcatga ggattcaaca     240

acccctgtgg ctaaccctct gcttgcattt actctcatca aacgcctgca gtctgactgg     300

aggaatgtgg tacatagtct ggaggccagt gagaacatcc gagctctgaa ggatggctat     360

gagaaggtgg agcaagacct tccagccttt gaggaccttg agggagcagc aagggccctg     420

atgcggctgc aggacgtgta catgctcaat gtgaaaggcc tggcccgagg tgtctttcag     480

agagtcactg gctctgccat cactgacctg tacagcccca aacggctctt ttctctcaca     540

ggggatgact gcttccaagt tggcaaggtg gcctatgaca tgggggatta ttaccatgcc     600

attccatggc tggaggaggc tgtcagtctc ttccgaggat cttacggaga gtggaagaca     660

gaggatgagg caagtctaga agatgccttg gatcacttgg cctttgctta tttccgggca     720

ggaaatgttt cgtgtgccct cagcctctct cgggagtttc ttctctacag cccagataat     780

aagaggatgg ccaggaatgt cttgaaatat gaaaggctct tggcagagag ccccaaccac     840

gtggtagctg aggctgtcat ccagaggccc aatatacccc acctgcagac cagagacacc     900

tacgaggggc tatgtcagac cctgggttcc cagcccactc tctaccagat ccctagcctc     960

tactgttcct atgagaccaa ttccaacgcc tacctgctgc tccagcccat ccggaaggag    1020

gtcatccacc tggagcccta cattgctctc taccatgact tcgtcagtga ctcagaggct    1080

cagaaaatta gagaacttgc agaaccatgg ctacagaggt cagtggtggc atcaggggag    1140

aagcagttac aagtggagta ccgcatcagc aaaagtgcct ggctgaagga cactgttgac    1200

ccaaaactgg tgaccctcaa ccaccgcatt gctgccctca caggccttga tgtccggcct    1260

ccctatgcag agtatctgca ggtggtgaac tatggcatcg gaggacacta tgagcctcac    1320

tttgaccatg ctacgtcacc aagcagcccc ctctacagaa tgaagtcagg aaaccgagtt 1380

gcaacattta tgatctatct gagctcggtg gaagctggag gagccacagc cttcatctat 1440

gccaacctca gcgtgcctgt ggttaggaat gcagcactgt tttggtggaa cctgcacagg 1500

agtggtgaag gggacagtga cacacttcat gctggctgtc ctgtcctggt gggagataag 1560

tgggtggcca acaagtggat acatgagtat ggacaggaat tccgcagacc ctgcagctcc 1620

agccctgaag actga 1635

<210> 64
<211> 508
<212> PRT
<213> Homo sapiens

<400> 64

<210>  65
<211>  1527
<212>  DNA
<213>  Homo sapiens

<400>  65
atgctgcgcc gcgctctgct gtgcctggcc gtggccgccc tggtgcgcgc cgacgccccc      60

gaggaggagg accacgtcct ggtgctgcgg aaaagcaact tcgcggaggc gctggcggcc     120

cacaagtacc tgctggtgga gttctatgcc ccttggtgtg gccactgcaa ggctctggcc     180

cctgagtatg ccaaagccgc tgggaagctg aaggcagaag gttccgagat caggttggcc     240

aaggtggacg ccacggagga gtctgacctg gcccagcagt acggcgtgcg cggctatccc     300

accatcaagt tcttcaggaa tggagacacg gcttccccca aggaatatac agctggcaga     360

gaggctgatg acatcgtgaa ctggctgaag aagcgcacgg gcccggctgc caccaccctg     420

cctgacggcg cagctgcaga gtccttggtg gagtccagcg aggtggctgt catcggcttc     480

ttcaaggacg tggagtcgga ctctgccaag cagtttttgc aggcagcaga ggccatcgat     540

gacataccat ttgggatcac ttccaacagt gacgtgttct ccaaatacca gctcgacaaa     600

gatggggttg tcctctttaa gaagtttgat gaaggccgga acaactttga aggggaggtc     660

accaaggaga acctgctgga ctttatcaaa cacaaccagc tgccccttgt catcgagttc     720

accgagcaga cagccccgaa gatttttgga ggtgaaatca agactcacat cctgctgttc     780

ttgcccaaga gtgtgtctga ctatgacggc aaactgagca acttcaaaac agcagccgag     840

agcttcaagg gcaagatcct gttcatcttc atcgacagcg accacaccga caaccagcgc     900

atcctcgagt tctttggcct gaagaaggaa gagtgcccgg ccgtgcgcct catcaccctg     960

gaggaggaga tgaccaagta caagcccgaa tcggaggagc tgacggcaga gaggatcaca    1020

gagttctgcc accgcttcct ggagggcaaa atcaagcccc acctgatgag ccaggagctg    1080

ccggaggact gggacaagca gcctgtcaag gtgcttgttg ggaagaactt tgaagacgtg    1140

gcttttgatg agaaaaaaaa cgtctttgtg gagttctatg ccccatggtg tggtcactgc    1200

aaacagttgg ctcccatttg ggataaactg ggagagacgt acaaggacca tgagaacatc 1260

gtcatcgcca agatggactc gactgccaac gaggtggagg ccgtcaaagt gcacagcttc 1320

cccacactca agttctttcc tgccagtgcc gacaggacgg tcattgatta caacggggaa 1380

cgcacgctgg atggttttaa gaaattcctg gagagcggtg gccaggatgg ggcaggggat 1440

gatgacgatc tcgaggacct ggaagaagca gaggagccag acatggagga agacgatgat 1500

cagaaagctg tgaaagatga actgtaa 1527

<210> 66
<211> 1464
<212> PRT
<213> Homo sapiens

<400> 66

<210> 67
<211> 4395
<212> DNA
<213> Homo sapiens

<400> 67
atgttcagct ttgtggacct ccggctcctg ctcctcttag cggccaccgc cctcctgacg 60

cacggccaag aggaaggcca agtcgagggc caagacgaag acatcccacc aatcacctgc 120

gtacagaacg gcctcaggta ccatgaccga gacgtgtgga aacccgagcc ctgccggatc 180

tgcgtctgcg acaacggcaa ggtgttgtgc gatgacgtga tctgtgacga gaccaagaac 240

tgccccggcg ccgaagtccc cgagggcgag tgctgtcccg tctgccccga cggctcagag 300

tcacccaccg accaagaaac caccggcgtc gagggaccca agggagacac tggcccccga 360

ggcccaaggg gacccgcagg cccccctggc cgagatggca tccctggaca gcctggactt 420

cccggacccc ccggaccccc cggacctccc ggaccccctg gcctcggagg aaactttgct 480

ccccagctgt cttatggcta tgatgagaaa tcaaccggag gaatttccgt gcctggcccc 540

atgggtccct ctggtcctcg tggtctccct ggcccccctg gtgcacctgg tccccaaggc 600

ttccaaggtc cccctggtga gcctggcgag cctggagctt caggtcccat gggtccccga 660

ggtcccccag gtccccctgg aaagaatgga gatgatgggg aagctggaaa acctggtcgt 720

cctggtgagc gtgggcctcc tgggcctcag ggtgctcgag gattgcccgg aacagctggc 780

ctccctggaa tgaagggaca cagaggtttc agtggtttgg atggtgccaa gggagatgct 840

ggtcctgctg gtcctaaggg tgagcctggc agccctggtg aaaatggagc tcctggtcag 900

atgggccccc gtggcctgcc tggtgagaga ggtcgccctg gagcccctgg ccctgctggt 960

gctcgtggaa atgatggtgc tactggtgct gccgggcccc ctggtcccac cggccccgct 1020

ggtcctcctg gcttccctgg tgctgttggt gctaagggtg aagctggtcc ccaagggccc 1080

cgaggctctg aaggtcccca gggtgtgcgt ggtgagcctg gcccccctgg ccctgctggt 1140

gctgctggcc ctgctggaaa ccctggtgct gatggacagc ctggtgctaa aggtgccaat 1200

ggtgctcctg gtattgctgg tgctcctggc ttccctggtg cccgaggccc ctctggaccc 1260

cagggccccg gcggccctcc tggtcccaag ggtaacagcg gtgaacctgg tgctcctggc 1320

agcaaaggag acactggtgc taagggagag cctggccctg ttggtgttca aggaccccct 1380

ggccctgctg gagaggaagg aaagcgagga gctcgaggtg aacccggacc cactggcctg 1440

cccggacccc ctggcgagcg tggtggacct ggtagccgtg gtttccctgg cgcagatggt 1500

gttgctggtc ccaagggtcc cgctggtgaa cgtggttctc ctggccctgc tggccccaaa 1560

ggatctcctg gtgaagctgg tcgtcccggt gaagctggtc tgcctggtgc caagggtctg 1620

actggaagcc ctggcagccc tggtcctgat ggcaaaactg gcccccctgg tcccgccggt 1680
caagatggtc gccccggacc cccaggccca cctggtgccc gtggtcaggc tggtgtgatg 1740
ggattccctg gacctaaagg tgctgctgga gagcccggca aggctggaga gcgaggtgtt 1800
cccggacccc ctggcgctgt cggtcctgct ggcaaagatg gagaggctgg agctcaggga 1860
ccccctggcc ctgctggtcc cgctggcgag agaggtgaac aaggccctgc tggctccccc 1920
ggattccagg gtctccctgg tcctgctggt cctccaggtg aagcaggcaa acctggtgaa 1980
cagggtgttc ctggagacct tggcgcccct ggcccctctg gagcaagagg cgagagaggt 2040
ttccctggcg agcgtggtgt gcaaggtccc cctggtcctg ctggtccccg aggggccaac 2100
ggtgctcccg gcaacgatgg tgctaagggt gatgctggtg cccctggagc tcccggtagc 2160
cagggcgccc ctggccttca gggaatgcct ggtgaacgtg gtgcagctgg tcttccaggg 2220
cctaagggtg acagaggtga tgctggtccc aaaggtgctg atggctctcc tggcaaagat 2280
ggcgtccgtg gtctgaccgg ccccattggt cctcctggcc ctgctggtgc ccctggtgac 2340
aagggtgaaa gtggtcccag cggccctgct ggtcccactg gagctcgtgg tgcccccgga 2400
gaccgtggtg agcctggtcc ccccggccct gctggctttg ctggcccccc tggtgctgac 2460
ggccaacctg gtgctaaagg cgaacctggt gatgctggtg ctaaaggcga tgctggtccc 2520
cctggccctg ccggacccgc tggaccccct ggccccattg gtaatgttgg tgctcctgga 2580
gccaaaggtg ctcgcggcag cgctggtccc cctggtgcta ctggtttccc tggtgctgct 2640
ggccgagtcg gtcctcctgg cccctctgga aatgctggac cccctggccc tcctggtcct 2700
gctggcaaag aaggcggcaa aggtccccgt ggtgagactg gccctgctgg acgtcctggt 2760
gaagttggtc cccctggtcc ccctggccct gctggcgaga aaggatcccc tggtgctgat 2820
ggtcctgctg gtgctcctgg tactcccggg cctcaaggta ttgctggaca gcgtggtgtg 2880
gtcggcctgc ctggtcagag aggagagaga ggcttccctg gtcttcctgg cccctctggt 2940
gaacctggca aacaaggtcc ctctggagca agtggtgaac gtggtccccc tggtcccatg 3000
ggcccccctg gattggctgg accccctggt gaatctggac gtgagggggc tcctggtgcc 3060
gaaggttccc ctggacgaga cggttctcct ggcgccaagg gtgaccgtgg tgagaccggc 3120
cccgctggac cccctggtgc tcctggtgct cctggtgccc ctggccccgt tggccctgct 3180
ggcaagagtg gtgatcgtgg tgagactggt cctgctggtc ccgccggtcc tgtcggccct 3240
gttggcgccc gtggccccgc cggaccccaa ggcccccgtg gtgacaaggg tgagacaggc 3300
gaacagggcg acagaggcat aaagggtcac cgtggcttct ctggcctcca gggtccccct 3360
ggccctcctg gctctcctgg tgaacaaggt ccctctggag cctctggtcc tgctggtccc 3420
cgaggtcccc ctggctctgc tggtgctcct ggcaaagatg gactcaacgg tctccctggc 3480
cccattgggc cccctggtcc tcgcggtcgc actggtgatg ctggtcctgt tggtcccccc 3540

ggccctcctg gacctcctgg tccccctggt cctcccagcg ctggtttcga cttcagcttc 3600

ctgccccagc cacctcaaga gaaggctcac gatggtggcc gctactaccg ggctgatgat 3660

gccaatgtgg ttcgtgaccg tgacctcgag gtggacacca ccctcaagag cctgagccag 3720

cagatcgaga acatccggag cccagagggc agccgcaaga accccgcccg cacctgccgt 3780

gacctcaaga tgtgccactc tgactggaag agtggagagt actggattga ccccaaccaa 3840

ggctgcaacc tggatgccat caaagtcttc tgcaacatgg agactggtga gacctgcgtg 3900

taccccactc agcccagtgt ggcccagaag aactggtaca tcagcaagaa ccccaaggac 3960

aagaggcatg tctggttcgg cgagagcatg accgatggat tccagttcga gtatggcggc 4020

cagggctccg accctgccga tgtggccatc cagctgacct tcctgcgcct gatgtccacc 4080

gaggcctccc agaacatcac ctaccactgc aagaacagcg tggcctacat ggaccagcag 4140

actggcaacc tcaagaaggc cctgctcctc cagggctcca acgagatcga gatccgcgcc 4200

gagggcaaca gccgcttcac ctacagcgtc actgtcgatg gctgcacgag tcacaccgga 4260

gcctggggca agacagtgat tgaatacaaa accaccaaga cctcccgcct gcccatcatc 4320

gatgtggccc ccttggacgt tggtgcccca gaccaggaat tcggcttcga cgttggccct 4380

gtctgcttcc tgtaa 4395

<210> 68
<211> 1366
<212> PRT
<213> Homo sapiens

<400> 68

<210> 69
<211> 4101
<212> DNA
<213> Homo sapiens

<400> 69
atgctcagct ttgtggatac gcggactttg ttgctgcttg cagtaacctt atgcctagca 60

acatgccaat ctttacaaga ggaaaccgta agaaagggcc cagccggaga tagaggacca 120

cgtggagaaa ggggtccacc aggcccccca ggcagagatg gtgaagatgg tcccacaggc 180

cctcctggtc cacctggtcc tcctggcccc cctggtctcg gtgggaactt tgctgctcag 240

tatgacggaa aaggagttgg acttggccct ggaccaatgg gcttaatggg acctagaggc 300

ccacctggtg cagctggagc cccaggccct caaggtttcc aaggacctgc tggtgagcct 360

ggtgaacctg gtcaaactgg tcctgcaggt gctcgtggtc cagctggccc tcctggcaag 420

gctggtgaag atggtcaccc tggaaaaccc ggacgacctg gtgagagagg agttgttgga 480

ccacagggtg ctcgtggttt ccctggaact cctggacttc ctggcttcaa aggcattagg 540

ggacacaatg gtctggatgg attgaaggga cagcccggtg ctcctggtgt gaagggtgaa 600

cctggtgccc ctggtgaaaa tggaactcca ggtcaaacag gagcccgtgg gcttcctggt 660

gagagaggac gtgttggtgc ccctggccca gctggtgccc gtggcagtga tggaagtgtg 720

ggtcccgtgg gtcctgctgg tcccattggg tctgctggcc ctccaggctt cccaggtgcc 780

cctggcccca agggtgaaat tggagctgtt ggtaacgctg gtcctgctgg tcccgccggt 840

ccccgtggtg aagtgggtct tccaggcctc tccggccccg ttggacctcc tggtaatcct 900

ggagcaaacg gccttactgg tgccaagggt gctgctggcc ttcccggcgt tgctggggct 960

cccggcctcc ctggaccccg cggtattcct ggccctgttg gtgctgccgg tgctactggt 1020

gccagaggac ttgttggtga gcctggtcca gctggctcca aaggagagag cggtaacaag 1080

ggtgagcccg gctctgctgg gccccaaggt cctcctggtc ccagtggtga agaaggaaag 1140

agaggcccta atggggaagc tggatctgcc ggccctccag gacctcctgg gctgagaggt 1200

agtcctggtt ctcgtggtct tcctggagct gatggcagag ctggcgtcat gggccctcct 1260

ggtagtcgtg gtgcaagtgg ccctgctgga gtccgaggac ctaatggaga tgctggtcgc 1320

cctggggagc ctggtctcat gggacccaga ggtcttcctg gttcccctgg aaatatcggc 1380

cccgctggaa aagaaggtcc tgtcggcctc cctggcatcg acggcaggcc tggcccaatt 1440

ggcccagctg gagcaagagg agagcctggc aacattggat tccctggacc caaaggcccc 1500

actggtgatc ctggcaaaaa cggtgataaa ggtcatgctg gtcttgctgg tgctcggggt 1560

gctccaggtc ctgatggaaa caatggtgct cagggacctc ctggaccaca gggtgttcaa 1620

ggtggaaaag gtgaacaggg tcccgctggt cctccaggct tccagggtct gcctggcccc 1680

tcaggtcccg ctggtgaagt tggcaaacca ggagaaaggg gtctccatgg tgagtttggt 1740

ctccctggtc ctgctggtcc aagaggggaa cgcggtcccc caggtgagag tggtgctgcc 1800

ggtcctactg gtcctattgg aagccgaggt ccttctggac ccccagggcc tgatggaaac 1860

aagggtgaac ctggtgttgt tggtgctgtg ggcactgctg gtccatctgg tcctagtgga 1920

ctcccaggag agaggggtgc tgctggcata cctggaggca agggagaaaa gggtgaacct 1980
ggtctcagag gtgaaattgg taaccctggc agagatggtg ctcgtggtgc tcctggtgct 2040
gtaggtgccc ctggtcctgc tggagccaca ggtgaccggg gcgaagctgg ggctgctggt 2100
cctgctggtc ctgctggtcc tcggggaagc cctggtgaac gtggtgaggt cggtcctgct 2160
ggccccaatg gatttgctgg tcctgctggt gctgctggtc aacctggtgc taaaggagaa 2220
agaggagcca aagggcctaa gggtgaaaac ggtgttgttg gtcccacagg ccccgttgga 2280
gctgctggcc cagctggtcc aaatggtccc cccggtcctg ctggaagtcg tggtgatgga 2340
ggcccccctg gtatgactgg tttccctggt gctgctggac ggactggtcc cccaggaccc 2400
tctggtattt ctggccctcc tggtccccct ggtcctgctg ggaaagaagg gcttcgtggt 2460
cctcgtggtg accaaggtcc agttggccga actggagaag taggtgcagt tggtccccct 2520
ggcttcgctg gtgagaaggg tccctctgga gaggctggta ctgctggacc tcctggcact 2580
ccaggtcctc agggtcttct tggtgctcct ggtattctgg gtctccctgg ctcgagaggt 2640
gaacgtggtc taccaggtgt tgctggtgct gtgggtgaac ctggtcctct tggcattgcc 2700
ggccctcctg gggcccgtgg tcctcctggt gctgtgggta gtcctggagt caacggtgct 2760
cctggtgaag ctggtcgtga tggcaaccct gggaacgatg gtcccccagg tcgcgatggt 2820
caacccggac acaagggaga gcgcggttac cctggcaata ttggtcccgt tggtgctgca 2880
ggtgcacctg gtcctcatgg ccccgtgggt cctgctggca aacatggaaa ccgtggtgaa 2940
actggtcctt ctggtcctgt tggtcctgct ggtgctgttg gcccaagagg tcctagtggc 3000
ccacaaggca ttcgtggcga taagggagag cccggtgaaa aggggcccag aggtcttcct 3060
ggcttaaagg gacacaatgg attgcaaggt ctgcctggta tcgctggtca ccatggtgat 3120
caaggtgctc ctggctccgt gggtcctgct ggtcctaggg gccctgctgg tccttctggc 3180
cctgctggaa aagatggtcg cactggacat cctggtacag ttggacctgc tggcattcga 3240
ggccctcagg gtcaccaagg ccctgctggc ccccctggtc cccctggccc tcctggacct 3300
ccaggtgtaa gcggtggtgg ttatgacttt ggttacgatg gagacttcta cagggctgac 3360
cagcctcgct cagcaccttc tctcagaccc aaggactatg aagttgatgc tactctgaag 3420
tctctcaaca accagattga gacccttctt actcctgaag gctctagaaa gaacccagct 3480
cgcacatgcc gtgacttgag actcagccac ccagagtgga gcagtggtta ctactggatt 3540
gaccctaacc aaggatgcac tatggatgct atcaaagtat actgtgattt ctctactggc 3600
gaaacctgta tccgggccca acctgaaaac atcccagcca agaactggta taggagctcc 3660
aaggacaaga aacacgtctg gctaggagaa actatcaatg ctggcagcca gtttgaatat 3720
aatgtagaag gagtgacttc caaggaaatg gctacccaac ttgccttcat gcgcctgctg 3780
gccaactatg cctctcagaa catcacctac cactgcaaga acagcattgc atacatggat 3840

gaggagactg gcaacctgaa aaaggctgtc attctacagg gctctaatga tgttgaactt    3900

gttgctgagg gcaacagcag gttcacttac actgttcttg tagatggctg ctctaaaaag    3960

acaaatgaat ggggaaagac aatcattgaa tacaaaacaa ataagccatc acgcctgccc    4020

ttccttgata ttgcaccttt ggacatcggt ggtgctgacc aggaattctt tgtggacatt    4080

ggcccagtct gtttcaaata a                                              4101

<210>  70
<211>  1466
<212>  PRT
<213>  Homo sapiens

<400>  70

Met Met Ser Phe Val Gln Lys Gly Ser Trp Leu Leu Leu Ala Leu Leu
1               5                   10                  15

His Pro Thr Ile Ile Leu Ala Gln Gln Glu Ala Val Glu Gly Gly Cys
            20                  25                  30

Ser His Leu Gly Gln Ser Tyr Ala Asp Arg Asp Val Trp Lys Pro Glu
        35                  40                  45

Pro Cys Gln Ile Cys Val Cys Asp Ser Gly Ser Val Leu Cys Asp Asp
    50                  55                  60

Ile Ile Cys Asp Asp Gln Glu Leu Asp Cys Pro Asn Pro Glu Ile Pro
65                  70                  75                  80

Phe Gly Glu Cys Cys Ala Val Cys Pro Gln Pro Pro Thr Ala Pro Thr
                85                  90                  95

Arg Pro Pro Asn Gly Gln Gly Pro Gln Gly Pro Lys Gly Asp Pro Gly
            100                 105                 110

Pro Pro Gly Ile Pro Gly Arg Asn Gly Asp Pro Gly Ile Pro Gly Gln
        115                 120                 125

Pro Gly Ser Pro Gly Ser Pro Gly Pro Pro Gly Ile Cys Glu Ser Cys
    130                 135                 140

Pro Thr Gly Pro Gln Asn Tyr Ser Pro Gln Tyr Asp Ser Tyr Asp Val
145                 150                 155                 160

Lys Ser Gly Val Ala Val Gly Gly Leu Ala Gly Tyr Pro Gly Pro Ala
                165                 170                 175

Gly Pro Pro Gly Pro Pro Gly Pro Pro Gly Thr Ser Gly His Pro Gly
            180                 185                 190

Ser Pro Gly Ser Pro Gly Tyr Gln Gly Pro Pro Gly Glu Pro Gly Gln
        195                 200                 205

Ala Gly Pro Ser Gly Pro Pro Gly Pro Pro Gly Ala Ile Gly Pro Ser
    210                 215                 220

Gly Pro Ala Gly Lys Asp Gly Glu Ser Gly Arg Pro Gly Arg Pro Gly
225                 230                 235                 240

Glu Arg Gly Leu Pro Gly Pro Pro Gly Ile Lys Gly Pro Ala Gly Ile
                245                 250                 255

Pro Gly Phe Pro Gly Met Lys Gly His Arg Gly Phe Asp Gly Arg Asn
            260                 265                 270

Gly Glu Lys Gly Glu Thr Gly Ala Pro Gly Leu Lys Gly Glu Asn Gly
        275                 280                 285

Leu Pro Gly Glu Asn Gly Ala Pro Gly Pro Met Gly Pro Arg Gly Ala
    290                 295                 300

Pro Gly Glu Arg Gly Arg Pro Gly Leu Pro Gly Ala Ala Gly Ala Arg
305                 310                 315                 320

Gly Asn Asp Gly Ala Arg Gly Ser Asp Gly Gln Pro Gly Pro Pro Gly
                325                 330                 335

Pro Pro Gly Thr Ala Gly Phe Pro Gly Ser Pro Gly Ala Lys Gly Glu
            340                 345                 350

Val Gly Pro Ala Gly Ser Pro Gly Ser Asn Gly Ala Pro Gly Gln Arg
        355                 360                 365

Gly Glu Pro Gly Pro Gln Gly His Ala Gly Ala Gln Gly Pro Pro Gly
    370                 375                 380

Pro Pro Gly Ile Asn Gly Ser Pro Gly Gly Lys Gly Glu Met Gly Pro
385                 390                 395                 400

Ala Gly Ile Pro Gly Ala Pro Gly Leu Met Gly Ala Arg Gly Pro Pro
                405                 410                 415

Gly Pro Ala Gly Ala Asn Gly Ala Pro Gly Leu Arg Gly Gly Ala Gly
            420                 425                 430

Glu Pro Gly Lys Asn Gly Ala Lys Gly Glu Pro Gly Pro Arg Gly Glu
        435                 440                 445

Arg Gly Glu Ala Gly Ile Pro Gly Val Pro Gly Ala Lys Gly Glu Asp
    450                 455                 460

Gly Lys Asp Gly Ser Pro Gly Glu Pro Gly Ala Asn Gly Leu Pro Gly
465                 470                 475                 480

Ala Ala Gly Glu Arg Gly Ala Pro Gly Phe Arg Gly Pro Ala Gly Pro
                485                 490                 495

Asn Gly Ile Pro Gly Glu Lys Gly Pro Ala Gly Glu Arg Gly Ala Pro
            500                 505                 510

Gly Pro Ala Gly Pro Arg Gly Ala Ala Gly Glu Pro Gly Arg Asp Gly
        515                 520                 525

Val Pro Gly Gly Pro Gly Met Arg Gly Met Pro Gly Ser Pro Gly Gly
    530                 535                 540

Pro Gly Ser Asp Gly Lys Pro Gly Pro Pro Gly Ser Gln Gly Glu Ser
545                 550                 555                 560

Gly Arg Pro Gly Pro Pro Gly Pro Ser Gly Pro Arg Gly Gln Pro Gly
                565                 570                 575

Val Met Gly Phe Pro Gly Pro Lys Gly Asn Asp Gly Ala Pro Gly Lys
            580                 585                 590

Asn Gly Glu Arg Gly Gly Pro Gly Gly Pro Gly Pro Gln Gly Pro Pro
        595                 600                 605

Gly Lys Asn Gly Glu Thr Gly Pro Gln Gly Pro Pro Gly Pro Thr Gly
    610                 615                 620

Pro Gly Gly Asp Lys Gly Asp Thr Gly Pro Pro Gly Pro Gln Gly Leu
625                 630                 635                 640

Gln Gly Leu Pro Gly Thr Gly Gly Pro Pro Gly Glu Asn Gly Lys Pro
                645                 650                 655

Gly Glu Pro Gly Pro Lys Gly Asp Ala Gly Ala Pro Gly Ala Pro Gly
            660                 665                 670

Gly Lys Gly Asp Ala Gly Ala Pro Gly Glu Arg Gly Pro Pro Gly Leu
        675                 680                 685

Ala Gly Ala Pro Gly Leu Arg Gly Gly Ala Gly Pro Pro Gly Pro Glu
    690                 695                 700

Gly Gly Lys Gly Ala Ala Gly Pro Pro Gly Pro Pro Gly Ala Ala Gly

705                 710                 715                 720

Thr Pro Gly Leu Gln Gly Met Pro Gly Glu Arg Gly Gly Leu Gly Ser
                725                 730                 735

Pro Gly Pro Lys Gly Asp Lys Gly Glu Pro Gly Gly Pro Gly Ala Asp
            740                 745                 750

Gly Val Pro Gly Lys Asp Gly Pro Arg Gly Pro Thr Gly Pro Ile Gly
        755                 760                 765

Pro Pro Gly Pro Ala Gly Gln Pro Gly Asp Lys Gly Glu Gly Gly Ala
    770                 775                 780

Pro Gly Leu Pro Gly Ile Ala Gly Pro Arg Gly Ser Pro Gly Glu Arg
785                 790                 795                 800

Gly Glu Thr Gly Pro Pro Gly Pro Ala Gly Phe Pro Gly Ala Pro Gly
                805                 810                 815

Gln Asn Gly Glu Pro Gly Gly Lys Gly Glu Arg Gly Ala Pro Gly Glu
            820                 825                 830

Lys Gly Glu Gly Gly Pro Pro Gly Val Ala Gly Pro Pro Gly Gly Ser
        835                 840                 845

Gly Pro Ala Gly Pro Pro Gly Pro Gln Gly Val Lys Gly Glu Arg Gly
    850                 855                 860

Ser Pro Gly Gly Pro Gly Ala Ala Gly Phe Pro Gly Ala Arg Gly Leu
865                 870                 875                 880

Pro Gly Pro Pro Gly Ser Asn Gly Asn Pro Gly Pro Pro Gly Pro Ser
                885                 890                 895

Gly Ser Pro Gly Lys Asp Gly Pro Pro Gly Pro Ala Gly Asn Thr Gly
            900                 905                 910

Ala Pro Gly Ser Pro Gly Val Ser Gly Pro Lys Gly Asp Ala Gly Gln
        915                 920                 925

Pro Gly Glu Lys Gly Ser Pro Gly Ala Gln Gly Pro Pro Gly Ala Pro
    930                 935                 940

Gly Pro Leu Gly Ile Ala Gly Ile Thr Gly Ala Arg Gly Leu Ala Gly
945                 950                 955                 960

Pro Pro Gly Met Pro Gly Pro Arg Gly Ser Pro Gly Pro Gln Gly Val
                965                 970                 975

Lys Gly Glu Ser Gly Lys Pro Gly Ala Asn Gly Leu Ser Gly Glu Arg
            980                 985                 990

Gly Pro Pro Gly Pro Gln Gly Leu Pro Gly Leu Ala Gly Thr Ala Gly
        995                 1000                1005

Glu Pro Gly Arg Asp Gly Asn Pro Gly Ser Asp Gly Leu Pro Gly
    1010                1015                1020

Arg Asp Gly Ser Pro Gly Gly Lys Gly Asp Arg Gly Glu Asn Gly
    1025                1030                1035

Ser Pro Gly Ala Pro Gly Ala Pro Gly His Pro Gly Pro Pro Gly
    1040                1045                1050

Pro Val Gly Pro Ala Gly Lys Ser Gly Asp Arg Gly Glu Ser Gly
    1055                1060                1065

Pro Ala Gly Pro Ala Gly Ala Pro Gly Pro Ala Gly Ser Arg Gly
    1070                1075                1080

Ala Pro Gly Pro Gln Gly Pro Arg Gly Asp Lys Gly Glu Thr Gly
    1085                1090                1095

Glu Arg Gly Ala Ala Gly Ile Lys Gly His Arg Gly Phe Pro Gly
    1100                1105                1110

Asn Pro Gly Ala Pro Gly Ser Pro Gly Pro Ala Gly Gln Gln Gly
    1115                1120                1125

Ala Ile Gly Ser Pro Gly Pro Ala Gly Pro Arg Gly Pro Val Gly
    1130                1135                1140

Pro Ser Gly Pro Pro Gly Lys Asp Gly Thr Ser Gly His Pro Gly
    1145                1150                1155

Pro Ile Gly Pro Pro Gly Pro Arg Gly Asn Arg Gly Glu Arg Gly
    1160                1165                1170

Ser Glu Gly Ser Pro Gly His Pro Gly Gln Pro Gly Pro Pro Gly
    1175                1180                1185

Pro Pro Gly Ala Pro Gly Pro Cys Cys Gly Gly Val Gly Ala Ala
    1190                1195                1200

Ala Ile Ala Gly Ile Gly Gly Glu Lys Ala Gly Gly Phe Ala Pro
    1205                1210                1215

Tyr Tyr Gly Asp Glu Pro Met Asp Phe Lys Ile Asn Thr Asp Glu
    1220                1225                1230

Ile Met Thr Ser Leu Lys Ser Val Asn Gly Gln Ile Glu Ser Leu
    1235                1240                1245

Ile Ser Pro Asp Gly Ser Arg Lys Asn Pro Ala Arg Asn Cys Arg
    1250                1255                1260

Asp Leu Lys Phe Cys His Pro Glu Leu Lys Ser Gly Glu Tyr Trp
    1265                1270                1275

Val Asp Pro Asn Gln Gly Cys Lys Leu Asp Ala Ile Lys Val Phe
    1280                1285                1290

Cys Asn Met Glu Thr Gly Glu Thr Cys Ile Ser Ala Asn Pro Leu
    1295                1300                1305

Asn Val Pro Arg Lys His Trp Trp Thr Asp Ser Ser Ala Glu Lys
    1310                1315                1320

Lys His Val Trp Phe Gly Glu Ser Met Asp Gly Gly Phe Gln Phe
    1325                1330                1335

Ser Tyr Gly Asn Pro Glu Leu Pro Glu Asp Val Leu Asp Val Gln
    1340                1345                1350

Leu Ala Phe Leu Arg Leu Leu Ser Ser Arg Ala Ser Gln Asn Ile
    1355                1360                1365

Thr Tyr His Cys Lys Asn Ser Ile Ala Tyr Met Asp Gln Ala Ser
    1370                1375                1380

Gly Asn Val Lys Lys Ala Leu Lys Leu Met Gly Ser Asn Glu Gly
    1385                1390                1395

Glu Phe Lys Ala Glu Gly Asn Ser Lys Phe Thr Tyr Thr Val Leu
    1400                1405                1410

Glu Asp Gly Cys Thr Lys His Thr Gly Glu Trp Ser Lys Thr Val
    1415                1420                1425

Phe Glu Tyr Arg Thr Arg Lys Ala Val Arg Leu Pro Ile Val Asp
    1430                1435                1440

Ile Ala Pro Tyr Asp Ile Gly Gly Pro Asp Gln Glu Phe Gly Val
    1445                1450                1455

Asp Val Gly Pro Val Cys Phe Leu
    1460                1465

<210> 71
<211> 4401
<212> DNA
<213> Homo sapiens

<400> 71
atgatgagct ttgtgcaaaa ggggagctgg ctacttctcg ctctgcttca tcccactatt 60

attttggcac aacaggaagc tgttgaagga ggatgttccc atcttggtca gtcctatgcg 120

gatagagatg tctggaagcc agaaccatgc caaatatgtg tctgtgactc aggatccgtt 180

ctctgcgatg acataatatg tgacgatcaa gaattagact gccccaaccc agaaattcca 240

tttggagaat gttgtgcagt ttgcccacag cctccaactg ctcctactcg ccctcctaat 300

ggtcaaggac ctcaaggccc caagggagat ccaggccctc ctggtattcc tgggagaaat 360

ggtgaccctg gtattccagg acaaccaggg tcccctggtt ctcctggccc ccctggaatc 420

tgtgaatcat gccctactgg tcctcagaac tattctcccc agtatgattc atatgatgtc 480

aagtctggag tagcagtagg aggactcgca ggctatcctg gaccagctgg ccccccaggc 540

cctcccggtc cccctggtac atctggtcat cctggttccc ctggatctcc aggataccaa 600

ggaccccctg gtgaacctgg gcaagctggt ccttcaggcc ctccaggacc tcctggtgct 660

ataggtccat ctggtcctgc tggaaaagat ggagaatcag gtagacccgg acgacctgga 720

gagcgaggat tgcctggacc tccaggtatc aaaggtccag ctgggatacc tggattccct 780

ggtatgaaag gacacagagg cttcgatgga cgaaatggag aaaagggtga aacaggtgct 840

cctggattaa agggtgaaaa tggtcttcca ggcgaaaatg gagctcctgg acccatgggt 900

ccaagagggg ctcctggtga gcgaggacgg ccaggacttc ctggggctgc aggtgctcgg 960

ggtaatgacg gtgctcgagg cagtgatggt caaccaggcc ctcctggtcc tcctggaact 1020

gccggattcc ctggatcccc tggtgctaag ggtgaagttg gacctgcagg gtctcctggt 1080

tcaaatggtg cccctggaca aagaggagaa cctggacctc agggacacgc tggtgctcaa 1140

ggtcctcctg gccctcctgg gattaatggt agtcctggtg gtaaaggcga aatgggtccc 1200

gctggcattc ctggagctcc tggactgatg ggagcccggg gtcctccagg accagccggt 1260

gctaatggtg ctcctggact gcgaggtggt gcaggtgagc ctggtaagaa tggtgccaaa 1320

ggagagcccg gaccacgtgg tgaacgcggt gaggctggta ttccaggtgt tccaggagct 1380

aaaggcgaag atggcaagga tggatcacct ggagaacctg gtgcaaatgg gcttccagga 1440

gctgcaggag aaaggggtgc ccctgggttc cgaggacctg ctggaccaaa tggcatccca 1500

ggagaaaagg gtcctgctgg agagcgtggt gctccaggcc ctgcagggcc cagaggagct 1560

gctggagaac ctggcagaga tggcgtccct ggaggtccag gaatgagggg catgcccgga 1620

agtccaggag gaccaggaag tgatgggaaa ccagggcctc ccggaagtca aggagaaagt 1680

ggtcgaccag gtcctcctgg gccatctggt ccccgaggtc agcctggtgt catgggcttc 1740
cccggtccta aaggaaatga tggtgctcct ggtaagaatg gagaacgagg tggccctgga 1800
ggacctggcc ctcagggtcc tcctggaaag aatggtgaaa ctggacctca aggaccccca 1860
gggcctactg ggcctggtgg tgacaaagga gacacaggac cccctggtcc acaaggatta 1920
caaggcttgc ctggtacagg tggtcctcca ggagaaaatg gaaaacctgg ggaaccaggt 1980
ccaaagggtg atgccggtgc acctggagct ccaggaggca agggtgatgc tggtgcccct 2040
ggtgaacgtg gacctcctgg attggcaggg gccccaggac ttagaggtgg agctggtccc 2100
cctggtcccg aaggaggaaa gggtgctgct ggtcctcctg ggccacctgg tgctgctggt 2160
actcctggtc tgcaaggaat gcctggagaa agaggaggtc ttggaagtcc tggtccaaag 2220
ggtgacaagg gtgaaccagg cggcccaggt gctgatggtg tcccagggaa agatggccca 2280
aggggtccta ctggtcctat tggtcctcct ggcccagctg gccagcctgg agataagggt 2340
gaaggtggtg cccccggact tccaggtata gctggacctc gtggtagccc tggtgagaga 2400
ggtgaaactg gccctccagg acctgctggt ttccctggtg ctcctggaca gaatggtgaa 2460
cctggtggta aaggagaaag aggggctccg ggtgagaaag gtgaaggagg ccctcctgga 2520
gttgcaggac cccctggagg ttctggacct gctggtcctc ctggtcccca aggtgtcaaa 2580
ggtgaacgtg gcagtcctgg tggacctggt gctgctggct tccctggtgc tcgtggtctt 2640
cctggtcctc ctggtagtaa tggtaaccca ggacccccag gtcccagcgg ttctccaggc 2700
aaggatgggc ccccaggtcc tgcgggtaac actggtgctc ctggcagccc tggagtgtct 2760
ggaccaaaag gtgatgctgg ccaaccagga gagaagggat cgcctggtgc ccagggccca 2820
ccaggagctc caggcccact tgggattgct gggatcactg gagcacgggg tcttgcagga 2880
ccaccaggca tgccaggtcc taggggaagc cctggccctc agggtgtcaa gggtgaaagt 2940
gggaaaccag gagctaacgg tctcagtgga gaacgtggtc cccctggacc ccagggtctt 3000
cctggtctgg ctggtacagc tggtgaacct ggaagagatg gaaaccctgg atcagatggt 3060
cttccaggcc gagatggatc tcctggtggc aagggtgatc gtggtgaaaa tggctctcct 3120
ggtgcccctg gcgctcctgg tcatccaggc ccacctggtc ctgtcggtcc agctggaaag 3180
agtggtgaca gaggagaaag tggccctgct ggccctgctg gtgctcccgg tcctgctggt 3240
tcccgaggtg ctcctggtcc tcaaggccca cgtggtgaca aaggtgaaac aggtgaacgt 3300
ggagctgctg gcatcaaagg acatcgagga ttccctggta atccaggtgc cccaggttct 3360
ccaggccctg ctggtcagca gggtgcaatc ggcagtccag gacctgcagg ccccagagga 3420
cctgttggac ccagtggacc tcctggcaaa gatggaacca gtggacatcc aggtcccatt 3480
ggaccaccag ggcctcgagg taacagaggt gaaagaggat ctgagggctc cccaggccac 3540
ccagggcaac caggccctcc tggacctcct ggtgcccctg gtccttgctg tggtggtgtt 3600

ggagccgctg ccattgctgg gattggaggt gaaaaagctg gcggttttgc cccgtattat 3660

ggagatgaac caatggattt caaaatcaac accgatgaga ttatgacttc actcaagtct 3720

gttaatggac aaatagaaag cctcattagt cctgatggtt ctcgtaaaaa ccccgctaga 3780

aactgcagag acctgaaatt ctgccatcct gaactcaaga gtggagaata ctgggttgac 3840

cctaaccaag gatgcaaatt ggatgctatc aaggtattct gtaatatgga aactggggaa 3900

acatgcataa gtgccaatcc tttgaatgtt ccacggaaac actggtggac agattctagt 3960

gctgagaaga aacacgtttg gtttggagag tccatggatg gtggttttca gtttagctac 4020

ggcaatcctg aacttcctga agatgtcctt gatgtgcagc tggcattcct tcgacttctc 4080

tccagccgag cttcccagaa catcacatat cactgcaaaa atagcattgc atacatggat 4140

caggccagtg gaaatgtaaa gaaggccctg aagctgatgg ggtcaaatga aggtgaattc 4200

aaggctgaag gaaatagcaa attcacctac acagttctgg aggatggttg cacgaaacac 4260

actggggaat ggagcaaaac agtctttgaa tatcgaacac gcaaggctgt gagactacct 4320

attgtagata ttgcacccta tgacattggt ggtcctgatc aagaatttgg tgtggacgtt 4380

ggccctgttt gcttttata a 4401

<210> 72
<211> 27
<212> DNA
<213> Artificial Sequence

<220>
<223> primer for cloning of human col1a1

<400> 72
cagccacaaa gagtctacat gtctagg 27

<210> 73
<211> 20
<212> DNA
<213> Artificial Sequence

<220>
<223> primer for cloning of human col1a1

<400> 73
aggttgggat ggagggagtt 20

<210> 74
<211> 37
<212> DNA
<213> Artificial Sequence

<220>
<223> primer for cloning of human col1a2

<400> 74
gccaagcttg catgctcagc tttgtggata cgcggac 37

<210>  75
<211>  44
<212>  DNA
<213>  Artificial Sequence

<220>
<223>  primer for cloning of human col1a2

<400>  75
cggtacccgg ggatccttat ttgaaacaga ctgggccaat gtcc                        44


<210>  76
<211>  34
<212>  DNA
<213>  Artificial Sequence

<220>
<223>  primer for cloning of human col1a1 with addition of restriction e
       nzyme site

<400>  76
tattcgaaac gatgttcagc tttgtggacc tccg                                   34


<210>  77
<211>  31
<212>  DNA
<213>  Artificial Sequence

<220>
<223>  primer for cloning of human col1a1 with addition of restriction e
       nzyme site

<400>  77
ttactagttt acaggaagca gacagggcca a                                      31


<210>  78
<211>  34
<212>  DNA
<213>  Artificial Sequence

<220>
<223>  primer for cloning of human col1a2 with addition of restriction e
       nzyme site

<400>  78
tattcgaaac gatgctcagc tttgtggata cgcg                                   34


<210>  79
<211>  35
<212>  DNA
<213>  Artificial Sequence

<220>
<223>  primer for cloning of human col1a2 with addition of restriction e
       nzyme site

<400>  79
ttactagttt atttgaaaca gactgggcca atgtc                                  35


<210>  80

<211>  35
<212>  DNA
<213>  Artificial Sequence

<220>
<223>  primer for cloning of expression unit with addition of restrictio
       n enzyme site

<400>  80
aacggccgtc taacatccaa agacgaaagg ttgaa                                35


<210>  81
<211>  35
<212>  DNA
<213>  Artificial Sequence

<220>
<223>  primer for cloning of expression unit with addition of restrictio
       n enzyme site

<400>  81
aacggccggc acaaacgaac gtctcactta atctt                                35


<210>  82
<211>  21
<212>  DNA
<213>  Artificial Sequence

<220>
<223>  primer for subcloning of plod2

<400>  82
tggagaggtg gtgatggaat t                                               21


<210>  83
<211>  22
<212>  DNA
<213>  Artificial Sequence

<220>
<223>  primer for subcloning of plod2

<400>  83
aaagagtgca gccattatcc tg                                              22